# EUROPEAN PATENT SPECIFICATION

(11) **EP 2 534 250 B1**
(45) Date of publication and mention of the grant of the patent: **12.04.2017**
(21) Application number: 11710314.3
(22) Date of filing: 08.02.2011
(51) Int. Cl.: C12N 15/82, C12N 9/06

(54) **IMPROVEMENT OF THE GRAIN FILLING OF A PLANT THROUGH THE MODULATION OF NADH-GLUTAMATE SYNTHASE ACTIVITY**
Verbesserung der Kornfüllung von Getreide durch Modulation der Aktivität der NADH-Glutamat-Synthase
Amélioration portant sur le remplissage des grains de blé via la modulation de l'activité de la nadh-glutamate synthase

(30) Priority: 08.02.2010 EP 10290058
(43) Date of publication of application: 19.12.2012
(73) Proprietor: Genoplante-Valor, 75015 Paris (FR)
(72) Inventor: SALSE, Jérôme, F-63610 Valbeleix (FR); QURAISHI, Umar, Masood, F-63100 Clermont-Ferrand (FR); PONT, Caroline, F-63610 Valbeleix (FR); MURAT, Florent, F-63000 Clermont-Ferrand (FR); LE GOUIS, Jacques, F-63320 Champeix (FR); LAFARGE, Stéphane, F-63670 La Roche Blanche (FR)
(74) Representative: Gevers & Orès
(86) International application number: PCT/IB2011/050534
(87) International publication number: WO 2011/095958

(56) References cited:
- EP-A1- 2 090 650
- WO-A2-03/000905
- US-A1- 2004 123 343
- DATABASE EMBL [Online] 18 June 2009 (2009-06-18), "WRIS_4912 cDNA library of a compatible interaction between stripe rust (Puccinia striiformis) and wheat Triticum aestivum cDNA 5' similar to Os01g0681900, mRNA sequence.", XP002585794, retrieved from EBI accession no. EMBL:GR304543 Database accession no. GR304543
- DATABASE EMBL [Online] 29 March 1997 (1997-03-29), "Oryza sativa gene for NADH-dependent glutamate synthase.", XP002585795, retrieved from EBI accession no. EMBL:AB001916 Database accession no. AB001916 -& GOTO SATOSHI ET AL: "Organization and structure of NADH-dependent glutamate synthase gene from rice plants", BIOCHIMICA ET BIOPHYSICA ACTA, vol. 1387, no. 1-2, 8 September 1998 (1998-09-08), pages 298-308, XP002585969, ISSN: 0006-3002
- YAMAYA TOMOYUKI ET AL: "Genetic manipulation and quantitative-trait loci mapping for nitrogen recycling in rice", JOURNAL OF EXPERIMENTAL BOTANY, vol. 53, no. 370, April 2002 (2002-04), pages 917-925, XP002585968, ISSN: 0022-0957
- BOISSON M ET AL: "Partial sequences of nitrogen metabolism genes in hexaploid wheat", THEORETICAL AND APPLIED GENETICS ; INTERNATIONAL JOURNAL OF PLANT BREEDING RESEARCH, SPRINGER, BERLIN, DE LNKD- DOI:10.1007/S00122-004-1913-4, vol. 110, no. 5, 1 March 2005 (2005-03-01) , pages 932-940, XP019321868, ISSN: 1432-2242
- CHICHKOVA S ET AL: "Transgenic tobacco plants that overexpress alfalfa NADH-glutamate synthase have higher carbon and nitrogen content", JOURNAL OF EXPERIMENTAL BOTANY, OXFORD UNIVERSITY PRESS, GB, vol. 52, no. 364, 1 November 2001 (2001-11-01), pages 2079-2087, XP002387035, ISSN: 0022-0957
- GOOD A G ET AL: "Can less yield more? Is reducing nutrient input into the environment compatible with maintaining crop production?", TRENDS IN PLANT SCIENCE, ELSEVIER SCIENCE, OXFORD, GB LNKD- DOI:10.1016/J.TPLANTS.2004.10.008, vol. 9, no. 12, 1 December 2004 (2004-12-01), pages 597-605, XP004750080, ISSN: 1360-1385
- SALSE JEROME ET AL: "Identification and characterization of shared duplications between rice and wheat provide new insight into grass genome evolution", PLANT CELL, vol. 20, no. 1, January 2008 (2008-01), pages 11-24, ISSN: 1040-4651
- HARUSHIMA YOSHIAKI ET AL: 'A high-density rice genetic linkage map with 2275 markers using a single F2 population' GENETICS, GENETICS SOCIETY OF AMERICA, AUSTIN, TX, US vol. 148, no. 1, 01 January 1998, pages 479 - 494, XP009134467 ISSN: 0016-6731

### SEQUENCE LISTING

<110> GENOPLANTE-VALOR
   SALSE, Jérôme
   QURAISHI, Umar Masood
   PONT, Caroline
   MURAT, Florent
   LEGOUIS, Jacques
   LAFARGE, Stéphane
<120> IMPROVEMENT OF THE GRAIN FILLING OF A PLANT THROUGH THE MODULATION OF NADH-GLUTAMATE SYNTHASE ACTIVITY
<130> MJP/XRN/cc-F1516/37
<150> EP 10290058.6
   <151> 2010-02-08
<160> 40
<170> PatentIn version 3.5
<210> 1
   <211> 2144
   <212> PRT
   <213> Triticum aestivum
<400> 1
   Met Pro Thr Ala Gln Gly Asp Trp Phe Glu Ala Ala Ala Pro Pro Gly
   1               5                   10                  15
   Gly Arg Arg Ala Arg Arg Ser Gln Ser Ala Ser Ala Pro Cys Arg Ser
               20                  25                  30
   Ala Arg Gln Ala His Gly Ala Met Ser Leu Asp Gly Gly Phe Leu Gly
           35                  40                  45
   Gly Ala Gln Arg Thr Glu Glu Arg Val Ala Pro Arg Pro Pro Arg Ala
       50                  55                  60
   Ala Ala Arg Asp Ala Glu Ser Ile Arg Pro Met Ser Leu Leu Pro Glu
   65                  70                  75                  80
   Ser Ser Ile Gly Leu Tyr Asp Pro Ala Phe Glu Arg Asp Ser Cys Gly
                   85                  90                  95
   Val Gly Phe Val Ala Glu Leu Ser Gly Val Asp Asn Arg Ala Thr Val
               100                 105                 110
   Val Asp Ala Ile Gln Met Leu Glu Arg Met Ala His Arg Gly Ala Cys
           115                 120                 125
   Gly Cys Glu Lys Asn Thr Gly Asp Gly Ala Gly Ile Leu Val Ala Leu
       130                 135                 140
   Pro His Thr Phe Phe Arg Glu Val Thr Lys Asp Ala Gly Phe Glu Leu
   145                 150                 155                 160
   Pro Pro Pro Gly Glu Tyr Ala Val Gly Met Val Phe Leu Pro Thr Asp
                   165                 170                 175
   Glu Lys Arg Arg Glu Arg Ser Lys Thr Glu Phe Thr Lys Val Ala Glu
               180                 185                 190
   Ser Leu Gly His Ser Ile Leu Gly Trp Arg Gln Val Pro Thr Asp Asn
           195                 200                 205
   Ser Asp Leu Gly Gln Ala Ala Leu Asp Thr Glu Pro Ala Ile Glu Gln
       210                 215                 220
   Val Phe Leu Thr Lys Ser Ser Lys Ser Lys Ala Asp Phe Glu Gln Gln
   225                 230                 235                 240
   Val Phe Ile Leu Arg Arg Leu Ser Ile Val Ser Ile Arg Ala Ala Leu
                   245                 250                 255
   Asn Leu Gln Arg Gly Gly Glu Arg Asp Phe Tyr Met Cys Ser Leu Ser
               260                 265                 270
   Ser Arg Thr Ile Val Tyr Lys Gly Gln Leu Met Pro Ser Gln Leu Gln
           275                 280                 285
   Gly Tyr Tyr Tyr Ala Asp Ile Gly His Glu Asn Phe Ser Ser Tyr Met
       290                 295                 300
   Ala Leu Val His Ser Arg Phe Ser Thr Asn Thr Phe Pro Ser Trp Asp
   305                 310                 315                 320
   Arg Ala Gln Pro Met Arg Val Leu Gly His Asn Gly Glu Ile Asn Thr
                   325                 330                 335
   Leu Lys Gly Asn Lys Asn Trp Met Lys Ala Arg Glu Gly Leu Leu Glu
               340                 345                 350
   Cys Glu Lys Leu Gly Leu Ser Gln Asp Glu Met Ser Lys Ile Leu Pro
           355                 360                 365
   Ile Val Asp Ala Thr Ser Ser Asp Ser Gly Ala Phe Asp Gly Val Leu
       370                 375                 380
   Glu Leu Leu Ile Arg Gly Gly Arg Ser Leu Pro Glu Ala Val Met Met
   385                 390                 395                 400
   Met Ile Pro Glu Ala Trp Gln Asn Asp Val Asn Met Glu Pro Asp Lys
                   405                 410                 415
   Lys Ala Leu Tyr Glu Phe Leu Ser Ala Leu Met Glu Pro Trp Asp Gly
               420                 425                 430
   Pro Ala Leu Ile Ser Phe Thr Asp Gly Arg Tyr Leu Gly Ala Thr Leu
           435                 440                 445
   Asp Arg Asn Gly Leu Arg Pro Gly Arg Phe Tyr Val Thr His Ser Gly
       450                 455                 460
   Arg Val Val Met Gly Ser Glu Val Gly Val Val Asp Ile Pro Ala Gln
   465                 470                 475                 480
   Asp Val Leu Arg Lys Gly Arg Leu Asn Pro Gly Met Met Leu Leu Val
                   485                 490                 495
   Asp Phe Asp Asn His Thr Val Val Asp Asp Glu Ala Leu Lys Ala Gln
               500                 505                 510
   Tyr Ser Lys Ala His Pro Tyr Gly Glu Trp Leu Lys Arg Gln Lys Met
           515                 520                 525
   Tyr Leu Lys Asp Ile Val Glu Ser Val Pro Glu Thr Asp Arg Val Ala
       530                 535                 540
   Pro Ser Ile Ser Gly Ser Ile Thr Gln Thr Asn Glu Asn Lys Glu Cys
   545                 550                 555                 560
   Val Gly Ile Asn Ala Ile Val Thr Pro Leu Lys Ala Phe Gly Tyr Thr
                   565                 570                 575
   Leu Glu Ala Leu Glu Met Leu Leu Leu Pro Met Ala Lys Asp Gly Val
               580                 585                 590
   Glu Ala Leu Gly Ser Met Gly Asn Asp Ala Pro Leu Ala Val Met Ser
           595                 600                 605
   Asn Arg Glu Lys Leu Thr Phe Glu Tyr Phe Lys Gln Met Phe Ala Gln
       610                 615                 620
   Val Thr Asn Pro Pro Ile Asp Pro Ile Arg Glu Lys Ile Val Thr Ser
   625                 630                 635                 640
   Met Glu Cys Met Ile Gly Pro Glu Gly Asp Leu Leu Glu Ile Thr Glu
                   645                 650                 655
   Lys Gln Cys Asn Arg Leu Ala Leu Lys Gly Pro Leu Val Ser Met Asp
               660                 665                 670
   Glu Met Glu Ser Ile Lys Lys Met Asn Tyr Arg Gly Trp Arg Ser Lys
           675                 680                 685
   Val Leu Asp Ile Thr Tyr Pro Lys Asn Ser Gly Arg Lys Gly Leu Glu
       690                 695                 700
   Glu Thr Leu Asp Arg Ile Cys Ala Glu Ala Arg Glu Ala Ile Arg Glu
   705                 710                 715                 720
   Gly Tyr Lys Ile Leu Val Leu Ser Asp Arg Gly Phe Ser Ser Asp Arg
                   725                 730                 735
   Val Ala Val Ser Ser Leu Leu Ala Val Gly Ala Val His Gln His Leu
               740                 745                 750
   Val Ala Asn Leu Glu Arg Thr Arg Val Gly Leu Leu Val Glu Ser Ala
           755                 760                 765
   Glu Pro Arg Glu Val His His Phe Cys Thr Leu Val Gly Phe Gly Ala
       770                 775                 780
   Asp Ala Ile Cys Pro Tyr Leu Ala Ile Glu Ala Ile Trp Cys Leu Gln
   785                 790                 795                 800
   Thr Asp Gly Lys Ile Pro Pro Thr Asp Ser Lys Glu Glu Leu Val Glu
                   805                 810                 815
   Lys Tyr Phe Tyr Ala Ser Ile Tyr Gly Met Met Lys Val Leu Ala Lys
               820                 825                 830
   Met Gly Ile Ser Thr Leu Ala Ser Tyr Lys Gly Ala Gln Ile Phe Glu
           835                 840                 845
   Ala Leu Gly Leu Ser Ser Glu Val Ile His Lys Cys Phe Glu Gly Thr
       850                 855                 860
   Pro Ser Arg Ile Glu Gly Ala Thr Phe Glu Met Leu Ala Arg Asp Ala
   865                 870                 875                 880
   Leu Arg Leu His Glu Leu Ala Phe Pro Ser Arg Thr Pro Pro Pro Gly
                   885                 890                 895
   Ser Ala Asp Ala Lys Ala Leu Pro Asn Pro Gly Asp Tyr His Trp Arg
               900                 905                 910
   Lys Asn Gly Glu Val His Leu Asn Asp Pro Leu Ala Met Ala Lys Leu
           915                 920                 925
   Gln Glu Ala Ala Lys Val Asn Ser Arg Glu Ala Tyr Lys Glu Tyr Ser
       930                 935                 940
   Lys Arg Ile Gln Glu Leu Asn Lys Ala Cys Asn Leu Arg Gly Met Leu
   945                 950                 955                 960
   Lys Phe Ile Asp Ser Thr Ser Lys Ile Ser Leu Asp Glu Val Glu Pro
                   965                 970                 975
   Ala Ser Glu Ile Val Lys Arg Phe Cys Thr Gly Ala Met Ser Tyr Gly
               980                 985                 990
   Ser Ile Ser Leu Glu Ala His Thr  Ala Leu Ala Val Ala  Met Asn Lys
           995                 1000                1005
   Leu Gly  Gly Lys Ser Asn Thr  Gly Glu Gly Gly Glu  Gln Pro Ser
       1010                 1015                 1020
   Arg Met  Glu Pro Leu Pro Asp  Gly Ser Met Asn Pro  Lys Arg Ser
       1025                 1030                 1035
   Ala Ile  Lys Gln Val Ala Ser  Gly Arg Phe Gly Val  Ser Ser Tyr
       1040                 1045                 1050
   Tyr Leu  Thr Asn Ala Asp Gly  Leu Gln Ile Lys Met  Ala Gln Gly
       1055                 1060                 1065
   Ala Lys  Pro Gly Glu Gly Gly  Glu Leu Pro Gly His  Lys Val Ile
       1070                 1075                 1080
   Gly Asp  Ile Ala Val Thr Arg  His Ser Thr Ala Gly  Val Gly Leu
       1085                 1090                 1095
   Ile Ser  Pro Pro Pro His His  Asp Ile Tyr Ser Ile  Glu Asp Leu
       1100                 1105                 1110
   Ala Gln  Leu Ile His Asp Leu  Lys Asn Ser Asn Pro  Gln Ala Arg
       1115                 1120                 1125
   Ile Ser  Val Lys Leu Val Ser  Glu Ala Gly Val Gly  Val Val Ala
       1130                 1135                 1140
   Ser Gly  Val Val Lys Gly His  Ala Asp His Val Leu  Ile Ser Gly
       1145                 1150                 1155
   His Asp  Gly Gly Thr Gly Ala  Ser Arg Trp Thr Gly  Ile Lys Asn
       1160                 1165                 1170
   Ala Gly  Leu Pro Trp Glu Leu  Gly Leu Ala Glu Thr  His Gln Thr
       1175                 1180                 1185
   Leu Val  Ala Asn Gly Leu Arg  Gly Arg Ala Val Leu  Gln Thr Asp
       1190                 1195                 1200
   Gly Gln  Leu Lys Thr Gly Arg  Asp Val Ala Val Ala  Cys Leu Leu
       1205                 1210                 1215
   Gly Ala  Glu Glu Phe Gly Phe  Ser Thr Ala Pro Leu  Ile Thr Leu
       1220                 1225                 1230
   Gly Cys  Ile Met Met Arg Lys  Cys His Thr Asn Thr  Cys Pro Val
       1235                 1240                 1245
   Gly Ile  Ala Thr Gln Asp Pro  Val Leu Arg Glu Lys  Phe Ala Gly
       1250                 1255                 1260
   Glu Pro  Glu His Val Ile Asn  Phe Phe Phe Met Leu  Ala Glu Glu
       1265                 1270                 1275
   Leu Arg  Glu Ile Met Ala Gln  Leu Gly Leu Arg Thr  Ile Asn Glu
       1280                 1285                 1290
   Met Val  Gly Arg Ser Asp Met  Leu Glu Val Asp Pro  Glu Val Val
       1295                 1300                 1305
   Lys Ser  Asn Glu Lys Leu Glu  Asn Ile Asp Leu Ser  Leu Ile Leu
       1310                 1315                 1320
   Lys Pro  Ala Ala Glu Ile Arg  Pro Gly Ala Ala Gln  Tyr Cys Val
       1325                 1330                 1335
   Glu Lys  Gln Asp His Gly Leu  Asp Met Ala Leu Asp  Asn Lys Leu
       1340                 1345                 1350
   Ile Ala  Leu Ser Arg Ala Ala  Leu Glu Lys Glu Val  Arg Val Phe
       1355                 1360                 1365
   Ile Glu  Thr Pro Ile Lys Asn  Thr Asn Arg Ala Val  Gly Thr Thr
       1370                 1375                 1380
   Leu Ser  His Glu Val Thr Lys  Arg Tyr His Met Lys  Gly Leu Asp
       1385                 1390                 1395
   Pro Gly  Thr Ile His Val Lys  Leu Thr Gly Ser Ala  Gly Gln Ser
       1400                 1405                 1410
   Phe Gly  Ala Phe Leu Cys Pro  Gly Ile Thr Leu Glu  Leu Glu Gly
   1415 1420 1425
   Asp Ser Asn Asp Tyr Val Gly Lys Gly Leu Ser Gly Gly Lys Ile
   1430 1435 1440
   Val Val Tyr Pro Pro Arg Asn Ser Thr Phe Ser Ala Glu Asp Asn
   1445 1450 1455
   Ile Val Ile Gly Asn Val Ala Leu Tyr Gly Ala Thr Lys Gly Glu
   1460 1465 1470
   Ala Tyr Phe Asn Gly Met Ala Ala Glu Arg Phe Cys Val Arg Asn
   1475 1480 1485
   Ser Gly Ala Arg Thr Val Val Glu Gly Ile Gly Asp His Gly Cys
   1490 1495 1500
   Glu Tyr Met Thr Gly Gly Thr Val Val Ile Leu Gly Lys Thr Gly
   1505 1510 1515
   Arg Asn Phe Ala Ala Gly Met Ser Gly Gly Ile Ala Tyr Val Tyr
   1520 1525 1530
   Asp Val Asp Gly Thr Phe Ser Val Arg Cys Asn Asn Glu Leu Val
   1535 1540 1545
   Asp Leu Tyr His Val Glu Glu Glu Asp Asp Val Thr Thr Leu Lys
   1550 1555 1560
   Met Met Ile Glu Gln His Arg Leu His Thr Glu Ser Val Leu Ala
   1565 1570 1575
   Lys Asp Ile Leu Ser Lys Phe Asp Thr Leu Leu Pro Lys Phe Val
   1580 1585 1590
   Lys Val Tyr Pro Arg Asp Tyr Lys Arg Val Leu Glu Glu Met Lys
   1595 1600 1605
   Ala Glu Lys Ala Ala Ala Arg Pro Thr Lys Glu Pro Lys Val Ala
   1610 1615 1620
   Asn Gly Val Ser Val Thr Thr Lys Lys Ile Gln Thr Glu Lys Ser
   1625 1630 1635
   Ser Ser Arg Pro Thr Arg Val Ala Asn Ala Lys Lys Tyr Arg Gly
   1640 1645 1650
   Phe Val Thr Tyr Glu Arg Glu Gly Val Ser Tyr Arg Asp Pro Asn
   1655 1660 1665
   Glu Arg Val Lys Asp Trp Asn Glu Val Ala Ile Glu Ser Val Pro
   1670 1675 1680
   Gly Pro Leu Leu Asn Thr Gln Ser Ala Arg Cys Met Asp Cys Gly
   1685 1690 1695
   Thr Pro Phe Cys His Gln Glu Ser Ser Gly Ala Gly Cys Pro Leu
   1700 1705 1710
   Gly Asn Lys Ile Pro Glu Phe Asn Glu Leu Val His Gln Asn Arg
   1715 1720 1725
   Trp Arg Glu Ala Leu Asp Arg Leu Leu Glu Thr Asn Asn Phe Pro
   1730 1735 1740
   Glu Phe Thr Gly Arg Val Cys Pro Ala Pro Cys Glu Gly Ser Cys
   1745 1750 1755
   Val Leu Gly Ile Ile Glu Asn Pro Val Ser Ile Lys Ser Ile Glu
   1760 1765 1770
   Cys Ser Ile Ile Asp Lys Gly Phe Glu Glu Gly Trp Met Val Pro
   1775 1780 1785
   Arg Pro Pro Leu Gln Arg Thr Gly Lys Lys Ile Ala Ile Val Gly
   1790 1795 1800
   Ser Gly Pro Ala Gly Leu Ala Ala Ala Asp Gln Leu Asn Lys Met
   1805 1810 1815
   Gly His Phe Val Thr Val Phe Glu Arg Ser Asp Arg Ile Gly Gly
   1820 1825 1830
   Leu Met Met Tyr Gly Val Pro Asn Met Lys Thr Asp Lys Ile Gly
   1835 1840 1845
   Val Val Gln Arg Arg Val Asn Leu Met Ala Glu Glu Gly Val Thr
   1850 1855 1860
   Phe Val Val Asn Ala Asn Val Gly Ser Asp Pro Leu Tyr Ser Ile
   1865 1870 1875
   Glu Arg Leu His Ser Glu Asn Asn Ala Val Ile Leu Ala Cys Gly
   1880 1885 1890
   Ala Thr Lys Pro Arg Asp Leu Ser Ile Pro Gly Arg Glu Leu Ala
   1895 1900 1905
   Gly Val His Phe Ala Met Glu Phe Leu His Ala Asn Thr Lys Ser
   1910 1915 1920
   Leu Leu Asp Ser Asn Leu Glu Asp Gly Arg Tyr Ile Ser Ala Gln
   1925 1930 1935
   Gly Lys Lys Val Val Val Ile Gly Gly Gly Asp Thr Gly Thr Asp
   1940 1945 1950
   Cys Ile Gly Thr Ser Val Arg His Gly Cys Ser Ser Ile Val Asn
   1955 1960 1965
   Leu Glu Leu Leu Thr Lys Pro Pro Ser Lys Arg Ala Ser Asp Asn
   1970 1975 1980
   Pro Trp Pro Gln Trp Pro Arg Val Phe Arg Val Asp Tyr Gly His
   1985 1990 1995
   Gln Glu Ala Ser Thr Lys Phe Gly Asn Asp Pro Arg Thr Tyr Glu
   2000 2005 2010
   Val Leu Thr Lys Arg Phe Ile Gly Asp Glu Asp Gly Lys Leu Lys
   2015 2020 2025
   Ala Leu Glu Val Val Arg Val Lys Trp Glu Lys Val Asp Gly Lys
   2030 2035 2040
   Phe Gln Phe Lys Glu Ile Glu Gly Ser Gln Glu Ile Ile Glu Ala
   2045 2050 2055
   Asp Leu Val Leu Leu Ala Met Gly Phe Leu Gly Pro Glu Glu Asn
   2060 2065 2070
   Ile Ala Asp Lys Leu Gly Leu Glu Lys Asp Asn Arg Ser Asn Phe
   2075 2080 2085
   Lys Ala Gln Phe Gly His Phe Gly Thr Ser Val Asp Gly Val Phe
   2090 2095 2100
   Ala Ala Gly Asp Cys Arg Arg Gly Gln Ser Leu Val Val Trp Ala
   2105 2110 2115
   Ile Thr Glu Gly Arg Glu Ala Ala Ala Ala Val Asp Lys Tyr Leu
   2120 2125 2130
   Ser Arg Asp Glu Gln Asn Val Ala Gly Leu Thr
   2135 2140
<210> 2
   <211> 10173
   <212> DNA
   <213> Triticum aestivum
<400> 2
   atgccgacgg cgcaggggga ttggtttgaa gcacgcggcg ccgccgggcg gccgcagggc      60
   ccgccgcagc cagtctgcct cggcgccgtg ccgctccgca aggcaggcgc acggcgccat     120
   gtccctggat ggcgggttcc tcggcggcgc gcagcgcacc gaggaacgcg tcgcgccacg     180
   cccgcctcgg gccgcggcgc gcgacgccga gtccatcagg cccatgtctc tgctacccga     240
   gagcagcatt gggctgtacg acccggcgtt cgagcgtgac tcgtgcggcg ttggcttcgt     300
   cgccgagctg tcgggcgttg acaaccgggc gaccgtgagt tctttgcacc aggacaccgc     360
   cgcagcttct ctcttttatc taccacgttg agatattgat tttgctgtga tttactttac     420
   aggtcgtcga tgccattcag atgcttgaaa gaatggcaca ccgaggtgcc tgcggctgtg     480
   agaaaaacac tggtgatggt gccggcattc tcgttgctct accacacacc ttcttccgag     540
   aggtgaataa acagaaaatt tcaagcaaac tcagttttcc ttgacagagt tattcatctt     600
   gggtgttgct ttgctattgc tgcaggtgac aaaggatgcc ggtttcgagt taccgccacc     660
   aggtgagtat gctgttggaa tggtcttcct gccaaccgat gagaagcgcc gcgagaggag     720
   caaaactgag tttacaaagg ttggttggtt ggtgtataga caattagaca gcgtgcgagg     780
   cgagtctgcg atggtaagct tatgtcttgg attgttttta ttcatgcagg tcgctgagtc     840
   tctaggacat tcgatacttg ggtggcgcca ggttcccact gacaattcag acttgggcca     900
   agctgcgctc gatactgaac cagccattga acaggttttc ctcaccaaga gttcaaaatc     960
   gaaggccgac ttcgaacagc aggtcctcca tgagtttaat tttctctcaa cacatgtaat    1020
   gagctttcct ctcaactaac ccatcaatta tcacagttgt ttatcctgag gaggctctca    1080
   attgtatcta tccgggccgc gctgaatctt cagcgtggag gagagagaga tttctacatg    1140
   tgctctctat cttcaaggtc cttacttctt ctgaattgca ttttcactac aaactgatga    1200
   ggttgagatc agggagccca attaagctcc tgactttaat ggaacattaa caacacaaaa    1260
   gttgatagga aagctgactg ggaaaaacaa tcctgggcta tctcttaagc attagaaatc    1320
   tgtaatgtaa ggacatttca tttttgctgt attcatgtga gtaattattg ttaaatcttc    1380
   tattgtggtt ttccaggacc attgtttaca agggccaact tatgccatcc cagctccaag    1440
   ggtactacta tgcggatata ggtcatgaaa acttctccag ttatatggct ctggtaaata    1500
   cgaaaccttg atactcttac aacaaaagtc atggtcgtag tcagcggatg ggaatcaata    1560
   atagcaagcc agttgtaaga tcaagactcg ctcctgtgca gacatcaaat ttgagctgtc    1620
   tattcatatg ctatttcagt cctgcttcgt gatgccttta agtgaagtac tagaaacttc    1680
   tcttagttga tacccgataa ccatgttgca gagtccagct gcagttctgc tatggccgac    1740
   tgcttgccga ctcttcagga tgtgaccgaa gaaattgtgt ttggtttgag gtagcttagg    1800
   gctcccaact gttttgcatg aaattatacg cagctgacgt atttttacaa aattcgttct 1860
   tcacaaggaa gttttgtttt gaatatctgc aaaagatgtt tataatatga tttctaaaag 1920
   tcatatcttg aaacaggttc actcaaggtt ctccaccaac accttcccca gctgggaccg 1980
   tgcacagcca atgcgtgtct taggccacaa tggagagatc aatactctca aagggaacaa 2040
   aaactggtaa tattattgtc aaatcttttc ctctttgctt taaccttttc ttggcagatg 2100
   caactatctc aatatgcacg ttgtatatat ggcaggatga aagctcgtga gggtctcttg 2160
   gagtgtgaga agcttggcct atcacaggat gaaatgtcaa agattcttcc aatagtagat 2220
   gccacttctt cagattcagg tccgcagtct aatatgcagc atgaattcca cttggcaaat 2280
   aaaaattcat ctcggttttg tgagttacta agtgtgaaaa aacatgtgaa ttctaggtgc 2340
   atttgatggt gttctcgagc tccttatccg tggtggaaga agcctgccag aagctgtgat 2400
   gatgatgatc cctgaggcgt ggcagaatga tgtaaacatg gaacctgata agaaagctct 2460
   gtacgagttc ttgtcagccc ttatggagcc ttgggatgga cctgctctca tatcttgtaa 2520
   aaaacttaaa cccgtttctt ttttctgatc atacttctaa ctgattactt aagctaacat 2580
   gggtacaacc tgtagttacc gatggccgct accttggagc taccctggat cgcaatggcc 2640
   ttaggcctgg tcgattttat gtaacccaca gtggacgtgt ggtcatgggt agtgaagttg 2700
   gtgttgtgga tattcctgcc caagatgtgt tgagaaaggg tcggcttaac cctggaatga 2760
   tgttactcgt tgacttcgac aaccatactg tagtagatga tgaagcactc aaggcacagt 2820
   actctaaagc tcacccgtat ggagaatggc tcaagcgaca aaagatgtac ctcaaagaca 2880
   ttgtagaatc tgtcccagaa actgacagag ttgctccaag catttctggt tctattacgg 2940
   taagtactta cgtggtccaa cttcctcatt cactgggtag tttggtgatg aaaattggta 3000
   aaccataagg aacatggtga ttttaatatt cttttacaat ttggtgtagc aaacaaatga 3060
   gaacaaggaa tgtgtaggca tcaatgcaat tgtgactcca ctaaaggcgt ttgggtaagc 3120
   aaggattgaa cattgtcaac tatcactcat gtttcaattt ctgctggcta atgatccaac 3180
   cgattaacta caggtacaca ttggaagccc tagaaatgtt gctgctgcca atggctaaag 3240
   atggagtgga agctcttgga tcaatgggaa atgatgcacc cctagcagtg atgtcaaaca 3300
   gagagaagct gacttttgag tacttcaagc agatgtttgc acaagtaaca aaccctccaa 3360
   tcgatccaat tagggagaag attgttacat ctatggaatg tatgattggg ccagaaggag 3420
   atttgctgga aataaccgaa aagcaatgca accgccttgc acttaaaggt cctttggtgt 3480
   caatggatga aatggaatct atcaagaaga tgaactaccg tggttggcgc agcaaggtgc 3540
   tcgacataac ttatccgaag aattctggaa ggaagggctt ggaagaaact ttggatagaa 3600
   tttgtgctga agcccgggaa gctatacgcg agggatacaa aattttagtt ctttcagaca 3660
   gaggtcagtg acttattcac tttatacttg catcccattg tttgatggta caaattactc 3720
   ataagttgac aaaaaattct agcagatggg tcaggtgtgc acaatttatt atcgttagct 3780
   tccagcaaac atatcacatc accatatagt aacttaaata tgatcataat tttgtttgat 3840
   agtcaattgt ttgcagtgta atgtgttgta tgaaggacat tcttactttg tcaccttgga 3900
   cataattgga atgtatatta agtttattat gcaactcaac atcttccaat actgaaattt 3960
   gttgtatttt cttgattcag gattttcttc agaccgtgtt gctgtcagtt ccctcttagc 4020
   agttggagca gtacatcaac accttgttgc aaatcttgag aggacacgcg taggattatt 4080
   ggttgagtct gctgaacctc gtgaagtgca ccatttctgt acactcgttg gatttggtgc 4140
   agatgctata tgcccttatt tggccattga agcaatttgg tgcttgcaga ctgatgggaa 4200
   gattccccct accgactcaa aggaggaact tgtcgaaaaa tatttttatg cttccatcta 4260
   tggaatgatg aaggttcttg caaagatggg aatatccacc cttgcatctt acaaaggggc 4320
   acagattttt gaagctcttg gactttcttc tgaagtgatt cacaagtgtt ttgaaggcac 4380
   tcctagcaga attgagggtg caacgttcga aatgcttgca cgtgatgctc tccgtcttca 4440
   cgagttggca ttcccatcaa gaacacctcc gcctggcagt gcagatgcaa aagcccttcc 4500
   caatccaggg gattaccact ggaggaaaaa tggtgaagtc catctaaatg atcctcttgc 4560
   aatggcaaaa ttacaagaag cagctaaagt aaacagccgg gaagcataca aagaatattc 4620
   taagcgaatt caagagctta acaaggcgtg caatctgcgt ggtatgctga aatttataga 4680
   tagcactagc aagatttctt tggatgaggt tgaacctgca agtgagatag tgaaacgctt 4740
   ttgtactggg gcaatgagtt atggatctat ttcgttggag gcacatactg cccttgctgt 4800
   ggccatgaac aaactgggag gcaaatctaa cacaggtatt tcatgcatat gtgacatttt 4860
   ttaccctttg gctggttgtt acccttctga tgaaatcatg tatagtagct ttacttgcac 4920
   atcaactctt agagccttgt tgcataacat atttttctta tttttgctgc agtagtgtag 4980
   tgtctatgtt tctactgttt aaatatgccc tcgaagttgg tactttatac atgaccatgt 5040
   ctagaaatgc actacttatt ttgatgctat cacatgaaaa ctatactctc atatttactt 5100
   gtcgacatac ttgtactgca cttgtgtgtt tggttgcatt ctcgtctcag catagttccc 5160
   tcttcatgca tgctcaactc aacacccctc ttcatgcatg ctttcttcat gcatgcttct 5220
   agtgtatttg tgctaagcta gtgtggactc atgcaccctc catacactct accaaacacc 5280
   caaaagtggg tcgagaaggg aactcttggg ctcatgcacc cttcatacac cctaccaaac 5340
   acacccttaa tgcgctaagc ttttcatctt cctgttgatg ttgaatgcac ttctgtgaac 5400
   aaattatccc agttcatagc aagagttaac ttctaagctt ttatctatct gttgatgttg 5460
   aatgcacttc tggtagcaaa ttatcctaat tcctagcaag atttaacttc tgcttgcgta 5520
   gaaacccaga cagcttgcac gcactattct tctttggact gtttgtatta gatattttct 5580
   gcccacacag ttaatattat attgccaaat ctttgtattt attttcgctc ctaaactaaa 5640
   ctcattttac ccatctgcta atttagggga gggaggggag cagccttctc gtatggagcc 5700
   tcttcctgat ggttcgatga atccaaaacg aagtgcaatc aagcaagttg ccagtggacg 5760
   atttggagtt tccagctatt atctgactaa tgcagatggg ctgcagataa aaatggctca 5820
   ggtacttgac aatgctgtgc attgcgcaat cactggaatg atctcagttt atagaaagca 5880
   actgtattca acgtcctgtg gttggcattt attctttttt tgtatcgtat atgtataggg 5940
   tgctaagcct ggtgaaggtg gtgagcttcc aggtcacaag gttatcggtg acattgcagt 6000
   taccagacat tctacagctg gtgttgggct tattagtcca cctcctcacc atgatatata 6060
   ttctatcgag gatcttgcac agcttatcca tgaccttaag gtacattacc tgatgctctt 6120
   ctatagccaa ggcatctgcc tatttatgta tggttgttag ttgctacttc tagattatat 6180
   tggttatcta aacgctgtga tggtctaacc ctcctgagaa gctagaatca cttgctaaac 6240
   tggtacaagt gatcctgcac atatgcatgt gtctccattt gggggtttac cctccttttt 6300
   gaaaaactca ttttgcaaat gttacctgct gatgtagtga tattcatgcc tgttttctct 6360
   ctgcagaatt caaatcctca agctcgaatc agcgtgaagc tagtgtctga ggctggtgtt 6420
   ggagttgtag ctagcggtgt tgtaaaagga cacgcagacc atgttcttat ttctggccat 6480
   gacggtggca ccggtgcatc aagatggaca ggtatcaaga atgctggact tccatgggaa 6540
   ctaggattgg ctgagacaca tcaaacttta gtggcaaatg ggcttcgtgg tcgagctgtc 6600
   ctacaaacag atggccaatt aaaaactggt agagatgttg ctgtggcgtg cttacttggt 6660
   gcagaggaat ttggtttcag cactgctcca ctgatcacac ttggctgcat tatgatgcgg 6720
   aagtgccata ccaatacttg ccctgttggt atagccactc aagatccagt gctccgagaa 6780
   aaatttgctg gagaaccaga gcatgtcatt aactttttct tcatgcttgc tgaggagcta 6840
   agagaaatta tggctcagct tggcctccgt acaattaatg aaatggttgg acgctctgat 6900
   atgcttgaag ttgatccaga agtagttaag agcaatgaga aacttgaaaa tattgatctc 6960
   tcattgatct taaaaccagc tgcagagatt cgtcctgggg ctgctcagta ctgtgttgaa 7020
   aagcaagacc atggccttga catggctttg gataacaaac ttatagcttt atcaagggct 7080
   gcacttgaaa aggaagttcg tgttttcatt gagactccaa tcaagaacac taatcgggca 7140
   gtaggcacca cacttagcca tgaagtcaca aaacgttatc acatgaaggg attagatcct 7200
   ggaaccattc atgtgaaact cactggaagt gctggtcaga gttttggtgc ttttctctgt 7260
   cctggaataa cccttgagct tgaaggagac agcaatgatt acgttggcaa aggattatct 7320
   ggtggaaaga ttgttgtgta cccacccagg aacagtacat ttagtgcaga agacaatatt 7380
   gtcattggta atgtggccct gtatggtgct accaagggag aagcatactt caatggaatg 7440
   gcagcagaaa ggttttgtgt tcgtaattct ggtgctcgaa cagtggttga aggaattggt 7500
   gatcatggat gcgagtacat gacagggggt actgtagtca tccttggtaa aacaggaaga 7560
   aattttgctg ctgggatgag tggaggcatc gcttatgttt atgatgttga tgggacattc 7620
   agtgtccgct gtaacaatga gttggttgat ctatatcatg tggaggaaga ggatgatgta 7680
   accactttga aaatgatgat agagcaacat cgacttcaca cagagagtgt cctggccaaa 7740
   gacatactct ctaaattcga cactcttctt ccaaaatttg taaaagtata cccaagggat 7800
   tataagaggg ttctagaaga aatgaaggca gaaaaagctg cagctaggcc tacaaaggaa 7860
   cctaaggtgg caaatggtgt ttctgtgaca actaaggtaa tatatgattt aagtaataat 7920
   tcttctgcat acctttttaa tttgaattta tagtagagat ggtgttctcc atttaggttt 7980
   atccatcaga aattgcaatg atacacattg ccactactgt gtgaacttct atttttgcct 8040
   aagtagatta actttgatct acagaaaata caaacagaga agtcatccag ccgaccaaca 8100
   cgtgttgcca acgccaaaaa gtaccggggt tttgtaacat atgagcgaga gggtgtttct 8160
   taccgtgatc caaatgagcg tgttaaagat tggaacgagg ttgccattga atcagttcca 8220
   gggccactgt taaacacaca gtctgctcgt tgtatggatt gtggcactcc tttctgtcat 8280
   caggtgaagc tgagttcatc ttttctattt aacattgatg attgtagttg gatttattgt 8340
   ggggttaagg attgatatgt ttagtcattg actacatatg acattgagat gctaagattt 8400
   catgattatt tgtacaggaa agctcaggtg ctggctgtcc tcttggaaat aagatcccag 8460
   agttcaatga attagttcac cagaatagat ggcgtgaagc attggatcgc ctactagaga 8520
   caaacaactt ccctgaattt actggacgtg tgtgccctgc tccttgtgag gggtcttgtg 8580
   ttcttggcat tattgagaac ccagtatcaa tcaaaagcat agaatgctcg attatagaca 8640
   aaggttttga agagggatgg atggtaccac gaccaccact tcaaagaaca gggtatgtct 8700
   tttttaccat ctagttcggc ttattccctt atttatttac ttctgtttca ttaataggta 8760
   acatttgagt tacactttct aatataaact gttcctttcg cagaaagaaa atcgctatag 8820
   ttggcagtgg tcctgctggt ttggctgccg ctgatcaact aaataaaatg ggccattttg 8880
   taactgtatt tgaacgttcg gatcgtatag gaggtcttat gatgtatgga gtaccaaaca 8940
   tgaagacaga caagattgga gttgttcagc gtcgtgtcaa tttaatggcc gaagagggtg 9000
   taacatttgt ggtgaatgct aatgtaggga gtgatccttt atactcaatt gaacgtctcc 9060
   attctgagaa caatgcagtt attttggctt gtggagctac aaaaccaagg taactaattg 9120
   gaacgaggat tttttttttc tagtttactc caagagtagc aacaatctca aaagaactga 9180
   catatatctt ttgacactaa ctgactaaca tcatgtggga tattcttgaa cagggacctc 9240
   agtattcctg gccgtgagct agctggagtt cattttgcca tggaatttct ccacgcaaat 9300
   accaaaagtt tgcttgatag caacctggag gatggaagat acatatctgc ccagggtaag 9360
   aaggtggtgg tcattggtgg tggagacaca ggcacagatt gcatcggtac gtctgttagg 9420
   catggttgca gcagcattgt aaatctggag cttctcacca agccaccaag caagagagct 9480
   tctgacaacc cctggcccca ggtaaatgca aaaaaaaaaa aactggatat ttccctgcac 9540
   atgtgaattt ggccattcca tttccagctt ggaaagttct taagtcatca ttctgttttt 9600
   gcatgctgtg gcctagagtc ttccgagtgg actatgggca ccaggaagca tctaccaaat      9660
   ttggaaatga tccaagaact tactaagtct taaccaagcg tttcattggg tgatgaagat      9720
   ggaaaattga aggcccttga ggtggtgcgc gtgaagtggg agaaagtaga tggaaaattc      9780
   cagttcaagg agattgaagg atcacaagag atcatcgagg cagaccttgt cctccttgcc      9840
   atgggattcc tgggccctga agaggttagt tatgcagact ctgaactact gtcacgcctg      9900
   agttttatct tgatgataaa tgttggacaa acaactcacc gttttatttc tctgcagaac      9960
   atcgctgaca aactgggttt agagaaagac aaccgttcca acttcaaagc tcaattcgga     10020
   cacttcggga ccagtgtgga tggcgttttt gccgctgggg attgcaggcg cgggcaatcg     10080
   ctggttgttt gggccatcac cgaagggcgt gaagctgctg ctgcagtaga taagtacttg     10140
   tcaagggatg aacaaaatgt cgcaggcctt aca                                  10173
<210> 3
   <211> 8964
   <212> DNA
   <213> Triticum aestivum
<220>
   <221> misc_feature
   <222> (913)..(913)
   <223> n is a, c, g, or t
<220>
   <221> misc_feature
   <222> (959)..(959)
   <223> n is a, c, g, or t
<220>
   <221> misc_feature
   <222> (1150)..(1150)
   <223> n is a, c, g, or t
<220>
   <221> misc_feature
   <222> (2562)..(2562)
   <223> n is a, c, g, or t
<220>
   <221> misc_feature
   <222> (3476)..(3476)
   <223> n is a, c, g, or t
<220>
   <221> misc_feature
   <222> (5868)..(5868)
   <223> n is a, c, g, or t
<220>
   <221> misc_feature
   <222> (5898)..(5898)
   <223> n is a, c, g, or t
<220>
   <221> misc_feature
   <222> (5939)..(5939)
   <223> n is a, c, g, or t
<400> 3
   gaggaggctc tcaattgtat ctatccgggc cgcgctgaat cttcagcgtg gaggagagag      60
   agatttctac atgtgctctc tatcttcaag gtccttactt cttctgaatt gcattttcac     120
   tacaaactga tgaggttgag atcagggagc ccaattaagc tcctgacttt aatggaacat     180
   taacaacaca aaagttgata ggaaagctga ctgggaaaaa caatcctggg ctatctctta     240
   agcattagaa atctgtaatg taaggacatt tcatttttgc tgtattcatg tgagtaatta     300
   ttgttaaatc ttctattgtg gttttccagg accattgttt acaagggcca acttatgcca     360
   tcccagctcc aagggtacta ctatgcggat ataggtcatg aaaacttctc cagttatatg     420
   gctctggtaa atacgaaacc ttgatactct tacaacaaaa gtcatggtcg tagtcagcgg     480
   atgggaatca ataatagcaa gccagttgta agatcaagac tcgctcctgt gcagacatca     540
   aatttgagct gtctattcat atgctatttc agtcctgctt cgtgatgcct ttaagtgaag     600
   tactagaaac ttctcttagt tgatacccga taaccatgtt gcagagtcca gctgcagttc     660
   tgctatggcc gactgcttgc cgactcttca ggatgtgacc gaagaaattg tgtttggttt     720
   gaggtagctt agggctccca actgttttgc atgaaattat acgcagctga cgtattttta     780
   caaaattcgt tcttcacaag gaagttttgt tttgaatatc tgcaaaagat gtttataata     840
   tgatttctaa aagtcatatc ttgaaacagg ttcactcaag gttctccacc aacaccttcc     900
   ccagctggga ccntgcacag ccaatgcgtg tcttaggcca caatggagag atcaatacnc     960
   tcaaagggaa caaaaactgg taatattatt gtcaaatctt ttcctctttg ctttaacctt    1020
   ttcttggcag atgcaactat ctcaatatgc acgttgtata tatggcagga tgaaagctcg    1080
   tgagggtctc ttggagtgtg agaagcttgg cctatcacag gatgaaatgt caaagattct    1140
   tccaatagtn gatgccactt cttcagattc aggtccgcag tctaatatgc agcatgaatt    1200
   ccacttggca aataaaaatt catctcggtt ttgtgagtta ctaagtgtga aaaaacatgt    1260
   gaattctagg tgcatttgat ggtgttctcg agctccttat ccgtggtgga agaagcctgc    1320
   cagaagctgt gatgatgatg atccctgagg cgtggcagaa tgatgtaaac atggaacctg    1380
   ataagaaagc tctgtacgag ttcttgtcag cccttatgga gccttgggat ggacctgctc    1440
   tcatatcttg taaaaaactt aaacccgttt cttttttctg atcatacttc taactgatta    1500
   cttaagctaa catgggtaca acctgtagtt accgatggcc gctaccttgg agctaccctg    1560
   gatcgcaatg gccttaggcc tggtcgattt tatgtgaccc acagtggacg tgtggtcatg    1620
   ggtagtgaag ttggtgttgt ggatattcct gcccaagatg tgttgagaaa gggtcggctt    1680
   aaccctggaa tgatgttact cgttgacttc gacaaccata ctgtagtaga tgatgaagca    1740
   ctcaaggcac agtactctaa agctcacccg tatggagaat ggctcaaacg acaaaagatg    1800
   tacctcaaag acattgtaga atctgtccca gaaactgaca gagttgctcc aagcatttct    1860
   ggttctatta cggtaagtac ttacgtggtc caacttcctc attcactggg tagtttggtg 1920
   acgaaaattg gtaaaccata agtaacatgg tgattttaat attcttttac aatttggtgt 1980
   agcaaacaaa tgagaacaag gaatgtgtag gcatcaatgc aattgtgact ccactaaagg 2040
   cgtttgggta agcaaggatt aaacattgtc aactatcact catgtttcag tttctgctgg 2100
   ctaatgatcc aaccgattaa ctacaggtac acattggaag ccctagaaat gttgctgctg 2160
   ccaatggcta aagatggagt ggaagctctt ggatcaatgg gaaatgatgc acccctagca 2220
   gtgatgtcaa acagagagaa gctgactttt gagtacttca agcagatgtt tgcacaagta 2280
   acaaaccctc caatcgatcc aattagggag aagattgtta catctatgga atgtatgatt 2340
   gggccagaag gagatttgct ggaaataacc gaaaagcaat gcaaccgcct tgcacttaaa 2400
   ggtcctttgg tgtcaatgga tgaaatggaa tctatcaaga agatgaacta ccgtggttgg 2460
   cgcagcaagg tgctcgacat aacttatccg aagaattctg gaaggaaggg cttggaagaa 2520
   actttggata gaatttgtgc tgaagcccgg gaagctatac gngagggata caaaatttta 2580
   gttctttcag acagaggtca gtgacttatt cactttatac ttgcatccca ttgtttgatg 2640
   gtacaaatta ctcataagtt gacaaaaaat tctagcagat gggtcaggtg tgcacaattt 2700
   attatcgtta gcttccagca aacatatcac atcaccatat agtaacttaa atatgatcat 2760
   aattttgttt gatagtcaat tgtttgcagt gtaatgtgtt gtatgaagga cattcttact 2820
   ttgtcacctt ggacataatt ggaatgtata ttaagtttat tatgcaactc aacatcttcc 2880
   aatactgaaa tttgttgtat tttcttgatt caggattttc ttcagaccgt gttgctgtca 2940
   gttccctctt agcagttgga gcagtacatc aacaccttgt tgcaaatctt gagaggacac 3000
   gcgtaggatt attggttgag tctgctgaac ctcgtgaagt gcaccatttc tgtacactcg 3060
   ttggatttgg tgcagatgct atatgccctt atttggccat tgaagcaatt tggtgcttgc 3120
   agactgatgg gaagattccc cctaccgact caaaggagga acttgtcgaa aaatattttt 3180
   atgcttccat ctatggaatg atgaaggttc ttgcaaagat gggaatatcc acccttgcat 3240
   cttacaaagg ggcacagatt tttgaagctc ttggactttc ttctgaagtg attcacaagt 3300
   gttttgaagg cactcctagc agaattgagg gtgcaacgtt cgaaatgctt gcacgtgatg 3360
   ctctccgtct tcacgagttg gcattcccat caagaacacc tccgcctggc agtgcagatg 3420
   caaaagccct tcccaatcca ggggattacc actggaggaa aaatggtgaa gtccanctaa 3480
   atgatcctct tgcaatggca aaattacaag aagcagctaa agtaaacagc cgggaagcat 3540
   acaaagaata ttctaagcga attcaagagc ttaacaaggc gtgcaatctg cgtggtatgc 3600
   tgaaatttat agatagcact agcaagattt ctttggatga ggttgaacct gcaagtgaga 3660
   tagtgaaacg cttttgtact ggggcaatga gttatggatc tatttcgttg gaggcacata 3720
   ctgcccttgc tgtggccatg aacaaactgg gaggcaaatc taacacaggt atttcatgca 3780
   tatgtgacat tttttaccct ttcgctggtt gttacccttc tgatgaaatc atgtatagta     3840
   gctttacttg cacatcaact cttagagcct tgttgcataa catatttttc ttatttttgc     3900
   tgcagtagtg tagtgtctat gtttctactg tttaaatatg ccctcgaagt tggtacttta     3960
   tacatgacca tgtctagaaa tgcactactt attttgatgc tatcacatga aaactatact     4020
   ctcatagcag tacaagtatt tatcttcctg ttgatgttga atgcacttct gtgaaaacta     4080
   tactctcata gtagtgtcta tgtttctaca agtatttatc acatgaaaac tatactcaca     4140
   taaaaccatt tcatcttcct gttgatgttg aatgcacttc tgtgaacaaa ttatcccagt     4200
   tcatagcaag agttaacttc taagctttta tctatctgtt gatgttgaat gcacttctgg     4260
   tagcaaatta tcctaattcc tagcaagatt taacttctgc ttgcgtagaa acccagacag     4320
   cttgcacgca ttattcttct ttggactgtt tgtattatag atattttctg cccacacagt     4380
   taatattata ttgccaaatc tttgtattta ttttcactcc taaactaaac tcattttacc     4440
   catctgctaa tttaggggag ggaggggagc agccttctcg tatggagcct cttcctgatg     4500
   gttcgatgaa tccaaaacga agtgcaatca agcaagttgc cagtggacga tttggagttt     4560
   ccagctatta tctgactaat gcagatgagc tgcagataaa aatggctcag gtacttgaca     4620
   atgctgtgca ttgcgcaatc actggaatga tctcagttta tagaaagcaa ctgtattcaa     4680
   cgtcctgtgg ttggcattta ttcttttttt gtatcgtata tgtatagggt gctaagcctg     4740
   gtgaaggtgg tgagcttcca ggtcacaagg ttatcggtga cattgcagtt accagacatt     4800
   ctacagctgg tgttgggctt attagtccac ctcctcacca tgatatatat tctatcgagg     4860
   atcttgcaca gcttatccat gaccttaagg tacattacct gatgctcttc tatagccaag     4920
   gcatctgcct atttatgtat ggttgttagt tgctacttct agattatatt ggttatctaa     4980
   acgctgtgat ggtctaaccc tcctgagaag ctagaatcac ttgctaaact ggtacaagtg     5040
   atcctgcaca tatgcatgtg tctccatttg gggtttaccc ctcctttttg aaaaactcat     5100
   tttgcaaatg ttacctgctg atgtagtgat attcatgcct gttttctctc tgcagaattc     5160
   aaatcctcaa gctcgaatca gcgtgaagct agtgtctgag gctggtgttg gagttgtagc     5220
   tagcggtgtt gtaaaaggac acgcagacca tgttcttatt tctggccatg acggtggcac     5280
   cggtgcatca agatggacag gtatcaagaa tgctggactt ccatggggaa ctaggattgg     5340
   ctgagacaca tcaaacttta gtggcaaatg ggcttcgtgg tcgagctgtc ctacaaacag     5400
   atggccaatt aaaaactggt agagatgttg ctgtggcgtg cttacttggt gcagaggaat     5460
   ttggtttcag cactgctcca ctgatcacac ttggctgcat tatgatgcgg aagtgccata     5520
   ccaatacttg ccctgttggt atagccactc aagatccagt gctccgagaa aaatttgctg     5580
   gagaaccaga gcatgtcatt aactttttct tcatgcttgc tgaggagcta agagaaatta     5640
   tggctcagct tggcctccgt acaattaatg aaatggttgg acgctctgat atgcttgaag     5700
   ttgatccaga agtagttaag agcaatgaga aacttgaaaa tattgatctc tcattgatct     5760
   taaaaccagc tgcagagatt cgtcctgggg ctgctcagta ctgtgttgaa aagcaagacc 5820
   atggccttga catggctttg gataacaaac ttatagcttt atcaaggnct gcacttgaaa 5880
   aggaagttcg tgttttcntt gagactccaa tcaagaacac taatcgggca gtaggcacna 5940
   cacttagcca tgaagtcaca aaacgttatc acatgaaggg attagatcct ggaaccattc 6000
   atgtgaaact cactggaagt gctggtcaga gttttggtgc ttttctctgt cctggaataa 6060
   cccttgagct tgaaggagac agcaatgatt acgttggcaa aggattatct ggtggaaaga 6120
   ttgttgtgta cccacccagg aacagtacat ttagtgcaga agacaatatt gtcattggta 6180
   atgtggccct gtatggtgct accaagggag aagcatactt caatggaatg gcagcagaaa 6240
   ggttttgtgt tcgtaattct ggtgctcgga cagtggttga aggaattggt gatcatggat 6300
   gcgagtacat gacagggggt actgtagtca tccttggtaa aacaggaaga aattttgctg 6360
   ctgggatgag tggaggcatc gcttatgttt atgatgttga tgggacattc agtgtccgct 6420
   gtaacaatga gttggttgat ctatatcatg tggaggaaga ggatgatgta accactttga 6480
   aaatgatgat agagcaacat cgacttcaca cagagagtgt cctggccaaa gacatactct 6540
   ctaaattcga cactcttctt ccaaaatttg taaaagtata cccaagggat tataagaggg 6600
   ttctagaaga aatgaaggca gaaaaagctg cagctaggcc tacaaaggaa cctaaggtgg 6660
   caaatggtgt ttctgtgaca actaaggtaa tatatgattt aagtaataat tcttctgcat 6720
   acctttttaa tttgaattta tagtagagat ggtgttctcc atttaggttt atccatcaga 6780
   aattgcaatg atacacattg ccactactgt gtgaacttct atttttgcct aagtagatta 6840
   actttgatct acagaaaata caaacagaga agtcatccag ccgaccaaca cgtgttgcca 6900
   acgccaaaaa gtaccggggt tttgtaacat atgagcgaga gggtgtttct taccgtgatc 6960
   caaatgagcg tgttaaagat tggaacgagg ttgccattga atcagttcca gggccactgt 7020
   taaacacaca gtctgctcgt tgtatggatt gtggcactcc tttctgtcat caggtgaagc 7080
   tgagttcatc ttttctattt aacattgatg attgtagttg gatttattgt ggggttaagg 7140
   attgatatgt ttagtcattg gctacatatg acattgagat gctaagattt catgattatt 7200
   tgtacaggaa agctcaggtg ctggctgtcc tcttggaaat aagatcccag agttcaatga 7260
   attagttcac cagaatagat ggcgtgaagc attggatcgc ctactagaga caaacaactt 7320
   ccctgaattt actggacgtg tgtgccctgc tccttgtgag gggtcttgtg ttcttggcat 7380
   tattgagaac ccagtatcaa tcaaaagcat agaatgctcg attatagaca aaggttttga 7440
   agagggatgg atggtaccac gaccaccact tcaaagaaca gggtatgtct tttttaccat 7500
   ctagttcggc ttattccctt atttatttac ttctgtttca ttaataggta acatttgagt 7560
   tacactttct aatataaact gttcctttcg cagaaagaaa atcgctatag ttggcagtgg 7620
   tcctgctggt ttggctgccg ctgatcaact aaataaaatg ggccattttg taactgtatt 7680
   tgaacgttcg gatcgtatag gaggtcttat gatgtatgga gtaccaaaca tgaagacaga     7740
   caagattgga gttgttcagc gtcgtgtcaa tttaatggcc gaagagggta taacatttgt     7800
   ggtgaatgct aatgtaggga gtgatccttt atactcaatt gaacgtctcc gttctgagaa     7860
   caatgcagtt attttggctt gtggagctac aaaaccaagg taactaattg gaacgaggat     7920
   tttttttttc tagtttactc caagagtagc aacaatctca aaagaactga catatatctt     7980
   ttgacactaa ctgactaaca tcatgtggga tattcttgaa cagggacctc agtattcctg     8040
   gccgtgagct agctggagtt cattttgcca tggaatttct ccacgcaaat accaaaagtt     8100
   tgcttgatag caacctggag gatggaagat acatatctgc ccagggtaag aaggtggtgg     8160
   tcattggtgg tggagacaca ggcacagatt gcatcggtac gtctgttagg catggttgca     8220
   gcagcattgt aaatctggag cttctcacca agccaccaag caagagagct tctgacaacc     8280
   cctggcccca ggtaaatgca aaaaaaaaaa actggatatt tccctgcaca tgtgaatttg     8340
   gccattccat ttccagcttg gaaagttctt aagtcatcat tctgtttttg catgcagtgg     8400
   cctagagtct tccgagtgga ctatgggcac caggaagcat ctaccaagtt tggaaatgat     8460
   ccaagaactt acgaagtctt aaccaagcgt ttcattggtg atgaagatgg aaaattgaag     8520
   gcccttgagg tggtgcgcgt gaagtgggag aaagtagatg gaaaattcca gttcaaggag     8580
   attgaaggat cacaagagat catcgaggca gaccttgtcc tccttgccat gggattcctg     8640
   ggccctgaag aggttagtta tgcagactct gaactactgt cacgcctgag ttttatcttg     8700
   atgataaatg ttggacaaac aactcaccgt tttatttctc tgcagaacat cgctgacaaa     8760
   ctgggtttag agaaagacaa ccgttccaac ttcaaagctc aattcggaca cttcgggacc     8820
   agtgtggatg gcgtttttgc cgctggggat tgcaggcgcg ggcaatcgct ggttgtttgg     8880
   gccatcaccg aagggcgtga agctgctgct gcagtagata agtacttgtc aagggatgaa     8940
   caaaatgtcg caggccttac atag                                            8964
<210> 4
   <211> 9113
   <212> DNA
   <213> Triticum aestivum
<220>
   <221> misc_feature
   <222> (1140)..(1140)
   <223> n is a, c, g, or t
<220>
   <221> misc_feature
   <222> (1155)..(1155)
   <223> n is a, c, g, or t
<220>
   <221> misc_feature
   <222> (1220)..(1220)
   <223> n is a, c, g, or t
<220>
   <221> misc_feature
   <222> (1239)..(1239)
   <223> n is a, c, g, or t
<220>
   <221> misc_feature
   <222> (1294)..(1294)
   <223> n is a, c, g, or t
<220>
   <221> misc_feature
   <222> (1351)..(1351)
   <223> n is a, c, g, or t
<220>
   <221> misc_feature
   <222> (2648)..(2648)
   <223> n is a, c, g, or t
<220>
   <221> misc_feature
   <222> (2747)..(2747)
   <223> n is a, c, g, or t
<220>
   <221> misc_feature
   <222> (2772)..(2772)
   <223> n is a, c, g, or t
<220>
   <221> misc_feature
   <222> (2791)..(2791)
   <223> n is a, c, g, or t
<220>
   <221> misc_feature
   <222> (4426)..(4426)
   <223> n is a, c, g, or t
<220>
   <221> misc_feature
   <222> (4431)..(4431)
   <223> n is a, c, g, or t
<220>
   <221> misc_feature
   <222> (6016)..(6016)
   <223> n is a, c, g, or t
<220>
   <221> misc_feature
   <222> (6046)..(6046)
   <223> n is a, c, g, or t
<220>
   <221> misc_feature
   <222> (6087)..(6087)
   <223> n is a, c, g, or t
<220>
   <221> misc_feature
   <222> (7938)..(7938)
   <223> n is a, c, g, or t
<220>
   <221> misc_feature
   <222> (7962)..(7962)
   <223> n is a, c, g, or t
<220>
   <221> misc_feature
   <222> (8118)..(8118)
   <223> n is a, c, g, or t
<220>
   <221> misc_feature
   <222> (8190)..(8190)
   <223> n is a, c, g, or t
<400> 4
   atcctgagga ggctctcaat tgtatctatc cgggccgcgc tgaatcttca gcgtggagga      60
   gagagagatt tctacatgtg ctctctatct tcaaggtcct tacttcttct gaattgcatt     120
   ttcactacaa actgatgagg ttgagatcag ggagcccaat taagctcctg actttaatgg     180
   aacattaaca acacaaaagt tgataggaaa gctgactggg aaaaacaatc ctgggctatc     240
   tcttaagcat tagaaatctg taatgtaagg acatttcatt tttgctgtat tcatgtgagt     300
   aattattgtt aaatcttcta ttgtggtttt ccaggaccat tgtttacaag ggccaactta     360
   tgccatccca gctccaaggg tactactatg cggatatagg tcatgaaaac ttctccagtt     420
   atatggctct ggtaaatacg aaaccttgat actcttacaa caaaagtcat ggtcgtagtc     480
   agcggatggg aatcaataat agcaagccag ttgtaagatc aagactcgct cctgtgcaga     540
   catcaaattt gagctgtcta ttcatatgct atttcagtcc tgcttcgtga tgcctttaag     600
   tgaagtacta gaaacttctc ttagttgata cccgataacc atgttgcaga gtccagctgc     660
   agttctgcta tggccgactg cttgccgact cttcaggatg tgaccgaaga aattgtgttt     720
   ggtttgaggt agcttagggc tcccaactgt tttgcatgaa attatacgca gctgacgtat     780
   ttttacaaaa ttcgttcttc acaaggaagt tttgttttga atatctgcaa aagatgttta     840
   taatatgatt tctaaaagtc atatcttgaa acaggttcac tcaaggttct ccaccaacac     900
   cttccccagc tgggaccgtg cacagccaat gcgtgtctta ggccacaatg gagagatcaa     960
   tactctcaaa gggaacaaaa actggtaata ttattgtcaa atcttttcct ctttgcttta    1020
   accttttctt ggcagatgca actatctcaa tatgcacgtt gtatatatgg caggatgaaa    1080
   gctcgtgagg gtctcttgga gtgtgagaag cttggcctat cacaggatga aatgtcaaan    1140
   attcttccaa tagtngatgc cacttcttca gattcaggtc cgcagtctaa tatgcagcat    1200
   gaattccact tggcaaatan aaattcatct cggttttgng agttactaag tgtgaaaaaa    1260
   catgtgaatt ctaggtgcat ttgatggtgt tctngagctc cttatccgtg gtggaagaag    1320
   cctgccagaa gctgtgatga tgatgatccc ngaggcgtgg cagaatgatg taaacatgga    1380
   acctgataag aaagctctgt acgagttctt gtcagccctt atggagcctt gggatggacc    1440
   tgctctcata tcttgtaaaa aacttaaacc cgtttctttt ttctgatcat acttctaact 1500
   gattacttaa gctaacatgg gtacaacctg tagttaccga tggccgctac cttggagcta 1560
   ccctggatcg caatggcctt aggcctggtc gattttatgt aacccacagt ggacgtgtgg 1620
   tcatgggtag tgaagttggt gttgtggata ttcctgccca agatgtgttg agaaagggtc 1680
   ggcttaaccc tggaatgatg ttactcgttg acttcgacga ccatactgta gtagatgatg 1740
   aagcactcaa ggcacagtac tctaaagctc acccgtatgg agaatggctc aagcgacaaa 1800
   agatgtacct caaagacatt gtagaatctg tcccagaaac tgacagagtt gctccaagca 1860
   tttctggttc tattacggta agtacttacg tggtccaact tcctcattca ctgggtagtt 1920
   tggtgatgaa aattggtaaa ccataaggaa catggtgatt ttaatattct tttacaattt 1980
   ggtgtagcaa acaaatgaga acaaggaatg tgtaggcatc aatgcaattg tgactccact 2040
   aaaggcgttt gggtaagcaa ggattgaaca ttgtcaacta tcactcatgt ttcaatttct 2100
   gctggctaat gatccaaccg attaactaca ggtacacatt ggaagcccta gaaatgttgc 2160
   tgctgccaat ggctaaagat ggagtggaag ctcttggatc aatgggaaat gatgcacccc 2220
   tagcagtgat gtcaaacaga gagaagctga cttttgagta cttcaagcag atgtttgcac 2280
   aagtaacaaa ccctccaatc gatccaatta gggagaagat tgttacatct atggaatgta 2340
   tgattgggcc agaaggagat ttgctggaaa taaccgaaaa gcaatgcaac cgccttgcac 2400
   ttaaaggtcc tttggtgtca atggatgaaa tggaatctat caagaagatg aactaccgtg 2460
   gttggcgcag caaggtgctc gacataactt atccgaagaa ttctggaagg aagggcttgg 2520
   aagaaacttt ggatagaatt tgtgctgaag cccgggaagc tatacgcaag ggatacaaaa 2580
   ttttagttct ttcagacaga ggtcagtgac ttattcactt tatacttgca tcccattgtt 2640
   tgatggtnca aattactcat aagttgacaa aaaattctag cagatgggtc aggtgtgcac 2700
   aatttattat cgttagcttc cagcaaacat atcacatcac catatantaa cttaaatatg 2760
   atcataattt tntttgatag tcaattgttt ncagtgtaat gtgttgtatg aaggacattc 2820
   ttactttgtc accttggaca taattggaat gtatattaag tttattatgc aactcaacat 2880
   cttccaatac tgaaatttgt tgtattttct tgattcagga ttttcttcag accgtgttgc 2940
   tgtcagttcc ctcttagcag ttggagcagt acatcaacac cttgttgcaa atcttgagag 3000
   gacacgcgta ggattattgg ttgagtctgc tgaacctcgt gaagtgcacc atttctgtac 3060
   actcgttgga tttggtgcag atgctatatg cccttatttg gccattgaag caatttggtg 3120
   cttgcagact gatgggaaga ttccccctac cgactcaaag gaggaacttg tcgaaaaata 3180
   tttttatgct tccatctatg gaatgatgaa ggttcttgca aagatgggaa tatccaccct 3240
   tgcatcttac aaaggggcac agatttttga agctcttgga ctttcttctg aagtgattca 3300
   caagtgtttt gaaggcactc ctagcagaat tgagggtgca acgttcgaaa tgcttgcacg 3360
   tgatgctctc cgtcttcacg agttggcatt cccatcaaga acacctccgc ctggcagtgc 3420
   agatgcaaaa gcccttccca atccagggga ttaccactgg aggaaaaatg gtgaagtcca 3480
   tctaaatgat cctcttgcaa tggcaaaatt acaagaagca gctaaagtaa acagccggga 3540
   agcatacaaa gaatattcta agcgaattca agagcttaac aaggcgtgca atctgcgtgg 3600
   tatgctgaaa tttatagata gcactagcaa gatttctttg gatgaggttg aacctgcaag 3660
   tgagatagtg aaacgctttt gtactggggc aatgagttat ggatctattt cgttggaggc 3720
   acatactgcc cttgctgtgg ccatgaacaa actgggaggc aaatctaaca caggtatttc 3780
   atgcatatgt gacatttttt accctttggc tggttgttac ccttctgatg aaatcatgta 3840
   tagtagcttt acttgcacat caactcttag agccttgttg cataacatat ttttcttatt 3900
   tttgctgcag tagtgtagtg tctatgtttc tactgtttaa atatgccctc gaagttggta 3960
   ctttatacat gaccatgtct agaaatgcac tacttatttt gatgctatca catgaaaact 4020
   atactctcat atttacttgt ggacatactt gtactgcact tgtgtgtttg gttgcattct 4080
   cgtctcagca tagttccctc ttcatgcatg ctcaactcaa cacccctctt catgcatgct 4140
   ttcttcatgc atgcttctag tgtatttgtg ctaagctagt gtggactcat gcaccctcca 4200
   tacactctac caaacaccca aaagtgggtc gagaagggaa ctcttgggct catgcaccct 4260
   tcatacaccc taccaaacac acccttaatg cgctaagctt ttcatcttcc tgttgatgtt 4320
   gaatgcactt ctgtgaacaa attatcccag ttcatagcaa gagttaactt ctaagctttt 4380
   atctatctgt tgatgttgaa tgcacttctg gtagcaaatt atcctnattc ntagcaagat 4440
   ttaacttctg cttgcgtaga aacccagaca gcttgcacgc actattcttc tttggactgt 4500
   ttgtattaga tattttctgc ccacacagtt aatattatat tgccaaatct ttgtatttat 4560
   tttcgctcct aaactaaact cattttaccc atctgctaat ttaggggagg gaggggagca 4620
   gccttctcgt atggagcctc ttcctgatgg ttcgatgaat ccaaaacgaa gtgcaatcaa 4680
   gcaagttgcc agtggacgat ttggagtttc cagctattat ctgactaatg cagatgagct 4740
   gcagataaaa atggctcagg tacttgacaa tgctgtgcat tgcgcaatca ctggaatgat 4800
   ctcagtttat agaaagcaac tgtattcaac gtcctgtggt tggcatttat tctttttttg 4860
   tatcgtatat gtatagggtg ctaagcctgg tgaaggtggt gagcttccag gtcacaaggt 4920
   tatcggtgac attgcagtta ccagacattc tacagctggt gttgggctta ttagtccacc 4980
   tcctcaccat gatatatatt ctatcgagga tcttgcacag cttatccatg accttaaggt 5040
   acattacctg atgctcttct atagccaagg catctgccta tttatgtatg gttgttagtt 5100
   gctacttcta gattatattg gttatctaaa cgctgtgatg gtctaaccct cctgagaagc 5160
   tagaatcact tgctaaactg gtacaagtga tcctgcacat atgcatgtgt ctccatttgg 5220
   gggtttaccc tcctttttga aaaactcatt ttgcaaatgt tacctgctga tgtagtgata 5280
   ttcatgcctg ttttctctct gcagaattca aatcctcaag ctcgaatcag cgtgaagcta 5340
   gtgtctgagg ctggtgttgg agttgtagct agcggtgttg taaaaggaca cgcagaccat 5400
   gttcttattt ctggccatga cggtggcacc ggtgcatcaa gatggacagg tatcaagaat 5460
   gctggacttc catggggaac taggattggc tgagacacat caaactttag tggcaaatgg 5520
   gcttcgtggt cgagctgtcc tacaaacaga tggccaatta aaaactggta gagatgttgc 5580
   tgtggcgtgc ttacttggtg cagaggaatt tggtttcagc actgctccac tgatcacact 5640
   tggctgcatt atgatgcgga agtgccatac caatacttgc cctgttggta tagccactca 5700
   agatccagtg ctccgagaaa aatttgctgg agaaccagag catgtcatta actttttctt 5760
   catgcttgct gaggagctaa gagaaattat ggctcagctt ggcctccgta caattaatga 5820
   aatggttgga cgctctgata tgcttgaagt tgatccagaa gtagttaaga gcaatgagaa 5880
   acttgaaaat attgatctct cattgatctt aaaaccagct gcagagattc gtcctggggc 5940
   tgctcagtac tgtgttgaaa agcaagacca tgccttgaca tggctttgga taacaaactt 6000
   atagctttat caaggnctgc acttgaaaag gaagttcgtg ttttcnttga gactccaatc 6060
   aagaacacta atcgggcagt aggcacnaca cttagccatg aagtcacaaa acgttatcac 6120
   atgaagggat tagatcctgg aaccattcat gtgaaactca ctggaagtgc tggtcagagt 6180
   tttggtgctt ttctctgtcc tggaataacc cttgagcttg aaggagacag caatgattac 6240
   gttggcaaag gattatctgg tggaaagatt gttgtgtacc cacccaggaa cagtacattt 6300
   agtgcagaag acaatattgt cattggtaat gtggccctgt atggtgctac caagggagaa 6360
   gcatacttca atggaatggc agcagaaagg ttttgtgttc gtaattctgg tgctcgaaca 6420
   gtggttgaag gaattggtga tcatggatgc gagtacatga cagggggtac tgtagtcatc 6480
   cttggtaaaa caggaagaaa ttttgctgct gggatgagtg gaggcatcgc ttatgtttat 6540
   gatgttgatg ggacattcag tgtccgctgt aacaatgagt tggttgatct atatcatgtg 6600
   gaggaagagg atgatgtaac cactttgaaa atgatgatag agcaacatcg acttcacaca 6660
   gagagtgtcc tggccaaaga catactctct aaattcgaca ctcttcttcc aaaatttgta 6720
   aaagtatacc caagggatta taagagggtt ctagaagaaa tgaaggcaga aaaagctgca 6780
   gctaggccta caaaggaacc taaggtggca aatggtgttt ctgtgacaac taaggtaata 6840
   tatgatttaa gtaataattc ttctgcatac ctttttaatt tgaatttata gtagagatgg 6900
   tgttctccat ttaggtttat ccatcagaaa ttgcaatgat acacattgcc actactgtgt 6960
   gaacttctat ttttgcctaa gtagattaac tttgatctac agaaaataca aacagagaag 7020
   tcatccagcc gaccaacacg tgttgccaac gccaaaaagt accggggttt tgtaacatat 7080
   gagcgagagg gtgtttctta ccgtgatcca aatgagcgtg ttaaagattg gaacgaggtt 7140
   gccattgaat cagttccagg gccactgtta aacacacagt ctgctcgttg tatggattgt 7200
   ggcactcctt tctgtcatca ggtgaagctg agttcatctt ttctatttaa cattgatgat 7260
   tgtagttgga tttattgtgg ggttaaggat tgatatgttt agtcattggc tacatatgac 7320
   attgagatgc taagatttca tgattatttg tacaggaaag ctcaggtgct ggctgtcctc 7380
   ttggaaataa gatcccagag ttcaatgaat tagttcacca gaatagatgg cgtgaagcat 7440
   tggatcgcct actagagaca aacaacttcc ctgaatttac tggacgtgtg tgccctgctc 7500
   cttgtgaggg gtcttgtgtt cttggcatta ttgagaaccc agtatcaatc aaaagcatag 7560
   aatgctcgat tatagacaaa ggttttgaag agggatggat ggtaccacga ccaccacttc 7620
   aaagaacagg gtatgtcttt tttaccatct agttcggctt attcccttat ttatttactt 7680
   ctgtttcatt aataggtaac atttgagtta cactttctaa tataaactgt tcctttcgca 7740
   gaaagaaaat cgctatagtt ggcagtggtc ctgctggttt ggctgccgct gatcaactaa 7800
   ataaaatggg ccattttgta actgtatttg aacgttcgga tcgtatagga ggtcttatga 7860
   tgtatggagt accaaacatg aagacagaca agattggagt tgttcagcgt cgtgtcaatt 7920
   taatggccga agagggtnta acatttgtgg tgaatgctaa tntagggagt gatcctttat 7980
   actcaattga acgtctccgt tctgagaaca atgcagttat tttggcttgt ggagctacaa 8040
   aaccaaggta actaattgga acgaggattt tttttttcta gtttactcca agagtagcaa 8100
   caatctcaaa agaactgnca tatatctttt gacactaact gactaacatc atgtgggata 8160
   ttcttgaaca gggacctcag tattcctggn cgtgagctag ctggagttca ttttgccatg 8220
   gaatttctcc acgcaaatac caaaagtttg cttgatagca acctggagga tggaagatac 8280
   atatctgccc agggtaagaa ggtggtggtc attggtggtg gagacacagg cacagattgc 8340
   atcggtacgt ctgttaggca tggttgcagc agcattgtaa atctggagct tctcaccaag 8400
   ccaccaagca agagagcttc tgacaacccc tggccccagg taaatgcaaa aaaaaaaaaa 8460
   ctggatattt ccctgcacat gtgaatttgg ccattccatt tccagcttgg aaagttctta 8520
   agtcatcatt ctgtttttgc atgcagtggc ctagagtctt ccgagtggac tatgggcacc 8580
   aggaagcatc taccaagttt ggaaatgatc caagaactta cgaagtctta accaagcgtt 8640
   tcattggtga tgaagatgga aaattgaagg cccttgaggt ggtgcgcgtg aagtgggaga 8700
   aagtagatgg aaaattccag ttcaaggaga ttgaaggatc acaagagatc atcgaggcag 8760
   accttgtcct ccttgccatg ggattcctgg gccctgaaga ggttagttat gcagactctg 8820
   aactactgtc acgcctgagt tttatcttga tgataaatgt tggacaaaca actcaccgtt 8880
   ttatttctct gcagaacatc gctgacaaac tgggtttaga gaaagacaac cgttccaact 8940
   tcaaagctca attcggacac ttcgggacca gtgtggatgg cgtttttgcc gctggggatt 9000
   gcaggcgcgg gcaatcgctg gttgtttggg ccatcaccga agggcgtgaa gctgctgctg 9060
   cagtagataa gtacttgtca agggatgaac aaaatgtcgc aggccttaca tag 9113
<210> 5
   <211> 6432
   <212> DNA
   <213> Triticum aestivum
<400> 5
   atgccgacgg cgcaggggga ttggtttgaa gccgcggcgc cgccgggcgg ccgcagggcc      60
   cgccgcagcc agtctgcctc ggcgccgtgc cgctccgcaa ggcaggcgca cggcgccatg     120
   tccctggatg gcgggttcct cggcggcgcg cagcgcaccg aggaacgcgt cgcgccacgc     180
   ccgcctcggg ccgcggcgcg cgacgccgag tccatcaggc ccatgtctct gctacccgag     240
   agcagcattg ggctgtacga cccggcgttc gagcgtgact cgtgcggcgt tggcttcgtc     300
   gccgagctgt cgggcgttga caaccgggcg accgtcgtcg atgccattca gatgcttgaa     360
   agaatggcac accgaggtgc ctgcggctgt gagaaaaaca ctggtgatgg tgccggcatt     420
   ctcgttgctc taccacacac cttcttccga gaggtgacaa aggatgccgg tttcgagtta     480
   ccgccaccag gtgagtatgc tgttggaatg gtcttcctgc caaccgatga gaagcgccgc     540
   gagaggagca aaactgagtt tacaaaggtc gctgagtctc taggacattc gatacttggg     600
   tggcgccagg ttcccactga caattcagac ttgggccaag ctgcgctcga tactgaacca     660
   gccattgaac aggttttcct caccaagagt tcaaaatcga aggccgactt cgaacagcag     720
   gtgtttatcc tgaggaggct ctcaattgta tctatccggg ccgcgctgaa tcttcagcgt     780
   ggaggagaga gagatttcta catgtgctct ctatcttcaa ggaccattgt ttacaagggc     840
   caacttatgc catcccagct ccaagggtac tactatgcgg atataggtca tgaaaacttc     900
   tccagttata tggctctggt tcactcaagg ttctccacca acaccttccc cagctgggac     960
   cgtgcacagc caatgcgtgt cttaggccac aatggagaga tcaatactct caaagggaac    1020
   aaaaactgga tgaaagctcg tgagggtctc ttggagtgtg agaagcttgg cctatcacag    1080
   gatgaaatgt caaagattct tccaatagta gatgccactt cttcagattc aggtgcattt    1140
   gatggtgttc tcgagctcct tatccgtggt ggaagaagcc tgccagaagc tgtgatgatg    1200
   atgatccctg aggcgtggca gaatgatgta aacatggaac ctgataagaa agctctgtac    1260
   gagttcttgt cagcccttat ggagccttgg gatggacctg ctctcatatc ttttaccgat    1320
   ggccgctacc ttggagctac cctggatcgc aatggcctta ggcctggtcg attttatgta    1380
   acccacagtg gacgtgtggt catgggtagt gaagttggtg ttgtggatat tcctgcccaa    1440
   gatgtgttga gaaagggtcg gcttaaccct ggaatgatgt tactcgttga cttcgacaac    1500
   catactgtag tagatgatga agcactcaag gcacagtact ctaaagctca cccgtatgga    1560
   gaatggctca agcgacaaaa gatgtacctc aaagacattg tagaatctgt cccagaaact    1620
   gacagagttg ctccaagcat ttctggttct attacgcaaa caaatgagaa caaggaatgt    1680
   gtaggcatca atgcaattgt gactccacta aaggcgtttg ggtacacatt ggaagcccta    1740
   gaaatgttgc tgctgccaat ggctaaagat ggagtggaag ctcttggatc aatgggaaat    1800
   gatgcacccc tagcagtgat gtcaaacaga gagaagctga cttttgagta cttcaagcag    1860
   atgtttgcac aagtaacaaa ccctccaatc gatccaatta gggagaagat tgttacatct 1920
   atggaatgta tgattgggcc agaaggagat ttgctggaaa taaccgaaaa gcaatgcaac 1980
   cgccttgcac ttaaaggtcc tttggtgtca atggatgaaa tggaatctat caagaagatg 2040
   aactaccgtg gttggcgcag caaggtgctc gacataactt atccgaagaa ttctggaagg 2100
   aagggcttgg aagaaacttt ggatagaatt tgtgctgaag cccgggaagc tatacgcgag 2160
   ggatacaaaa ttttagttct ttcagacaga ggattttctt cagaccgtgt tgctgtcagt 2220
   tccctcttag cagttggagc agtacatcaa caccttgttg caaatcttga gaggacacgc 2280
   gtaggattat tggttgagtc tgctgaacct cgtgaagtgc accatttctg tacactcgtt 2340
   ggatttggtg cagatgctat atgcccttat ttggccattg aagcaatttg gtgcttgcag 2400
   actgatggga agattccccc taccgactca aaggaggaac ttgtcgaaaa atatttttat 2460
   gcttccatct atggaatgat gaaggttctt gcaaagatgg gaatatccac ccttgcatct 2520
   tacaaagggg cacagatttt tgaagctctt ggactttctt ctgaagtgat tcacaagtgt 2580
   tttgaaggca ctcctagcag aattgagggt gcaacgttcg aaatgcttgc acgtgatgct 2640
   ctccgtcttc acgagttggc attcccatca agaacacctc cgcctggcag tgcagatgca 2700
   aaagcccttc ccaatccagg ggattaccac tggaggaaaa atggtgaagt ccatctaaat 2760
   gatcctcttg caatggcaaa attacaagaa gcagctaaag taaacagccg ggaagcatac 2820
   aaagaatatt ctaagcgaat tcaagagctt aacaaggcgt gcaatctgcg tggtatgctg 2880
   aaatttatag atagcactag caagatttct ttggatgagg ttgaacctgc aagtgagata 2940
   gtgaaacgct tttgtactgg ggcaatgagt tatggatcta tttcgttgga ggcacatact 3000
   gcccttgctg tggccatgaa caaactggga ggcaaatcta acacagggga gggaggggag 3060
   cagccttctc gtatggagcc tcttcctgat ggttcgatga atccaaaacg aagtgcaatc 3120
   aagcaagttg ccagtggacg atttggagtt tccagctatt atctgactaa tgcagatggg 3180
   ctgcagataa aaatggctca gggtgctaag cctggtgaag gtggtgagct tccaggtcac 3240
   aaggttatcg gtgacattgc agttaccaga cattctacag ctggtgttgg gcttattagt 3300
   ccacctcctc accatgatat atattctatc gaggatcttg cacagcttat ccatgacctt 3360
   aagaattcaa atcctcaagc tcgaatcagc gtgaagctag tgtctgaggc tggtgttgga 3420
   gttgtagcta gcggtgttgt aaaaggacac gcagaccatg ttcttatttc tggccatgac 3480
   ggtggcaccg gtgcatcaag atggacaggt atcaagaatg ctggacttcc atgggaacta 3540
   ggattggctg agacacatca aactttagtg gcaaatgggc ttcgtggtcg agctgtccta 3600
   caaacagatg gccaattaaa aactggtaga gatgttgctg tggcgtgctt acttggtgca 3660
   gaggaatttg gtttcagcac tgctccactg atcacacttg gctgcattat gatgcggaag 3720
   tgccatacca atacttgccc tgttggtata gccactcaag atccagtgct ccgagaaaaa 3780
   tttgctggag aaccagagca tgtcattaac tttttcttca tgcttgctga ggagctaaga 3840
   gaaattatgg ctcagcttgg cctccgtaca attaatgaaa tggttggacg ctctgatatg 3900
   cttgaagttg atccagaagt agttaagagc aatgagaaac ttgaaaatat tgatctctca 3960
   ttgatcttaa aaccagctgc agagattcgt cctggggctg ctcagtactg tgttgaaaag 4020
   caagaccatg gccttgacat ggctttggat aacaaactta tagctttatc aagggctgca 4080
   cttgaaaagg aagttcgtgt tttcattgag actccaatca agaacactaa tcgggcagta 4140
   ggcaccacac ttagccatga agtcacaaaa cgttatcaca tgaagggatt agatcctgga 4200
   accattcatg tgaaactcac tggaagtgct ggtcagagtt ttggtgcttt tctctgtcct 4260
   ggaataaccc ttgagcttga aggagacagc aatgattacg ttggcaaagg attatctggt 4320
   ggaaagattg ttgtgtaccc acccaggaac agtacattta gtgcagaaga caatattgtc 4380
   attggtaatg tggccctgta tggtgctacc aagggagaag catacttcaa tggaatggca 4440
   gcagaaaggt tttgtgttcg taattctggt gctcgaacag tggttgaagg aattggtgat 4500
   catggatgcg agtacatgac agggggtact gtagtcatcc ttggtaaaac aggaagaaat 4560
   tttgctgctg ggatgagtgg aggcatcgct tatgtttatg atgttgatgg gacattcagt 4620
   gtccgctgta acaatgagtt ggttgatcta tatcatgtgg aggaagagga tgatgtaacc 4680
   actttgaaaa tgatgataga gcaacatcga cttcacacag agagtgtcct ggccaaagac 4740
   atactctcta aattcgacac tcttcttcca aaatttgtaa aagtataccc aagggattat 4800
   aagagggttc tagaagaaat gaaggcagaa aaagctgcag ctaggcctac aaaggaacct 4860
   aaggtggcaa atggtgtttc tgtgacaact aagaaaatac aaacagagaa gtcatccagc 4920
   cgaccaacac gtgttgccaa cgccaaaaag taccggggtt ttgtaacata tgagcgagag 4980
   ggtgtttctt accgtgatcc aaatgagcgt gttaaagatt ggaacgaggt tgccattgaa 5040
   tcagttccag ggccactgtt aaacacacag tctgctcgtt gtatggattg tggcactcct 5100
   ttctgtcatc aggaaagctc aggtgctggc tgtcctcttg gaaataagat cccagagttc 5160
   aatgaattag ttcaccagaa tagatggcgt gaagcattgg atcgcctact agagacaaac 5220
   aacttccctg aatttactgg acgtgtgtgc cctgctcctt gtgaggggtc ttgtgttctt 5280
   ggcattattg agaacccagt atcaatcaaa agcatagaat gctcgattat agacaaaggt 5340
   tttgaagagg gatggatggt accacgacca ccacttcaaa gaacaggaaa gaaaatcgct 5400
   atagttggca gtggtcctgc tggtttggct gccgctgatc aactaaataa aatgggccat 5460
   tttgtaactg tatttgaacg ttcggatcgt ataggaggtc ttatgatgta tggagtacca 5520
   aacatgaaga cagacaagat tggagttgtt cagcgtcgtg tcaatttaat ggccgaagag 5580
   ggtgtaacat ttgtggtgaa tgctaatgta gggagtgatc ctttatactc aattgaacgt 5640
   ctccattctg agaacaatgc agttattttg gcttgtggag ctacaaaacc aagggacctc 5700
   agtattcctg gccgtgagct agctggagtt cattttgcca tggaatttct ccacgcaaat 5760
   accaaaagtt tgcttgatag caacctggag gatggaagat acatatctgc ccagggtaag 5820
   aaggtggtgg tcattggtgg tggagacaca ggcacagatt gcatcggtac gtctgttagg 5880
   catggttgca gcagcattgt aaatctggag cttctcacca agccaccaag caagagagct 5940
   tctgacaacc cctggcccca gtggcctaga gtcttccgag tggactatgg gcaccaggaa 6000
   gcatctacca aatttggaaa tgatccaaga acttactaag tcttaaccaa gcgtttcatt 6060
   ggtgatgaag atggaaaatt gaaggccctt gaggtggtgc gcgtgaagtg ggagaaagta 6120
   gatggaaaat tccagttcaa ggagattgaa ggatcacaag agatcatcga ggcagacctt 6180
   gtcctccttg ccatgggatt cctgggccct gaagagaaca tcgctgacaa actgggttta 6240
   gagaaagaca accgttccaa cttcaaagct caattcggac acttcgggac cagtgtggat 6300
   ggcgtttttg ccgctgggga ttgcaggcgc gggcaatcgc tggttgtttg ggccatcacc 6360
   gaagggcgtg aagctgctgc tgcagtagat aagtacttgt caagggatga acaaaatgtc 6420
   gcaggcctta ca 6432
<210> 6
   <211> 2167
   <212> PRT
   <213> Oryza sativa
<400> 6
   Met Ser Ala Ala Gln Gly Met Ala Tyr Lys Leu Arg Thr Asp Ala Ala
   1               5                   10                  15
   Pro Thr Gly Ala Gly Arg Arg Ala Arg Arg Ser His Ser Ser Val Ala
               20                  25                  30
   Ala Pro Tyr Arg Ala Ala Arg Leu Val Gln Gly Gly Val Ser Ile Glu
           35                  40                  45
   Gly Gly Leu Val Gly Gly Cys Gln Leu Thr Glu Glu Arg Val Ala Ala
       50                  55                  60
   Arg Pro Pro Arg Ala Ala Ala Arg Asp Ala Glu Pro Val Arg Pro Leu
   65                  70                  75                  80
   Ser Thr Leu Pro Glu Ser Ser Ile Gly Leu Tyr Asp Pro Ser Arg Glu
                   85                  90                  95
   Arg Asp Ser Cys Gly Val Gly Phe Val Ala Glu Leu Ser Gly Asp Tyr
               100                 105                 110
   Lys Arg Ala Thr Val Asn Asp Ala Leu Glu Met Leu Glu Arg Met Ala
           115                 120                 125
   His Arg Gly Ala Cys Gly Cys Glu Lys Asn Thr Gly Asp Gly Ala Gly
   130 135 140
   Ile Leu Val Ala Leu Pro His Asn Phe Phe Arg Glu Val Thr Lys Asp
   145 150 155 160
   Ala Gly Phe Glu Leu Pro Gln Pro Gly Glu Tyr Ala Val Gly Met Val
   165 170 175
   Phe Leu Pro Ile Asp Glu Lys Arg Arg Glu Arg Ser Lys Ala Glu Phe
   180 185 190
   Gln Lys Val Ala Glu Ser Leu Gly His Val Ile Leu Gly Trp Arg Arg
   195 200 205
   Val Pro Thr Asp Asn Ser Asp Leu Gly Glu Ser Ala Leu Gln Thr Glu
   210 215 220
   Pro Val Ile Glu Gln Val Phe Leu Thr Lys Ser Ser Ser Ser Glu Ala
   225 230 235 240
   Asp Phe Glu Gln Gln Leu Tyr Ile Leu Arg Arg Leu Ser Ile Leu Ser
   245 250 255
   Ile Arg Ala Ala Leu Asn Leu Arg Arg Gly Gly Lys Arg Asp Phe Tyr
   260 265 270
   Met Cys Ser Leu Ser Ser Arg Thr Ile Val Tyr Lys Gly Gln Leu Lys
   275 280 285
   Pro Cys Gln Leu Lys Gly Tyr Tyr Tyr Ala Asp Leu Gly His Glu Asn
   290 295 300
   Phe Thr Ser Tyr Met Ala Leu Val His Ser Arg Phe Ser Thr Asn Thr
   305 310 315 320
   Phe Pro Ser Trp Asp Arg Ala Gln Pro Met Arg Val Leu Gly His Asn
   325 330 335
   Gly Glu Ile Asn Thr Leu Lys Gly Asn Lys Asn Trp Met Lys Ala Arg
   340 345 350
   Glu Gly Leu Leu Glu Cys Glu Lys Leu Gly Leu Thr Lys Asp Gln Phe
   355 360 365
   Ser Lys Ile Leu Pro Ile Val Asp Ala Thr Ser Ser Asp Ser Gly Ala
   370 375 380
   Phe Asp Gly Val Leu Glu Leu Leu Ile Arg Gly Gly Arg Ser Leu Pro
   385 390 395 400
   Glu Ala Val Met Met Met Ile Pro Glu Ala Trp Gln Asn Asp Val Asn
                   405                 410                 415
   Met Glu Pro Glu Lys Lys Ala Leu Tyr Glu Phe Leu Ser Ala Leu Met
               420                 425                 430
   Glu Pro Trp Asp Gly Pro Ala Leu Ile Ser Phe Thr Asp Gly Arg Tyr
           435                 440                 445
   Leu Gly Ala Thr Leu Asp Arg Asn Gly Leu Arg Pro Gly Arg Phe Tyr
       450                 455                 460
   Val Thr His Ser Gly Arg Val Val Met Gly Ser Glu Val Gly Val Val
   465                 470                 475                 480
   Asp Val Pro Ser Lys Asp Val Leu Arg Lys Gly Arg Leu Asn Pro Gly
                   485                 490                 495
   Met Met Leu Leu Val Asp Phe Glu Asn His Thr Val Val Asp Asp Glu
               500                 505                 510
   Ala Leu Lys Ala Gln Tyr Ser Lys Ala His Pro Tyr Gly Glu Trp Leu
           515                 520                 525
   Lys Arg Gln Lys Ile Tyr Leu Lys Asp Ile Val Glu Ser Val Pro Glu
       530                 535                 540
   Thr Glu Arg Val Ala Pro Gly Ile Ser Gly Ser Leu Thr Gln Lys Asn
   545                 550                 555                 560
   Glu Lys Lys Glu His Ala Gly Val Asn Gly Ile Val Thr Pro Leu Lys
                   565                 570                 575
   Ala Phe Gly Tyr Thr Val Glu Ala Leu Glu Met Leu Leu Leu Pro Met
               580                 585                 590
   Ala Lys Asp Gly Val Glu Ala Leu Gly Ser Met Gly Asn Asp Thr Pro
           595                 600                 605
   Leu Ala Val Met Ser Asn Arg Glu Lys Leu Thr Phe Glu Tyr Phe Lys
       610                 615                 620
   Gln Met Phe Ala Gln Val Thr Asn Pro Pro Ile Asp Pro Ile Arg Glu
   625                 630                 635                 640
   Lys Ile Val Thr Ser Met Glu Cys Met Ile Gly Pro Glu Gly Asp Leu
                   645                 650                 655
   Leu Glu Thr Thr Glu Lys Gln Cys Asn Arg Leu Ala Leu Glu Gly Pro
               660                 665                 670
   Leu Val Ser Ile Asp Glu Met Glu Ala Ile Lys Lys Met Asn Tyr Arg
           675                 680                 685
   Gly Trp Arg Ser Lys Val Leu Asp Ile Thr Tyr Pro Lys Lys Ser Gly
       690                 695                 700
   Arg Lys Gly Leu Glu Glu Thr Leu Asp Arg Ile Cys Thr Glu Ala Arg
   705                 710                 715                 720
   Gly Ala Ile Lys Lys Gly Tyr Thr Val Leu Val Leu Ser Asp Arg Gly
                   725                 730                 735
   Phe Ser Ser Asp Arg Val Ala Val Ser Ser Leu Leu Ala Val Gly Ala
               740                 745                 750
   Val His Gln His Leu Val Ala Asn Leu Glu Arg Thr Arg Val Gly Leu
           755                 760                 765
   Leu Val Glu Ser Ala Glu Pro Arg Glu Val His His Phe Cys Thr Leu
       770                 775                 780
   Val Gly Phe Gly Ala Asp Ala Val Cys Pro Tyr Leu Ala Ile Glu Ala
   785                 790                 795                 800
   Ile Trp Cys Leu Gln Asn Asp Gly Lys Ile Pro Pro Asn Gly Asp Gly
                   805                 810                 815
   Lys Pro Tyr Ser Lys Glu Glu Leu Val Lys Lys Tyr Phe Tyr Ala Ser
               820                 825                 830
   Asn Tyr Gly Met Met Lys Val Leu Ala Lys Met Gly Ile Ser Thr Leu
           835                 840                 845
   Ala Ser Tyr Lys Gly Ala Gln Ile Phe Glu Ala Leu Gly Leu Ser Ser
       850                 855                 860
   Glu Val Ile Arg Lys Cys Phe Asp Gly Thr Pro Ser Arg Ile Glu Gly
   865                 870                 875                 880
   Ala Thr Phe Glu Met Leu Ala Arg Asp Ala Leu Arg Leu His Glu Leu
                   885                 890                 895
   Ala Phe Pro Ser Arg Ala Pro Pro Pro Gly Ser Ala Asp Ala Lys Ala
               900                 905                 910
   Leu Pro Asn Pro Gly Asp Tyr His Trp Arg Lys Asn Gly Glu Val His
   915 920 925
   Leu Asn Asp Pro Leu Ala Met Ala Lys Leu Gln Glu Ala Ala Arg Val
   930 935 940
   Asn Ser Arg Ala Ala Tyr Lys Glu Tyr Ser Arg Arg Ile Gln Glu Leu
   945 950 955 960
   Asn Lys Thr Cys Asn Leu Arg Gly Met Leu Lys Phe Lys Asp Thr Ala
   965 970 975
   Asp Met Ile Ser Val Asp Glu Val Glu Pro Ala Ser Glu Ile Val Lys
   980 985 990
   Arg Phe Val Thr Gly Ala Met Ser Tyr Gly Ser Ile Ser Leu Glu Ala
   995 1000 1005
   His Thr Ala Leu Ala Met Ala Met Asn Lys Leu Gly Gly Lys Ser
   1010 1015 1020
   Asn Thr Gly Glu Gly Gly Glu Gln Pro Ser Arg Met Glu Pro Leu
   1025 1030 1035
   Ala Asn Gly Ser Met Asn Pro Lys Arg Ser Ala Ile Lys Gln Val
   1040 1045 1050
   Ala Ser Gly Arg Phe Gly Val Ser Ser Tyr Tyr Leu Thr Asn Ala
   1055 1060 1065
   Asp Glu Leu Gln Ile Lys Met Ala Gln Gly Ala Lys Pro Gly Glu
   1070 1075 1080
   Gly Gly Glu Leu Pro Gly His Lys Val Ile Gly Asp Ile Ala Val
   1085 1090 1095
   Thr Arg His Ser Thr Ala Gly Val Gly Leu Ile Ser Pro Pro Pro
   1100 1105 1110
   His His Asp Ile Tyr Ser Ile Glu Asp Leu Ala Gln Leu Ile His
   1115 1120 1125
   Asp Leu Lys Asn Ser Asn Pro Arg Ala Arg Ile Ser Val Lys Leu
   1130 1135 1140
   Val Ser Glu Ala Gly Val Gly Val Val Ala Ser Gly Val Val Lys
   1145 1150 1155
   Gly His Ala Asp His Val Leu Ile Ser Gly His Asp Gly Gly Thr
   1160 1165 1170
   Gly Ala Ser Arg Trp Thr Gly Ile Lys Asn Ala Gly Leu Pro Trp
   1175 1180 1185
   Glu Leu Gly Leu Ala Glu Thr His Gln Thr Leu Val Ala Asn Gly
   1190 1195 1200
   Leu Arg Gly Arg Ala Ile Leu Gln Thr Asp Gly Gln Leu Lys Thr
   1205 1210 1215
   Gly Lys Asp Val Ala Val Ala Cys Leu Leu Gly Ala Glu Glu Phe
   1220 1225 1230
   Gly Phe Ser Thr Ala Pro Leu Ile Thr Leu Gly Cys Ile Met Met
   1235 1240 1245
   Arg Lys Cys His Thr Asn Thr Cys Pro Val Gly Ile Ala Thr Gln
   1250 1255 1260
   Asp Pro Val Leu Arg Glu Lys Phe Ala Gly Glu Pro Glu His Val
   1265 1270 1275
   Ile Asn Phe Phe Phe Met Leu Ala Glu Glu Leu Arg Glu Ile Met
   1280 1285 1290
   Ser Gln Leu Gly Phe Arg Thr Ile Thr Glu Met Val Gly Arg Ser
   1295 1300 1305
   Asp Met Leu Glu Val Asp Pro Glu Val Val Lys Ser Asn Glu Lys
   1310 1315 1320
   Leu Glu Asn Ile Asp Leu Ser Leu Ile Leu Lys Pro Ala Ala Glu
   1325 1330 1335
   Ile Arg Pro Gly Ala Ala Gln Tyr Cys Val Glu Lys Gln Asp His
   1340 1345 1350
   Gly Leu Asp Met Ala Leu Asp Asn Lys Leu Ile Ala Leu Ser Lys
   1355 1360 1365
   Ala Ala Leu Glu Lys Glu Val Arg Val Phe Ile Glu Thr Pro Ile
   1370 1375 1380
   Gln Asn Thr Asn Arg Ala Val Gly Thr Met Leu Ser His Glu Val
   1385 1390 1395
   Thr Lys Arg Tyr His Met Lys Gly Leu Pro Ala Gly Thr Ile His
   1400 1405 1410
   Val Lys  Leu Thr Gly Ser Ala  Gly Gln Ser Leu Gly  Ala Phe Leu
       1415                 1420                 1425
   Cys Pro  Gly Ile Thr Leu Glu  Leu Glu Gly Asp Ser  Asn Asp Tyr
       1430                 1435                 1440
   Val Gly  Lys Gly Leu Ser Gly  Gly Lys Ile Val Val  Tyr Pro Pro
       1445                 1450                 1455
   Arg Asp  Ser Thr Phe Ile Pro  Glu Asp Asn Ile Val  Ile Gly Asn
       1460                 1465                 1470
   Val Ala  Leu Tyr Gly Ala Thr  Ile Gly Glu Ala Tyr  Phe Asn Gly
       1475                 1480                 1485
   Met Ala  Ala Glu Arg Phe Cys  Val Arg Asn Ser Gly  Ala Gln Ala
       1490                 1495                 1500
   Val Val  Glu Gly Ile Gly Asp  His Gly Cys Glu Tyr  Met Thr Gly
       1505                 1510                 1515
   Gly Thr  Val Val Ile Leu Gly  Lys Thr Gly Arg Asn  Phe Ala Ala
       1520                 1525                 1530
   Gly Met  Ser Gly Gly Ile Ala  Tyr Val Tyr Asp Ile  Asp Gly Lys
       1535                 1540                 1545
   Phe Ser  Val Arg Cys Asn His  Glu Leu Val Asp Leu  Tyr His Val
       1550                 1555                 1560
   Glu Glu  Glu Glu Asp Ile Thr  Thr Leu Lys Met Met  Ile Glu Gln
       1565                 1570                 1575
   His Arg  Leu Asn Thr Gly Ser  Val Val Ala Arg Asp  Ile Leu Ser
       1580                 1585                 1590
   Asn Phe  Asp Thr Leu Leu Pro  Lys Phe Val Lys Val  Phe Pro Arg
       1595                 1600                 1605
   Asp Tyr  Lys Arg Val Leu Asp  Asn Met Lys Ala Glu  Lys Ala Ala
       1610                 1615                 1620
   Ala Lys  Leu Ala Lys Glu Pro  Lys Ile Ser Asn Gly  Val Ser Val
       1625                 1630                 1635
   Thr Thr  Lys Lys Val Gln Pro  Glu Gln Ser Thr Asn  Arg Pro Thr
       1640                 1645                 1650
   Arg Val  Ser Asn Ala Lys Lys  Tyr Arg Gly Phe Ile  Ser Tyr Glu
       1655                 1660                 1665
   Arg Glu  Ser Ile Ser Tyr Arg  Asp Pro Asn Glu Arg  Val Lys Asp
       1670                 1675                 1680
   Trp Lys  Glu Val Ala Ile Glu  Ser Val Pro Gly Pro  Leu Leu Asn
       1685                 1690                 1695
   Thr Gln  Ser Ala Arg Cys Met  Asp Cys Gly Thr Pro  Phe Cys His
       1700                 1705                 1710
   Gln Glu  Ser Ser Gly Ala Gly  Cys Pro Leu Gly Asn  Lys Ile Pro
       1715                 1720                 1725
   Glu Phe  Asn Glu Leu Val His  Gln Asn Arg Trp Arg  Glu Ala Leu
       1730                 1735                 1740
   Asp Arg  Leu Leu Glu Thr Asn  Asn Phe Pro Glu Phe  Thr Gly Arg
       1745                 1750                 1755
   Val Cys  Pro Ala Pro Cys Glu  Gly Ser Cys Val Leu  Gly Ile Ile
       1760                 1765                 1770
   Glu Asn  Pro Val Ser Ile Lys  Ser Ile Glu Cys Ala  Ile Ile Asp
       1775                 1780                 1785
   Lys Gly  Phe Glu Glu Gly Trp  Met Val Pro Arg Pro  Pro Leu Gln
       1790                 1795                 1800
   Arg Thr  Gly Lys Lys Val Ala  Ile Ile Gly Ser Gly  Pro Ala Gly
       1805                 1810                 1815
   Leu Ala  Ala Ala Asp Gln Leu  Asn Lys Met Gly His  Phe Val Thr
       1820                 1825                 1830
   Val Phe  Glu Arg Ala Asp Arg  Ile Gly Gly Leu Met  Met Tyr Gly
       1835                 1840                 1845
   Val Pro  Asn Met Lys Thr Asp  Lys Ile Glu Ile Val  Gln Arg Arg
       1850                 1855                 1860
   Val Asn  Leu Met Ala Glu Glu  Gly Ile Thr Phe Val  Val Asn Ala
       1865                 1870                 1875
   Asn Val  Gly Ser Asp Pro Leu  Tyr Ser Ile Glu Arg  Leu Arg Ser
       1880                 1885                 1890
   Glu Asn Asp Ala Val Ile Leu Ala Cys Gly Ala Thr Lys Pro Arg
   1895 1900 1905
   Asp Leu Gly Ile Pro Gly Arg Glu Leu Ser Gly Val His Phe Ala
   1910 1915 1920
   Met Glu Phe Leu His Ala Asn Thr Lys Ser Leu Leu Asp Ser Asn
   1925 1930 1935
   Leu Glu Asp Gly Arg Tyr Ile Ser Ala Lys Gly Lys Lys Val Val
   1940 1945 1950
   Val Ile Gly Gly Gly Asp Thr Gly Thr Asp Cys Ile Gly Thr Ser
   1955 1960 1965
   Ile Arg His Gly Cys Thr Ser Ile Val Asn Leu Glu Leu Leu Thr
   1970 1975 1980
   Lys Pro Pro Ser Lys Arg Ala Ala Asp Asn Pro Trp Pro Gln Trp
   1985 1990 1995
   Pro Arg Ile Phe Arg Val Asp Tyr Gly His Gln Glu Ala Ser Ser
   2000 2005 2010
   Lys Phe Gly Asn Asp Pro Arg Thr Tyr Glu Val Leu Thr Lys Arg
   2015 2020 2025
   Phe Ile Gly Asp Glu Asn Gly Asn Val Lys Ala Leu Glu Val Val
   2030 2035 2040
   Arg Val Lys Trp Glu Lys Val Asp Gly Arg Phe Gln Phe Lys Glu
   2045 2050 2055
   Ile Glu Gly Ser Asn Glu Thr Ile Glu Ala Asp Leu Val Leu Leu
   2060 2065 2070
   Ala Met Gly Phe Leu Gly Pro Glu Ala Thr Ile Ala Glu Lys Leu
   2075 2080 2085
   Gly Leu Glu Lys Asp Asn Arg Ser Asn Phe Lys Ala Gln Phe Gly
   2090 2095 2100
   Asn Phe Ala Thr Ser Val Asp Gly Ile Phe Ala Ala Gly Asp Cys
   2105 2110 2115
   Arg Arg Gly Gln Ser Leu Val Val Trp Ala Ile Thr Glu Gly Arg
   2120 2125 2130
   Gln Ala Ala Ala Ala Val Asp Lys Tyr Leu Ser Arg Asn Glu Gln
           2135                2140                2145
   Asp Ala  Ala Glu Asp Ile Thr  Pro Ser Gly Ala Gly  Phe Val Gln
       2150                2155                2160
   Pro Val  Ala Ala
       2165
<210> 7
   <211> 20
   <212> DNA
   <213> Artificial
<220>
   <223> Primer
<400> 7
   ttagtggcaa atgggcttcg 20
<210> 8
   <211> 21
   <212> DNA
   <213> Artificial
<220>
   <223> Primer
<400> 8
   cgccacagca acatctctac c 21
<210> 9
   <211> 21
   <212> DNA
   <213> Artificial
<220>
   <223> Primer
<400> 9
   cagctgcaga gattcgtcct g 21
<210> 10
   <211> 23
   <212> DNA
   <213> Artificial
<220>
   <223> Primer
<400> 10
   tgttatccaa agccatgtca agg 23
<210> 11
   <211> 20
   <212> DNA
   <213> Artificial
<220>
   <223> Primer
<400> 11
   tggaatggca gcagaaaggt 20
<210> 12
   <211> 21
   <212> DNA
   <213> Artificial
<220>
   <223> Primer
<400> 12
   tcgcatccat gatcaccaat t 21
<210> 13
   <211> 23
   <212> DNA
   <213> Artificial
<220>
   <223> Primer
<400> 13
   gcaccatttc tgtacactcg ttg 23
<210> 14
   <211> 22
   <212> DNA
   <213> Artificial
<220>
   <223> Primer
<400> 14
   atcttcccat cagtctgcaa gc 22
<210> 15
   <211> 22
   <212> DNA
   <213> Artificial
<220>
   <223> Primer
<400> 15
   ttcaagagct taacaaggcg tg 22
<210> 16
   <211> 22
   <212> DNA
   <213> Artificial
<220>
   <223> Primer
<400> 16
   cacttgcagg ttcaacctca tc 22
<210> 17
   <211> 20
   <212> DNA
   <213> Artificial
<220>
   <223> Primer
<400> 17
   tgggaaatga tgcaccccta 20
<210> 18
   <211> 23
   <212> DNA
   <213> Artificial
<220>
   <223> Primer
<400> 18
   cttgtgcaaa catctgcttg aag 23
<210> 19
   <211> 21
   <212> DNA
   <213> Artificial
<220>
   <223> Primer
<400> 19
   aattctggaa ggaagggctt g 21
<210> 20
   <211> 23
   <212> DNA
   <213> Artificial
<220>
   <223> Primer
<400> 20
   tttgtatccc tcgcgtatag ctt 23
<210> 21
   <211> 500
   <212> DNA
   <213> Artificial
<220>
   <223> NADH-GoGAT fragment
<400> 21
   ggggtgcagc catgccgacg gcgcagggga ttggtttgaa gcacgcgacg ccgacgggcg        60
   tcggccgcag ggcccggcgc agccacgccg cgtccgcgca tggccgctcc gcgaggcagg       120
   cgcacggtgc catgtctctg gagggcggcg ggttcctcgg cggcgcgcac cgcatcgagg       180
   aacgcgtcgc gccatgcccg cctcgggccg cggcgcgtga cgcagagtcg atacggccca       240
   tgtctctgct acccgagagc agcattgggc tctacaaccc ggcgttcgag cgtgactcgt       300
   gcggcgttgg tttcgtcgcc gagctgtcgg gcgttgacaa ccgggcgacc gtcgtcgatg       360
   ccattcagat gcttgaaaga atggcacacc gaggtgcctg cggctgtgag aaaaacactg       420
   gtgatggtgc cggcattctc gttgctctac cacacaactt cttccgagag gtgacaaagg       480
   atgccggttt cgagttaccg                                                   500
<210> 22
   <211> 2144
   <212> PRT
   <213> Triticum aestivum
<400> 22
   Met Pro Thr Ala Gln Gly Ile Gly Leu Lys His Ala Ala Pro Pro Gly
   1               5                   10                  15
   Gly Arg Arg Ala Arg Arg Ser Gln Ser Ala Ala Ala Pro Gly Arg Ser
               20                  25                  30
   Ala Arg Gln Ala His Gly Ala Met Ser Leu Asp Gly Gly Phe Leu Gly
           35                  40                  45
   Gly Ala Gln Arg Thr Glu Glu Arg Val Ala Pro Arg Pro Pro Arg Ala
       50                  55                  60
   Ala Ala Arg Asp Ala Glu Ser Ile Arg Pro Met Ser Leu Leu Pro Glu
   65                  70                  75                  80
   Ser Ser Ile Gly Leu Tyr Asp Pro Ala Phe Glu Arg Asp Ser Cys Gly
                   85                  90                  95
   Val Gly Phe Val Ala Glu Leu Ser Gly Val Asp Asn Arg Ala Thr Val
               100                 105                 110
   Val Asp Ala Ile Gln Met Leu Glu Arg Met Ala His Arg Gly Ala Cys
           115                 120                 125
   Gly Cys Glu Lys Asn Thr Gly Asp Gly Ala Gly Ile Leu Val Ala Leu
       130                 135                 140
   Pro His Thr Phe Phe Arg Glu Val Thr Lys Asp Ala Gly Phe Glu Leu
   145                 150                 155                 160
   Pro Pro Pro Gly Glu Tyr Ala Val Gly Met Val Phe Leu Pro Thr Asp
                   165                 170                 175
   Glu Lys Arg Arg Glu Arg Ser Lys Thr Glu Phe Thr Lys Val Ala Glu
               180                 185                 190
   Ser Leu Gly His Ser Ile Leu Gly Trp Arg Gln Val Pro Thr Asp Asn
           195                 200                 205
   Ser Asp Leu Gly Gln Ala Ala Leu Asp Thr Glu Pro Ala Ile Glu Gln
       210                 215                 220
   Val Phe Leu Thr Lys Ser Ser Lys Ser Lys Ala Asp Phe Glu Gln Gln
   225                 230                 235                 240
   Leu Phe Ile Leu Arg Arg Leu Ser Ile Val Ser Ile Arg Ala Ala Leu
                   245                 250                 255
   Asn Leu Gln Arg Gly Gly Glu Arg Asp Phe Tyr Met Cys Ser Leu Ser
               260                 265                 270
   Ser Arg Thr Ile Val Tyr Lys Gly Gln Leu Met Pro Ser Gln Leu Gln
           275                 280                 285
   Gly Tyr Tyr Tyr Ala Asp Ile Gly His Glu Asn Phe Ser Ser Tyr Met
       290                 295                 300
   Ala Leu Val His Ser Arg Phe Ser Thr Asn Thr Phe Pro Ser Trp Asp
   305                 310                 315                 320
   Arg Ala Gln Pro Met Arg Val Leu Gly His Asn Gly Glu Ile Asn Thr
                   325                 330                 335
   Leu Lys Gly Asn Lys Asn Trp Met Lys Ala Arg Glu Gly Leu Leu Glu
               340                 345                 350
   Cys Glu Lys Leu Gly Leu Ser Gln Asp Glu Met Ser Lys Ile Leu Pro
           355                 360                 365
   Ile Val Asp Ala Thr Ser Ser Asp Ser Gly Ala Phe Asp Gly Val Leu
       370                 375                 380
   Glu Leu Leu Ile Arg Gly Gly Arg Ser Leu Pro Glu Ala Val Met Met
   385                 390                 395                 400
   Met Ile Pro Glu Ala Trp Gln Asn Asp Val Asn Met Glu Pro Asp Lys
                   405                 410                 415
   Lys Ala Leu Tyr Glu Phe Leu Ser Ala Leu Met Glu Pro Trp Asp Gly
               420                 425                 430
   Pro Ala Leu Ile Ser Phe Thr Asp Gly Arg Tyr Leu Gly Ala Thr Leu
           435                 440                 445
   Asp Arg Asn Gly Leu Arg Pro Gly Arg Phe Tyr Val Thr His Ser Gly
       450                 455                 460
   Arg Val Val Met Gly Ser Glu Val Gly Val Val Asp Ile Pro Ala Gln
   465                 470                 475                 480
   Asp Val Leu Arg Lys Gly Arg Leu Asn Pro Gly Met Met Leu Leu Val
                   485                 490                 495
   Asp Phe Asp Asn His Thr Val Val Asp Asp Glu Ala Leu Lys Ala Gln
               500                 505                 510
   Tyr Ser Lys Ala His Pro Tyr Gly Glu Trp Leu Lys Arg Gln Lys Met
           515                 520                 525
   Tyr Leu Lys Asp Ile Val Glu Ser Val Pro Glu Thr Asp Arg Val Ala
       530                 535                 540
   Pro Ser Ile Ser Gly Ser Ile Thr Gln Thr Asn Glu Asn Lys Glu Cys
   545                 550                 555                 560
   Val Gly Ile Asn Ala Ile Val Thr Pro Leu Lys Ala Phe Gly Tyr Thr
                   565                 570                 575
   Leu Glu Ala Leu Glu Met Leu Leu Leu Pro Met Ala Lys Asp Gly Val
               580                 585                 590
   Glu Ala Leu Gly Ser Met Gly Asn Asp Ala Pro Leu Ala Val Met Ser
           595                 600                 605
   Asn Arg Glu Lys Leu Thr Phe Glu Tyr Phe Lys Gln Met Phe Ala Gln
       610                 615                 620
   Val Thr Asn Pro Pro Ile Asp Pro Ile Arg Glu Lys Ile Val Thr Ser
   625                 630                 635                 640
   Met Glu Cys Met Ile Gly Pro Glu Gly Asp Leu Leu Glu Ile Thr Glu
                   645                 650                 655
   Lys Gln Cys Asn Arg Leu Ala Leu Lys Gly Pro Leu Val Ser Met Asp
               660                 665                 670
   Glu Met Glu Ser Ile Lys Lys Met Asn Tyr Arg Gly Trp Arg Ser Lys
           675                 680                 685
   Val Leu Asp Ile Thr Tyr Pro Lys Asn Ser Gly Arg Lys Gly Leu Glu
       690                 695                 700
   Glu Thr Leu Asp Arg Ile Cys Ala Glu Ala Arg Glu Ala Ile Arg Glu
   705                 710                 715                 720
   Gly Tyr Lys Ile Leu Val Leu Ser Asp Arg Gly Phe Ser Ser Asp Arg
                   725                 730                 735
   Val Ala Val Ser Ser Leu Leu Ala Val Gly Ala Val His Gln His Leu
               740                 745                 750
   Val Ala Asn Leu Glu Arg Thr Arg Val Gly Leu Leu Val Glu Ser Ala
           755                 760                 765
   Glu Pro Arg Glu Val His His Phe Cys Thr Leu Val Gly Phe Gly Ala
       770                 775                 780
   Asp Ala Ile Cys Pro Tyr Leu Ala Ile Glu Ala Ile Trp Cys Leu Gln
   785                 790                 795                 800
   Thr Asp Gly Lys Ile Pro Pro Thr Asp Ser Lys Glu Glu Leu Val Glu
                   805                 810                 815
   Lys Tyr Phe Tyr Ala Ser Ile Tyr Gly Met Met Lys Val Leu Ala Lys
               820                 825                 830
   Met Gly Ile Ser Thr Leu Ala Ser Tyr Lys Gly Ala Gln Ile Phe Glu
           835                 840                 845
   Ala Leu Gly Leu Ser Ser Glu Val Ile His Lys Cys Phe Glu Gly Thr
       850                 855                 860
   Pro Ser Arg Ile Glu Gly Ala Thr Phe Glu Met Leu Ala Arg Asp Ala
   865                 870                 875                 880
   Leu Arg Leu His Glu Leu Ala Phe Pro Ser Arg Thr Pro Pro Pro Gly
                   885                 890                 895
   Ser Ala Asp Ala Lys Ala Leu Pro Asn Pro Gly Asp Tyr His Trp Arg
               900                 905                 910
   Lys Asn Gly Glu Val His Leu Asn Asp Pro Leu Ala Met Ala Lys Leu
           915                 920                 925
   Gln Glu Ala Ala Lys Val Asn Ser Arg Glu Ala Tyr Lys Glu Tyr Ser
       930                 935                 940
   Lys Arg Ile Gln Glu Leu Asn Lys Ala Cys Asn Leu Arg Gly Met Leu
   945                 950                 955                 960
   Lys Phe Ile Asp Ser Thr Ser Lys Ile Ser Leu Asp Glu Val Glu Pro
   965 970 975
   Ala Ser Glu Ile Val Lys Arg Phe Cys Thr Gly Ala Met Ser Tyr Gly
   980 985 990
   Ser Ile Ser Leu Glu Ala His Thr Ala Leu Ala Val Ala Met Asn Lys
   995 1000 1005
   Leu Gly Gly Lys Ser Asn Thr Gly Glu Gly Gly Glu Gln Pro Ser
   1010 1015 1020
   Arg Met Glu Pro Leu Pro Asp Gly Ser Met Asn Pro Lys Arg Ser
   1025 1030 1035
   Ala Ile Lys Gln Val Ala Ser Gly Arg Phe Gly Val Ser Ser Tyr
   1040 1045 1050
   Tyr Leu Thr Asn Ala Asp Gly Leu Gln Ile Lys Met Ala Gln Gly
   1055 1060 1065
   Ala Lys Pro Gly Glu Gly Gly Glu Leu Pro Gly His Lys Val Ile
   1070 1075 1080
   Gly Asp Ile Ala Val Thr Arg His Ser Thr Ala Gly Val Gly Leu
   1085 1090 1095
   Ile Ser Pro Pro Pro His His Asp Ile Tyr Ser Ile Glu Asp Leu
   1100 1105 1110
   Ala Gln Leu Ile His Asp Leu Lys Asn Ser Asn Pro Gln Ala Arg
   1115 1120 1125
   Ile Ser Val Lys Leu Val Ser Glu Ala Gly Val Gly Val Val Ala
   1130 1135 1140
   Ser Gly Val Val Lys Gly His Ala Asp His Val Leu Ile Ser Gly
   1145 1150 1155
   His Asp Gly Gly Thr Gly Ala Ser Arg Trp Thr Gly Ile Lys Asn
   1160 1165 1170
   Ala Gly Leu Pro Trp Glu Leu Gly Leu Ala Glu Thr His Gln Thr
   1175 1180 1185
   Leu Val Ala Asn Gly Leu Arg Gly Arg Ala Val Leu Gln Thr Asp
   1190 1195 1200
   Gly Gln Leu Lys Thr Gly Arg Asp Val Ala Val Ala Cys Leu Leu
   1205 1210 1215
   Gly Ala  Glu Glu Phe Gly Phe  Ser Thr Ala Pro Leu  Ile Thr Leu
       1220                 1225                 1230
   Gly Cys  Ile Met Met Arg Lys  Cys His Thr Asn Thr  Cys Pro Val
       1235                 1240                 1245
   Gly Ile  Ala Thr Gln Asp Pro  Val Leu Arg Glu Lys  Phe Ala Gly
       1250                 1255                 1260
   Glu Pro  Glu His Val Ile Asn  Phe Phe Phe Met Leu  Ala Glu Glu
       1265                 1270                 1275
   Leu Arg  Glu Ile Met Ala Gln  Leu Gly Leu Arg Thr  Ile Asn Glu
       1280                 1285                 1290
   Met Val  Gly Arg Ser Asp Met  Leu Glu Val Asp Pro  Glu Val Val
       1295                 1300                 1305
   Lys Ser  Asn Glu Lys Leu Glu  Asn Ile Asp Leu Ser  Leu Ile Leu
       1310                 1315                 1320
   Lys Pro  Ala Ala Glu Ile Arg  Pro Gly Ala Ala Gln  Tyr Cys Val
       1325                 1330                 1335
   Glu Lys  Gln Asp His Gly Leu  Asp Met Ala Leu Asp  Asn Lys Leu
       1340                 1345                 1350
   Ile Ala  Leu Ser Arg Ala Ala  Leu Glu Lys Glu Val  Arg Val Phe
       1355                 1360                 1365
   Ile Glu  Thr Pro Ile Lys Asn  Thr Asn Arg Ala Val  Gly Thr Thr
       1370                 1375                 1380
   Leu Ser  His Glu Val Thr Lys  Arg Tyr His Met Lys  Gly Leu Asp
       1385                 1390                 1395
   Pro Gly  Thr Ile His Val Lys  Leu Thr Gly Ser Ala  Gly Gln Ser
       1400                 1405                 1410
   Phe Gly  Ala Phe Leu Cys Pro  Gly Ile Thr Leu Glu  Leu Glu Gly
       1415                 1420                 1425
   Asp Ser  Asn Asp Tyr Val Gly  Lys Gly Leu Ser Gly  Gly Lys Ile
       1430                 1435                 1440
   Val Val  Tyr Pro Pro Arg Asn  Ser Thr Phe Ser Ala  Glu Asp Asn
       1445                 1450                 1455
   Ile Val Ile Gly Asn Val Ala Leu Tyr Gly Ala Thr Lys Gly Glu
   1460 1465 1470
   Ala Tyr Phe Asn Gly Met Ala Ala Glu Arg Phe Cys Val Arg Asn
   1475 1480 1485
   Ser Gly Ala Arg Thr Val Val Glu Gly Ile Gly Asp His Gly Cys
   1490 1495 1500
   Glu Tyr Met Thr Gly Gly Thr Val Val Ile Leu Gly Lys Thr Gly
   1505 1510 1515
   Arg Asn Phe Ala Ala Gly Met Ser Gly Gly Ile Ala Tyr Val Tyr
   1520 1525 1530
   Asp Val Asp Gly Thr Phe Ser Val Arg Cys Asn Asn Glu Leu Val
   1535 1540 1545
   Asp Leu Tyr His Val Glu Glu Glu Asp Asp Val Thr Thr Leu Lys
   1550 1555 1560
   Met Met Ile Glu Gln His Arg Leu His Thr Glu Ser Val Leu Ala
   1565 1570 1575
   Lys Asp Ile Leu Ser Lys Phe Asp Thr Leu Leu Pro Lys Phe Val
   1580 1585 1590
   Lys Val Tyr Pro Arg Asp Tyr Lys Arg Val Leu Glu Glu Met Lys
   1595 1600 1605
   Ala Glu Lys Ala Ala Ala Arg Pro Thr Lys Glu Pro Lys Val Ala
   1610 1615 1620
   Asn Gly Val Ser Val Thr Thr Lys Lys Ile Gln Thr Glu Lys Ser
   1625 1630 1635
   Ser Ser Arg Pro Thr Arg Val Ala Asn Ala Lys Lys Tyr Arg Gly
   1640 1645 1650
   Phe Val Thr Tyr Glu Arg Glu Gly Val Ser Tyr Arg Asp Pro Asn
   1655 1660 1665
   Glu Arg Val Lys Asp Trp Asn Glu Val Ala Ile Glu Ser Val Pro
   1670 1675 1680
   Gly Pro Leu Leu Asn Thr Gln Ser Ala Arg Cys Met Asp Cys Gly
   1685 1690 1695
   Thr Pro  Phe Cys His Gln Glu  Ser Ser Gly Ala Gly  Cys Pro Leu
       1700                 1705                 1710
   Gly Asn  Lys Ile Pro Glu Phe  Asn Glu Leu Val His  Gln Asn Arg
       1715                 1720                 1725
   Trp Arg  Glu Ala Leu Asp Arg  Leu Leu Glu Thr Asn  Asn Phe Pro
       1730                 1735                 1740
   Glu Phe  Thr Gly Arg Val Cys  Pro Ala Pro Cys Glu  Gly Ser Cys
       1745                 1750                 1755
   Val Leu  Gly Ile Ile Glu Asn  Pro Val Ser Ile Lys  Ser Ile Glu
       1760                 1765                 1770
   Cys Ser  Ile Ile Asp Lys Gly  Phe Glu Glu Gly Trp  Met Val Pro
       1775                 1780                 1785
   Arg Pro  Pro Leu Gln Arg Thr  Gly Lys Lys Ile Ala  Ile Val Gly
       1790                 1795                 1800
   Ser Gly  Pro Ala Gly Leu Ala  Ala Ala Asp Gln Leu  Asn Lys Met
       1805                 1810                 1815
   Gly His  Phe Val Thr Val Phe  Glu Arg Ser Asp Arg  Ile Gly Gly
       1820                 1825                 1830
   Leu Met  Met Tyr Gly Val Pro  Asn Met Lys Thr Asp  Lys Ile Gly
       1835                 1840                 1845
   Val Val  Gln Arg Arg Val Asn  Leu Met Ala Glu Glu  Gly Val Thr
       1850                 1855                 1860
   Phe Val  Val Asn Ala Asn Val  Gly Ser Asp Pro Leu  Tyr Ser Ile
       1865                 1870                 1875
   Glu Arg  Leu His Ser Glu Asn  Asn Ala Val Ile Leu  Ala Cys Gly
       1880                 1885                 1890
   Ala Thr  Lys Pro Arg Asp Leu  Ser Ile Pro Gly Arg  Glu Leu Ala
       1895                 1900                 1905
   Gly Val  His Phe Ala Met Glu  Phe Leu His Ala Asn  Thr Lys Ser
       1910                 1915                 1920
   Leu Leu  Asp Ser Asn Leu Glu  Asp Gly Arg Tyr Ile  Ser Ala Gln
       1925                 1930                 1935
   Gly Lys  Lys Val Val Val Ile  Gly Gly Gly Asp Thr  Gly Thr Asp
   1940 1945 1950
   Cys Ile Gly Thr Ser Val Arg His Gly Cys Ser Ser Ile Val Asn
   1955 1960 1965
   Leu Glu Leu Leu Thr Lys Pro Pro Ser Lys Arg Ala Ser Asp Asn
   1970 1975 1980
   Pro Trp Pro Gln Trp Pro Arg Val Phe Arg Val Asp Tyr Gly His
   1985 1990 1995
   Gln Glu Ala Ser Thr Lys Phe Gly Asn Asp Pro Arg Thr Tyr Glu
   2000 2005 2010
   Val Leu Thr Lys Arg Phe Ile Gly Asp Glu Asp Gly Lys Leu Lys
   2015 2020 2025
   Ala Leu Glu Val Val Arg Val Lys Trp Glu Lys Val Asp Gly Lys
   2030 2035 2040
   Phe Gln Phe Lys Glu Ile Glu Gly Ser Gln Glu Ile Ile Glu Ala
   2045 2050 2055
   Asp Leu Val Leu Leu Ala Met Gly Phe Leu Gly Pro Glu Glu Asn
   2060 2065 2070
   Ile Ala Asp Lys Leu Gly Leu Glu Lys Asp Asn Arg Ser Asn Phe
   2075 2080 2085
   Lys Ala Gln Phe Gly His Phe Gly Thr Ser Val Asp Gly Val Phe
   2090 2095 2100
   Ala Ala Gly Asp Cys Arg Arg Gly Gln Ser Leu Val Val Trp Ala
   2105 2110 2115
   Ile Thr Glu Gly Arg Glu Ala Ala Ala Ala Val Asp Lys Tyr Leu
   2120 2125 2130
   Ser Arg Asp Glu Gln Asn Val Ala Gly Leu Thr
   2135 2140
<210> 23
   <211> 10174
   <212> DNA
   <213> Triticum aestivum
<400> 23
   atgccgacgg cgcaggggat tggtttgaag cacgcggcgc cgccgggcgg ccgcagggcc      60
   cgccgcagcc agtccgcggc cgcgccgggc cgctccgcaa ggcaggcgca cggcgccatg     120
   tccctggatg gcgggttcct cggcggcgcg cagcgcaccg aggaacgcgt cgcgccacgc 180
   ccgcctcggg ccgcggcgcg cgacgccgag tccatcaggc ccatgtctct gctacccgag 240
   agcagcattg ggctgtacga cccggcgttc gagcgtgact cgtgcggcgt tggcttcgtc 300
   gccgagctgt cgggcgttga caaccgggcg accgtgagtt ctttgcacca ggacaccgcc 360
   gcagcttctc tcttttatct accacgttga gatattgatt ttgctgtgat ttactttaca 420
   ggtcgtcgat gccattcaga tgcttgaaag aatggcacac cgaggtgcct gcggctgtga 480
   gaaaaacact ggtgatggtg ccggcattct cgttgctcta ccacacacct tcttccgaga 540
   ggtgaataaa cagaaaattt caagcaaact cagttttcct tgacagagtt attcatcttg 600
   ggtgttgctt tgctattgct gcaggtgaca aaggatgccg gtttcgagtt accgccacca 660
   ggtgagtatg ctgttggaat ggtcttcctg ccaaccgatg agaagcgccg cgagaggagc 720
   aaaactgagt ttacaaaggt tggttggttg gtgtatagac aattagacag cgtgcgaggc 780
   gagtctgcga tggtaagctt atgtcttgga ttgtttttat tcatgcaggt cgctgagtct 840
   ctaggacatt cgatacttgg gtggcgccag gttcccactg acaattcaga cttgggccaa 900
   gctgcgctcg atactgaacc agccattgaa caggttttcc tcaccaagag ttcaaaatcg 960
   aaggccgact tcgaacagca ggtcctccat gagtttaatt ttctctcaac acatgtaatg 1020
   agctttcctc tcaactaacc catcaattat cacagttgtt tatcctgagg aggctctcaa 1080
   ttgtatctat ccgggccgcg ctgaatcttc agcgtggagg agagagagat ttctacatgt 1140
   gctctctatc ttcaaggtcc ttacttcttc tgaattgcat tttcactaca aactgatgag 1200
   gttgagatca gggagcccaa ttaagctcct gactttaatg gaacattaac aacacaaaag 1260
   ttgataggaa agctgactgg gaaaaacaat cctgggctat ctcttaagca ttagaaatct 1320
   gtaatgtaag gacatttcat ttttgctgta ttcatgtgag taattattgt taaatcttct 1380
   attgtggttt tccaggacca ttgtttacaa gggccaactt atgccatccc agctccaagg 1440
   gtactactat gcggatatag gtcatgaaaa cttctccagt tatatggctc tggtaaatac 1500
   gaaaccttga tactcttaca acaaaagtca tggtcgtagt cagcggatgg gaatcaataa 1560
   tagcaagcca gttgtaagat caagactcgc tcctgtgcag acatcaaatt tgagctgtct 1620
   attcatatgc tatttcagtc ctgcttcgtg atgcctttaa gtgaagtact agaaacttct 1680
   cttagttgat acccgataac catgttgcag agtccagctg cagttctgct atggccgact 1740
   gcttgccgac tcttcaggat gtgaccgaag aaattgtgtt tggtttgagg tagcttaggg 1800
   ctcccaactg ttttgcatga aattatacgc agctgacgta tttttacaaa attcgttctt 1860
   cacaaggaag ttttgttttg aatatctgca aaagatgttt ataatatgat ttctaaaagt 1920
   catatcttga aacaggttca ctcaaggttc tccaccaaca ccttccccag ctgggaccgt 1980
   gcacagccaa tgcgtgtctt aggccacaat ggagagatca atactctcaa agggaacaaa 2040
   aactggtaat attattgtca aatcttttcc tctttgcttt aacctttcct tggcagatgc 2100
   aactatctca atatgcacgt tgtatatatg gcaggatgaa agctcgtgag ggtctcttgg 2160
   agtgtgagaa gcttggccta tcacaggatg aaatgtcaaa gattcttcca atagtagatg 2220
   ccacttcttc agattcaggt ccgcagtcta atatgcagca tgaattccac ttggcaaata 2280
   aaaattcatc tcggttttgt gagttactaa gtgtgaaaaa acatgtgaat tctaggtgca 2340
   tttgatggtg ttctcgagct ccttatccgt ggtggaagaa gcctgccaga agctgtgatg 2400
   atgatgatcc ctgaggcgtg gcagaatgat gtaaacatgg aacctgataa gaaagctctg 2460
   tacgagttct tgtcagccct tatggagcct tgggatggac ctgctctcat atcttgtaaa 2520
   aaacttaaac ccgtttcttt tttctgatca tacttctaac tgattactta agctaacatg 2580
   ggtacaacct gtagttaccg atggccgcta ccttggagct accctggatc gcaatggcct 2640
   taggcctggt cgattttatg taacccacag tggacgtgtg gtcatgggta gtgaagttgg 2700
   tgttgtggat attcctgccc aagatgtgtt gagaaagggt cggcttaacc ctggaatgat 2760
   gttactcgtt gacttcgaca accatactgt agtagatgat gaagcactca aggcacagta 2820
   ctctaaagct cacccgtatg gagaatggct caagcgacaa aagatgtacc tcaaagacat 2880
   tgtagaatct gtcccagaaa ctgacagagt tgctccaagc atttctggtt ctattacggt 2940
   aagtacttac gtggtccaac ttcctcattc actgggtagt ttggtgatga aaattggtaa 3000
   accataagga acatggtgat tttaatattc ttttacaatt tggtgtagca aacaaatgag 3060
   aacaaggaat gtgtaggcat caatgcaatt gtgactccac taaaggcgtt tgggtaagca 3120
   aggattgaac attgtcaact atcactcatg tttcaatttc tgctggctaa tgatccaacc 3180
   gattaactac aggtacacat tggaagccct agaaatgttg ctgctgccaa tggctaaaga 3240
   tggagtggaa gctcttggat caatgggaaa tgatgcaccc ctagcagtga tgtcaaacag 3300
   agagaagctg acttttgagt acttcaagca gatgtttgca caagtaacaa accctccaat 3360
   cgatccaatt agggagaaga ttgttacatc tatggaatgt atgattgggc cagaaggaga 3420
   tttgctggaa ataaccgaaa agcaatgcaa ccgccttgca cttaaaggtc ctttggtgtc 3480
   aatggatgaa atggaatcta tcaagaagat gaactaccgt ggttggcgca gcaaggtgct 3540
   cgacataact tatccgaaga attctggaag gaagggcttg gaagaaactt tggatagaat 3600
   ttgtgctgaa gcccgggaag ctatacgcga gggatacaaa attttagttc tttcagacag 3660
   aggtcagtga cttattcact ttatacttgc atcccattgt ttgatggtac aaattactca 3720
   taagttgaca aaaaattcta gcagatgggt caggtgtgca caatttatta tcgttagctt 3780
   ccagcaaaca tatcacatca ccatatagta acttaaatat gatcataatt ttgtttgata 3840
   gtcaattgtt tgcagtgtaa tgtgttgtat gaaggacatt cttactttgt caccttggac 3900
   ataattggaa tgtatattaa gtttattatg caactcaaca tcttccaata ctgaaatttg 3960
   ttgtattttc ttgattcagg attttcttca gaccgtgttg ctgtcagttc cctcttagca 4020
   gttggagcag tacatcaaca ccttgttgca aatcttgaga ggacacgcgt aggattattg 4080
   gttgagtctg ctgaacctcg tgaagtgcac catttctgta cactcgttgg atttggtgca 4140
   gatgctatat gcccttattt ggccattgaa gcaatttggt gcttgcagac tgatgggaag 4200
   attcccccta ccgactcaaa ggaggaactt gtcgaaaaat atttttatgc ttccatctat 4260
   ggaatgatga aggttcttgc aaagatggga atatccaccc ttgcatctta caaaggggca 4320
   cagatttttg aagctcttgg actttcttct gaagtgattc acaagtgttt tgaaggcact 4380
   cctagcagaa ttgagggtgc aacgttcgaa atgcttgcac gtgatgctct ccgtcttcac 4440
   gagttggcat tcccatcaag aacacctccg cctggcagtg cagatgcaaa agcccttccc 4500
   aatccagggg attaccactg gaggaaaaat ggtgaagtcc atctaaatga tcctcttgca 4560
   atggcaaaat tacaagaagc agctaaagta aacagccggg aagcatacaa agaatattct 4620
   aagcgaattc aagagcttaa caaggcgtgc aatctgcgtg gtatgctgaa atttatagat 4680
   agcactagca agatttcttt ggatgaggtt gaacctgcaa gtgagatagt gaaacgcttt 4740
   tgtactgggg caatgagtta tggatctatt tcgttggagg cacatactgc ccttgctgtg 4800
   gccatgaaca aactgggagg caaatctaac acaggtattt catgcatatg tgacattttt 4860
   taccctttgg ctggttgtta cccttctgat gaaatcatgt atagtagctt tacttgcaca 4920
   tcaactctta gagccttgtt gcataacata tttttcttat ttttgctgca gtagtgtagt 4980
   gtctatgttt ctactgttta aatatgccct cgaagttggt actttataca tgaccatgtc 5040
   tagaaatgca ctacttattt tgatgctatc acatgaaaac tatactctca tatttacttg 5100
   tcgacatact tgtactgcac ttgtgtgttt ggttgcattc tcgtctcagc atagttccct 5160
   cttcatgcat gctcaactca acacccctct tcatgcatgc tttcttcatg catgcttcta 5220
   gtgtatttgt gctaagctag tgtggactca tgcaccctcc atacactcta ccaaacaccc 5280
   aaaagtgggt cgagaaggga actcttgggc tcatgcaccc ttcatacacc ctaccaaaca 5340
   cacccttaat gcgctaagct tttcatcttc ctgttgatgt tgaatgcact tctgtgaaca 5400
   aattatccca gttcatagca agagttaact tctaagcttt tatctatctg ttgatgttga 5460
   atgcacttct ggtagcaaat tatcctaatt cctagcaaga tttaacttct gcttgcgtag 5520
   aaacccagac agcttgcacg cactattctt ctttggactg tttgtattag atattttctg 5580
   cccacacagt taatattata ttgccaaatc tttgtattta ttttcgctcc taaactaaac 5640
   tcattttacc catctgctaa tttaggggag ggaggggagc agccttctcg tatggagcct 5700
   cttcctgatg gttcgatgaa tccaaaacga agtgcaatca agcaagttgc cagtggacga 5760
   tttggagttt ccagctatta tctgactaat gcagatgggc tgcagataaa aatggctcag 5820
   gtacttgaca atgctgtgca ttgcgcaatc actggaatga tctcagttta tagaaagcaa 5880
   ctgtattcaa cgtcctgtgg ttggcattta ttcttttttt gtatcgtata tgtatagggt 5940
   gctaagcctg gtgaaggtgg tgagcttcca ggtcacaagg ttatcggtga cattgcagtt 6000
   accagacatt ctacagctgg tgttgggctt attagtccac ctcctcacca tgatatatat 6060
   tctatcgagg atcttgcaca gcttatccat gaccttaagg tacattacct gatgctcttc 6120
   tatagccaag gcatctgcct atttatgtat ggttgttagt tgctacttct agattatatt 6180
   ggttatctaa acgctgtgat ggtctaaccc tcctgagaag ctagaatcac ttgctaaact 6240
   ggtacaagtg atcctgcaca tatgcatgtg tctccatttg ggggtttacc ctcctttttg 6300
   aaaaactcat tttgcaaatg ttacctgctg atgtagtgat attcatgcct gttttctctc 6360
   tgcagaattc aaatcctcaa gctcgaatca gcgtgaagct agtgtctgag gctggtgttg 6420
   gagttgtagc tagcggtgtt gtaaaaggac acgcagacca tgttcttatt tctggccatg 6480
   acggtggcac cggtgcatca agatggacag gtatcaagaa tgctggactt ccatgggaac 6540
   taggattggc tgagacacat caaactttag tggcaaatgg gcttcgtggt cgagctgtcc 6600
   tacaaacaga tggccaatta aaaactggta gagatgttgc tgtggcgtgc ttacttggtg 6660
   cagaggaatt tggtttcagc actgctccac tgatcacact tggctgcatt atgatgcgga 6720
   agtgccatac caatacttgc cctgttggta tagccactca agatccagtg ctccgagaaa 6780
   aatttgctgg agaaccagag catgtcatta actttttctt catgcttgct gaggagctaa 6840
   gagaaattat ggctcagctt ggcctccgta caattaatga aatggttgga cgctctgata 6900
   tgcttgaagt tgatccagaa gtagttaaga gcaatgagaa acttgaaaat attgatctct 6960
   cattgatctt aaaaccagct gcagagattc gtcctggggc tgctcagtac tgtgttgaaa 7020
   agcaagacca tggccttgac atggctttgg ataacaaact tatagcttta tcaagggctg 7080
   cacttgaaaa ggaagttcgt gttttcattg agactccaat caagaacact aatcgggcag 7140
   taggcaccac acttagccat gaagtcacaa aacgttatca catgaaggga ttagatcctg 7200
   gaaccattca tgtgaaactc actggaagtg ctggtcagag ttttggtgct tttctctgtc 7260
   ctggaataac ccttgagctt gaaggagaca gcaatgatta cgttggcaaa ggattatctg 7320
   gtggaaagat tgttgtgtac ccacccagga acagtacatt tagtgcagaa gacaatattg 7380
   tcattggtaa tgtggccctg tatggtgcta ccaagggaga agcatacttc aatggaatgg 7440
   cagcagaaag gttttgtgtt cgtaattctg gtgctcgaac agtggttgaa ggaattggtg 7500
   atcatggatg cgagtacatg acagggggta ctgtagtcat ccttggtaaa acaggaagaa 7560
   attttgctgc tgggatgagt ggaggcatcg cttatgttta tgatgttgat gggacattca 7620
   gtgtccgctg taacaatgag ttggttgatc tatatcatgt ggaggaagag gatgatgtaa 7680
   ccactttgaa aatgatgata gagcaacatc gacttcacac agagagtgtc ctggccaaag 7740
   acatactctc taaattcgac actcttcttc caaaatttgt aaaagtatac ccaagggatt 7800
   ataagagggt tctagaagaa atgaaggcag aaaaagctgc agctaggcct acaaaggaac 7860
   ctaaggtggc aaatggtgtt tctgtgacaa ctaaggtaat atatgattta agtaataatt 7920
   cttctgcata cctttttaat ttgaatttat agtagagatg gtgttctcca tttaggttta 7980
   tccatcagaa attgcaatga tacacattgc cactactgtg tgaacttcta tttttgccta 8040
   agtagattaa ctttgatcta cagaaaatac aaacagagaa gtcatccagc cgaccaacac 8100
   gtgttgccaa cgccaaaaag taccggggtt ttgtaacata tgagcgagag ggtgtttctt 8160
   accgtgatcc aaatgagcgt gttaaagatt ggaacgaggt tgccattgaa tcagttccag 8220
   ggccactgtt aaacacacag tctgctcgtt gtatggattg tggcactcct ttctgtcatc 8280
   aggtgaagct gagttcatct ttctatttta acattgatga ttgtagttgg atttattgtg 8340
   gggttaagga ttgatatgtt tagtcattga ctacatatga cattgagatg ctaagatttc 8400
   atgattattt gtacaggaaa gctcaggtgc tggctgtcct cttggaaata agatcccaga 8460
   gttcaatgaa ttagttcacc agaatagatg gcgtgaagca ttggatcgcc tactagagac 8520
   aaacaacttc cctgaattta ctggacgtgt gtgccctgct ccttgtgagg ggtcttgtgt 8580
   tcttggcatt attgagaacc cagtatcaat caaaagcata gaatgctcga ttatagacaa 8640
   aggttttgaa gagggatgga tggtaccacg accaccactt caaagaacag ggtatgtctt 8700
   ttttaccatc tagttcggct tattccctta tttatttact tctgtttcat taataggtaa 8760
   catttgagtt acactttcta atataaactg ttcctttcgc agaaagaaaa tcgctatagt 8820
   tggcagtggt cctgctggtt tggctgccgc tgatcaacta aataaaatgg gccattttgt 8880
   aactgtattt gaacgttcgg atcgtatagg aggtcttatg atgtatggag taccaaacat 8940
   gaagacagac aagattggag ttgttcagcg tcgtgtcaat ttaatggccg aagagggtgt 9000
   aacatttgtg gtgaatgcta atgtagggag tgatccttta tactcaattg aacgtctcca 9060
   ttctgagaac aatgcagtta ttttggcttg tggagctaca aaaccaaggt aactaattgg 9120
   aacgaggatt ttttttttct agtttactcc aagagtagca acaatctcaa aagaactgac 9180
   atatatcttt tgacactaac tgactaacat catgtgggat attcttgaac agggacctca 9240
   gtattcctgg ccgtgagcta gctggagttc attttgccat ggaatttctc cacgcaaata 9300
   ccaaaagttt gcttgatagc aacctggagg atggaagata catatctgcc cagggtaaga 9360
   aggtggtggt cattggtggt ggagacacag gcacagattg catcggtacg tctgttaggc 9420
   atggttgcag cagcattgta aatctggagc ttctcaccaa gccaccaagc aagagagctt 9480
   ctgacaaccc ctggccccag gtaaatgcaa aaaaaaaaaa actggatatt tccctgcaca 9540
   tgtgaatttg gccattccat ttccagcttg gaaagttctt aagtcatcat tctgtttttg 9600
   catgctgtgg cctagagtct tccgagtgga ctatgggcac caggaagcat ctaccaaatt 9660
   tggaaatgat ccaagaactt acgaagtctt aaccaagcgt ttcattggtg atgaagatgg 9720
   aaaattgaag gcccttgagg tggtgcgcgt gaagtgggag aaagtagatg gaaaattcca 9780
   gttcaaggag attgaaggat cacaagagat catcgaggca gaccttgtcc tccttgccat 9840
   gggattcctg ggccctgaag aggttagtta tgcagactct gaactactgt cacgcctgag 9900
   ttttatcttg atgataaatg ttggacaaac aactcaccgt tttatttctc tgcagaacat      9960
   cgctgacaaa ctgggtttag agaaagacaa ccgttccaac ttcaaagctc aattcggaca     10020
   cttcgggacc agtgtggatg gcgtttttgc cgctggggat tgcaggcgcg ggcaatcgct     10080
   ggttgtttgg gccatcaccg aagggcgtga agctgctgct gcagtagata agtacttgtc     10140
   aagggatgaa caaaatgtcg caggccttac atag                                 10174
<210> 24
   <211> 10036
   <212> DNA
   <213> Triticum aestivum
<220>
   <221> misc_feature
   <222> (1985)..(1985)
   <223> n is a, c, g, or t
<220>
   <221> misc_feature
   <222> (2031)..(2031)
   <223> n is a, c, g, or t
<220>
   <221> misc_feature
   <222> (2222)..(2222)
   <223> n is a, c, g, or t
<220>
   <221> misc_feature
   <222> (3634)..(3634)
   <223> n is a, c, g, or t
<220>
   <221> misc_feature
   <222> (4548)..(4548)
   <223> n is a, c, g, or t
<220>
   <221> misc_feature
   <222> (6940)..(6940)
   <223> n is a, c, g, or t
<220>
   <221> misc_feature
   <222> (6970)..(6970)
   <223> n is a, c, g, or t
<220>
   <221> misc_feature
   <222> (7011)..(7011)
   <223> n is a, c, g, or t
<400> 24
   atgtcggcgg cgcaagggat tggtttgaag cacgcggcgc cgacgggcgt cggccgcagg    60
   gcccggcgca gccacgccgc gtccgcgcat ggccgctccg cgaggcaggc gcacggcgcc   120
   atgtctctgg agggcggcgg gttcctcggc ggcgcgcacc gcatcgagga acgcgtcgcg   180
   ccatgcccgc ctcgggccgc ggcgcgcgac gcagagtcga tacggcccat gtctctgcta     240
   cccgagagca gcattgggct ctacaacccg gcgttcgagc gtgactcgtg cggcgttggt     300
   ttcgtcgccg agctgtcggg cgttgacaac cgggcgaccg tgagttcttt gcaccaagga     360
   caccgctgca gcttctctct tccatctaca gggttgagat attgatttgg ctgtgattta     420
   ctttacaggt cgtcgatgcc attcagatgc ttgaaagaat ggcacaccga ggtgcctgcg     480
   gctgtgagaa aaacactggt gatggtgccg gcattctcgt tgctctacca cacaacttct     540
   tccgagaggt gaatcaacga aaattgcaag caaactcagt tttccctgac agagttattc     600
   atcttgggtg ttgctctgct attgccgcag gtgacaaagg atgccggttt cgagttaccg     660
   ccaccaggtg agtatgctgt tggaatggtc ttcctgccaa ccgatgagaa gcgtcgcgag     720
   aggagcaaaa ctgagtttac aaaggttggt tggttgctgt atagacaatt agacagcgtg     780
   tgaggcgagt ctgcgatgat aagcttatgc cttggatttt ttttattcac gcaggtcgcg     840
   gagtcgctag gacattcgat acttgggtgg cgccaggttc ccactgacaa ttcagacttg     900
   ggccaagctg ctctcgacac tgaaccagcg attgaacagg ttttcctcac caagagtcca     960
   aactcgaagg ccgacttcga acagcaggtc ctccatgagt ttaattttct ctcaacacat    1020
   gtaatgagct ttcctctcaa ctaacccatc aattatcaca gttgtttatc ctgaggaggc    1080
   tctcaattgt atctatccgg gccgcgctga atcttcagcg tggaggagag agagatttct    1140
   acatgtgctc tctatcttca aggtccttac ttcttctgaa ttgcattttc actacaaact    1200
   gatgaggttg agatcaggga gcccaattaa gctcctgact ttaatggaac attaacaaca    1260
   caaaagttga taggaaagct gactgggaaa aacaatcctg ggctatctct taagcattag    1320
   aaatctgtaa tgtaaggaca tttcattttt gctgtattca tgtgagtaat tattgttaaa    1380
   tcttctattg tggttttcca ggaccattgt ttacaagggc caacttatgc catcccagct    1440
   ccaagggtac tactatgcgg atataggtca tgaaaacttc tccagttata tggctctggt    1500
   aaatacgaaa ccttgatact cttacaacaa aagtcatggt cgtagtcagc ggatgggaat    1560
   caataatagc aagccagttg taagatcaag actcgctcct gtgcagacat caaatttgag    1620
   ctgtctattc atatgctatt tcagtcctgc ttcgtgatgc ctttaagtga agtactagaa    1680
   acttctctta gttgataccc gataaccatg ttgcagagtc cagctgcagt tctgctatgg    1740
   ccgactgctt gccgactctt caggatgtga ccgaagaaat tgtgtttggt ttgaggtagc    1800
   ttagggctcc caactgtttt gcatgaaatt atacgcagct gacgtatttt tacaaaattc    1860
   gttcttcaca aggaagtttt gttttgaata tctgcaaaag atgtttataa tatgatttct    1920
   aaaagtcata tcttgaaaca ggttcactca aggttctcca ccaacacctt ccccagctgg    1980
   gaccntgcac agccaatgcg tgtcttaggc cacaatggag agatcaatac nctcaaaggg    2040
   aacaaaaact ggtaatatta ttgtcaaatc ttttcctctt tgctttaacc ttttcttggc    2100
   agatgcaact atctcaatat gcacgttgta tatatggcag gatgaaagct cgtgagggtc    2160
   tcttggagtg tgagaagctt ggcctatcac aggatgaaat gtcaaagatt cttccaatag 2220
   tngatgccac ttcttcagat tcaggtccgc agtctaatat gcagcatgaa ttccacttgg 2280
   caaataaaaa ttcatctcgg ttttgtgagt tactaagtgt gaaaaaacat gtgaattcta 2340
   ggtgcatttg atggtgttct cgagctcctt atccgtggtg gaagaagcct gccagaagct 2400
   gtgatgatga tgatccctga ggcgtggcag aatgatgtaa acatggaacc tgataagaaa 2460
   gctctgtacg agttcttgtc agcccttatg gagccttggg atggacctgc tctcatatct 2520
   tgtaaaaaac ttaaacccgt ttcttttttc tgatcatact tctaactgat tacttaagct 2580
   aacatgggta caacctgtag ttaccgatgg ccgctacctt ggagctaccc tggatcgcaa 2640
   tggccttagg cctggtcgat tttatgtgac ccacagtgga cgtgtggtca tgggtagtga 2700
   agttggtgtt gtggatattc ctgcccaaga tgtgttgaga aagggtcggc ttaaccctgg 2760
   aatgatgtta ctcgttgact tcgacaacca tactgtagta gatgatgaag cactcaaggc 2820
   acagtactct aaagctcacc cgtatggaga atggctcaaa cgacaaaaga tgtacctcaa 2880
   agacattgta gaatctgtcc cagaaactga cagagttgct ccaagcattt ctggttctat 2940
   tacggtaagt acttacgtgg tccaacttcc tcattcactg ggtagtttgg tgacgaaaat 3000
   tggtaaacca taagtaacat ggtgatttta atattctttt acaatttggt gtagcaaaca 3060
   aatgagaaca aggaatgtgt aggcatcaat gcaattgtga ctccactaaa ggcgtttggg 3120
   taagcaagga ttaaacattg tcaactatca ctcatgtttc agtttctgct ggctaatgat 3180
   ccaaccgatt aactacaggt acacattgga agccctagaa atgttgctgc tgccaatggc 3240
   taaagatgga gtggaagctc ttggatcaat gggaaatgat gcacccctag cagtgatgtc 3300
   aaacagagag aagctgactt ttgagtactt caagcagatg tttgcacaag taacaaaccc 3360
   tccaatcgat ccaattaggg agaagattgt tacatctatg gaatgtatga ttgggccaga 3420
   aggagatttg ctggaaataa ccgaaaagca atgcaaccgc cttgcactta aaggtccttt 3480
   ggtgtcaatg gatgaaatgg aatctatcaa gaagatgaac taccgtggtt ggcgcagcaa 3540
   ggtgctcgac ataacttatc cgaagaattc tggaaggaag ggcttggaag aaactttgga 3600
   tagaatttgt gctgaagccc gggaagctat acgngaggga tacaaaattt tagttctttc 3660
   agacagaggt cagtgactta ttcactttat acttgcatcc cattgtttga tggtacaaat 3720
   tactcataag ttgacaaaaa attctagcag atgggtcagg tgtgcacaat ttattatcgt 3780
   tagcttccag caaacatatc acatcaccat atagtaactt aaatatgatc ataattttgt 3840
   ttgatagtca attgtttgca gtgtaatgtg ttgtatgaag gacattctta ctttgtcacc 3900
   ttggacataa ttggaatgta tattaagttt attatgcaac tcaacatctt ccaatactga 3960
   aatttgttgt attttcttga ttcaggattt tcttcagacc gtgttgctgt cagttccctc 4020
   ttagcagttg gagcagtaca tcaacacctt gttgcaaatc ttgagaggac acgcgtagga 4080
   ttattggttg agtctgctga acctcgtgaa gtgcaccatt tctgtacact cgttggattt 4140
   ggtgcagatg ctatatgccc ttatttggcc attgaagcaa tttggtgctt gcagactgat 4200
   gggaagattc cccctaccga ctcaaaggag gaacttgtcg aaaaatattt ttatgcttcc 4260
   atctatggaa tgatgaaggt tcttgcaaag atgggaatat ccacccttgc atcttacaaa 4320
   ggggcacaga tttttgaagc tcttggactt tcttctgaag tgattcacaa gtgttttgaa 4380
   ggcactccta gcagaattga gggtgcaacg ttcgaaatgc ttgcacgtga tgctctccgt 4440
   cttcacgagt tggcattccc atcaagaaca cctccgcctg gcagtgcaga tgcaaaagcc 4500
   cttcccaatc caggggatta ccactggagg aaaaatggtg aagtccanct aaatgatcct 4560
   cttgcaatgg caaaattaca agaagcagct aaagtaaaca gccgggaagc atacaaagaa 4620
   tattctaagc gaattcaaga gcttaacaag gcgtgcaatc tgcgtggtat gctgaaattt 4680
   atagatagca ctagcaagat ttctttggat gaggttgaac ctgcaagtga gatagtgaaa 4740
   cgcttttgta ctggggcaat gagttatgga tctatttcgt tggaggcaca tactgccctt 4800
   gctgtggcca tgaacaaact gggaggcaaa tctaacacag gtatttcatg catatgtgac 4860
   attttttacc ctttcgctgg ttgttaccct tctgatgaaa tcatgtatag tagctttact 4920
   tgcacatcaa ctcttagagc cttgttgcat aacatatttt tcttattttt gctgcagtag 4980
   tgtagtgtct atgtttctac tgtttaaata tgccctcgaa gttggtactt tatacatgac 5040
   catgtctaga aatgcactac ttattttgat gctatcacat gaaaactata ctctcatagc 5100
   agtacaagta tttatcttcc tgttgatgtt gaatgcactt ctgtgaaaac tatactctca 5160
   tagtagtgtc tatgtttcta caagtattta tcacatgaaa actatactca cataaaacca 5220
   tttcatcttc ctgttgatgt tgaatgcact tctgtgaaca aattatccca gttcatagca 5280
   agagttaact tctaagcttt tatctatctg ttgatgttga atgcacttct ggtagcaaat 5340
   tatcctaatt cctagcaaga tttaacttct gcttgcgtag aaacccagac agcttgcacg 5400
   cattattctt ctttggactg tttgtattat agatattttc tgcccacaca gttaatatta 5460
   tattgccaaa tctttgtatt tattttcact cctaaactaa actcatttta cccatctgct 5520
   aatttagggg agggagggga gcagccttct cgtatggagc ctcttcctga tggttcgatg 5580
   aatccaaaac gaagtgcaat caagcaagtt gccagtggac gatttggagt ttccagctat 5640
   tatctgacta atgcagatga gctgcagata aaaatggctc aggtacttga caatgctgtg 5700
   cattgcgcaa tcactggaat gatctcagtt tatagaaagc aactgtattc aacgtcctgt 5760
   ggttggcatt tattcttttt ttgtatcgta tatgtatagg gtgctaagcc tggtgaaggt 5820
   ggtgagcttc caggtcacaa ggttatcggt gacattgcag ttaccagaca ttctacagct 5880
   ggtgttgggc ttattagtcc acctcctcac catgatatat attctatcga ggatcttgca 5940
   cagcttatcc atgaccttaa ggtacattac ctgatgctct tctatagcca aggcatctgc 6000
   ctatttatgt atggttgtta gttgctactt ctagattata ttggttatct aaacgctgtg 6060
   atggtctaac cctcctgaga agctagaatc acttgctaaa ctggtacaag tgatcctgca 6120
   catatgcatg tgtctccatt tgggggttta ccctcctttt tgaaaaactc attttgcaaa 6180
   tgttacctgc tgatgtagtg atattcatgc ctgttttctc tctgcagaat tcaaatcctc 6240
   aagctcgaat cagcgtgaag ctagtgtctg aggctggtgt tggagttgta gctagcggtg 6300
   ttgtaaaagg acacgcagac catgttctta tttctggcca tgacggtggc accggtgcat 6360
   caagatggac aggtatcaag aatgctggac ttccatgggg aactaggatt ggctgagaca 6420
   catcaaactt tagtggcaaa tgggcttcgt ggtcgagctg tcctacaaac agatggccaa 6480
   ttaaaaactg gtagagatgt tgctgtggcg tgcttacttg gtgcagagga atttggtttc 6540
   agcactgctc cactgatcac acttggctgc attatgatgc ggaagtgcca taccaatact 6600
   tgccctgttg gtatagccac tcaagatcca gtgctccgag aaaaatttgc tggagaacca 6660
   gagcatgtca ttaacttttt cttcatgctt gctgaggagc taagagaaat tatggctcag 6720
   cttggcctcc gtacaattaa tgaaatggtt ggacgctctg atatgcttga agttgatcca 6780
   gaagtagtta agagcaatga gaaacttgaa aatattgatc tctcattgat cttaaaacca 6840
   gctgcagaga ttcgtcctgg ggctgctcag tactgtgttg aaaagcaaga ccatggcctt 6900
   gacatggctt tggataacaa acttatagct ttatcaaggn ctgcacttga aaaggaagtt 6960
   cgtgttttcn ttgagactcc aatcaagaac actaatcggg cagtaggcac nacacttagc 7020
   catgaagtca caaaacgtta tcacatgaag ggattagatc ctggaaccat tcatgtgaaa 7080
   ctcactggaa gtgctggtca gagttttggt gcttttctct gtcctggaat aacccttgag 7140
   cttgaaggag acagcaatga ttacgttggc aaaggattat ctggtggaaa gattgttgtg 7200
   tacccaccca ggaacagtac atttagtgca gaagacaata ttgtcattgg taatgtggcc 7260
   ctgtatggtg ctaccaaggg agaagcatac ttcaatggaa tggcagcaga aaggttttgt 7320
   gttcgtaatt ctggtgctcg gacagtggtt gaaggaattg gtgatcatgg atgcgagtac 7380
   atgacagggg gtactgtagt catccttggt aaaacaggaa gaaattttgc tgctgggatg 7440
   agtggaggca tcgcttatgt ttatgatgtt gatgggacat tcagtgtccg ctgtaacaat 7500
   gagttggttg atctatatca tgtggaggaa gaggatgatg taaccacttt gaaaatgatg 7560
   atagagcaac atcgacttca cacagagagt gtcctggcca aagacatact ctctaaattc 7620
   gacactcttc ttccaaaatt tgtaaaagta tacccaaggg attataagag ggttctagaa 7680
   gaaatgaagg cagaaaaagc tgcagctagg cctacaaagg aacctaaggt ggcaaatggt 7740
   gtttctgtga caactaaggt aatatatgat ttaagtaata attcttctgc ataccttttt 7800
   aatttgaatt tatagtagag atggtgttct ccatttaggt ttatccatca gaaattgcaa 7860
   tgatacacat tgccactact gtgtgaactt ctatttttgc ctaagtagat taactttgat 7920
   ctacagaaaa tacaaacaga gaagtcatcc agccgaccaa cacgtgttgc caacgccaaa 7980
   aagtaccggg gttttgtaac atatgagcga gagggtgttt cttaccgtga tccaaatgag 8040
   cgtgttaaag attggaacga ggttgccatt gaatcagttc cagggccact gttaaacaca 8100
   cagtctgctc gttgtatgga ttgtggcact cctttctgtc atcaggtgaa gctgagttca 8160
   tcttttctat ttaacattga tgattgtagt tggatttatt gtggggttaa ggattgatat 8220
   gtttagtcat tggctacata tgacattgag atgctaagat ttcatgatta tttgtacagg 8280
   aaagctcagg tgctggctgt cctcttggaa ataagatccc agagttcaat gaattagttc 8340
   accagaatag atggcgtgaa gcattggatc gcctactaga gacaaacaac ttccctgaat 8400
   ttactggacg tgtgtgccct gctccttgtg aggggtcttg tgttcttggc attattgaga 8460
   acccagtatc aatcaaaagc atagaatgct cgattataga caaaggtttt gaagagggat 8520
   ggatggtacc acgaccacca cttcaaagaa cagggtatgt cttttttacc atctagttcg 8580
   gcttattccc ttatttattt acttctgttt cattaatagg taacatttga gttacacttt 8640
   ctaatataaa ctgttccttt cgcagaaaga aaatcgctat agttggcagt ggtcctgctg 8700
   gtttggctgc cgctgatcaa ctaaataaaa tgggccattt tgtaactgta tttgaacgtt 8760
   cggatcgtat aggaggtctt atgatgtatg gagtaccaaa catgaagaca gacaagattg 8820
   gagttgttca gcgtcgtgtc aatttaatgg ccgaagaggg tataacattt gtggtgaatg 8880
   ctaatgtagg gagtgatcct ttatactcaa ttgaacgtct ccgttctgag aacaatgcag 8940
   ttattttggc ttgtggagct acaaaaccaa ggtaactaat tggaacgagg attttttttt 9000
   tctagtttac tccaagagta gcaacaatct caaaagaact gacatatatc ttttgacact 9060
   aactgactaa catcatgtgg gatattcttg aacagggacc tcagtattcc tggccgtgag 9120
   ctagctggag ttcattttgc catggaattt ctccacgcaa ataccaaaag tttgcttgat 9180
   agcaacctgg aggatggaag atacatatct gcccagggta agaaggtggt ggtcattggt 9240
   ggtggagaca caggcacaga ttgcatcggt acgtctgtta ggcatggttg cagcagcatt 9300
   gtaaatctgg agcttctcac caagccacca agcaagagag cttctgacaa cccctggccc 9360
   caggtaaatg caaaaaaaaa aaactggata tttccctgca catgtgaatt tggccattcc 9420
   atttccagct tggaaagttc ttaagtcatc attctgtttt tgcatgcagt ggcctagagt 9480
   cttccgagtg gactatgggc accaggaagc atctaccaag tttggaaatg atccaagaac 9540
   ttacgaagtc ttaaccaagc gtttcattgg tgatgaagat ggaaaattga aggcccttga 9600
   ggtggtgcgc gtgaagtggg agaaagtaga tggaaaattc cagttcaagg agattgaagg 9660
   atcacaagag atcatcgagg cagaccttgt cctccttgcc atgggattcc tgggccctga 9720
   agaggttagt tatgcagact ctgaactact gtcacgcctg agttttatct tgatgataaa 9780
   tgttggacaa acaactcacc gttttatttc tctgcagaac atcgctgaca aactgggttt 9840
   agagaaagac aaccgttcca acttcaaagc tcaattcgga cacttcggga ccagtgtgga 9900
   tggcgttttt gccgctgggg attgcaggcg cgggcaatcg ctggttgttt gggccatcac 9960
   cgaagggcgt gaagctgctg ctgcagtaga taagtacttg tcaagggatg aacaaaatgt    10020
   cgcaggcctt acatag                                                    10036
<210> 25
   <211> 10180
   <212> DNA
   <213> Triticum aestivum
<220>
   <221> misc_feature
   <222> (2207)..(2207)
   <223> n is a, c, g, or t
<220>
   <221> misc_feature
   <222> (2222)..(2222)
   <223> n is a, c, g, or t
<220>
   <221> misc_feature
   <222> (2287)..(2287)
   <223> n is a, c, g, or t
<220>
   <221> misc_feature
   <222> (2306)..(2306)
   <223> n is a, c, g, or t
<220>
   <221> misc_feature
   <222> (2361)..(2361)
   <223> n is a, c, g, or t
<220>
   <221> misc_feature
   <222> (2418)..(2418)
   <223> n is a, c, g, or t
<220>
   <221> misc_feature
   <222> (3715)..(3715)
   <223> n is a, c, g, or t
<220>
   <221> misc_feature
   <222> (3814)..(3814)
   <223> n is a, c, g, or t
<220>
   <221> misc_feature
   <222> (3839)..(3839)
   <223> n is a, c, g, or t
<220>
   <221> misc_feature
   <222> (3858)..(3858)
   <223> n is a, c, g, or t
<220>
   <221> misc_feature
   <222> (5493)..(5493)
   <223> n is a, c, g, or t
<220>
   <221> misc_feature
   <222> (5498)..(5498)
   <223> n is a, c, g, or t
<220>
   <221> misc_feature
   <222> (7083)..(7083)
   <223> n is a, c, g, or t
<220>
   <221> misc_feature
   <222> (7113)..(7113)
   <223> n is a, c, g, or t
<220>
   <221> misc_feature
   <222> (7154)..(7154)
   <223> n is a, c, g, or t
<220>
   <221> misc_feature
   <222> (9005)..(9005)
   <223> n is a, c, g, or t
<220>
   <221> misc_feature
   <222> (9029)..(9029)
   <223> n is a, c, g, or t
<220>
   <221> misc_feature
   <222> (9185)..(9185)
   <223> n is a, c, g, or t
<220>
   <221> misc_feature
   <222> (9257)..(9257)
   <223> n is a, c, g, or t
<400> 25
   atgtcggcgg cgcaagggat tggtttgaag cacgcggcgc cgacgggcgt cggccgcagg        60
   gcccggcgca gccacgccgc gtccgcgcat ggccgctccg cgaggcaggc gcacggcgcc       120
   atgtctctgg agggcggcgg gttcctcggc ggcgcgcacc gcattgagga acgcgtcgcg       180
   ccatgcccgc ctcgggccgc ggcgcgcgac gcagagtcca tacggcccat gtctctgcta       240
   cccgagagca gcattgggct ctacaacccg gcgttcgagc gtgactcgtg cggcgttggt       300
   ttcgtcgccg agctgtcggg cgttgacaac cgggcgaccg tgagttcttt gcaccaagga       360
   caccgctgca gcttctctct tccatctaca gggttgagat attgatttgg ctgtgattta       420
   ctttacaggt cgtcgatgcc attcagatgc ttgaaagaat ggcacaccga ggtgcctgcg       480
   gctgtgagaa aaacactggt gatggtgccg gcattctcgt tgctctacca cacaacttct       540
   tccgagaggt gaatcaatga aaattgcaag caaactcagt tttccttgac agagttattc       600
   atcttgggtg tcgctctgct attgccgcag gtgacaaagg atgccggttt cgagttaccg       660
   ccaccaggtg agtatgctgt tggaatggtc ttcctgccaa ccgatgagaa gcgccgcgag 720
   aggagcaaaa ctgagtttac aaaggttggt tggttggtgt atagacaatt agacagcgtg 780
   cgaggcgagt ctgcgatggt aagcttatgt gttggatttt ttttattcat gcaggtcgct 840
   gagtcgctag gacattcgat acttgggtgg cgccaggttc ccactgacaa ttcagacttg 900
   ggccaagctg cgctcgacac tgaaccagcg attgaacagg ttttcctcac aaagagttca 960
   aaatcgaagg ccgacttcga acagcaggtt ctccatgagt ttaattttct ctcaacacat 1020
   gtaatgagct ttcctctcaa ctaacccatc aattatcaca gttgtttatc ctgaggaggc 1080
   tctcaattgt atctatccgg gccgcgctga atcttcagcg tggaggagag agagatttct 1140
   acatgtgctc tctatcttca aggtccttac ttcttctgaa ttgcattttc actacaaact 1200
   gatgaggttg agatcaggga gcccaattaa gctcctgact ttaatggaac attaacaaca 1260
   caaaagttga taggaaagct gactgggaaa aacaatcctg ggctatctct taagcattag 1320
   aaatctgtaa tgtaaggaca tttcattttt gctgtattca tgtgagtaat tattgttaaa 1380
   tcttctattg tggttttcca ggaccattgt ttacaagggc caacttatgc catcccagct 1440
   ccaagggtac tactatgcgg atataggtca tgaaaacttc tccagttata tggctctggt 1500
   aaatacgaaa ccttgatact cttacaacaa aagtcatggt cgtagtcagc ggatgggaat 1560
   caataatagc aagccagttg taagatcaag actcgctcct gtgcagacat caaatttgag 1620
   ctgtctattc atatgctatt tcagtcctgc ttcgtgatgc ctttaagtga agtactagaa 1680
   acttctctta gttgataccc gataaccatg ttgcagagtc cagctgcagt tctgctatgg 1740
   ccgactgctt gccgactctt caggatgtga ccgaagaaat tgtgtttggt ttgaggtagc 1800
   ttagggctcc caactgtttt gcatgaaatt atacgcagct gacgtatttt tacaaaattc 1860
   gttcttcaca aggaagtttt gttttgaata tctgcaaaag atgtttataa tatgatttct 1920
   aaaagtcata tcttgaaaca ggttcactca aggttctcca ccaacacctt ccccagctgg 1980
   gaccgtgcac agccaatgcg tgtcttaggc cacaatggag agatcaatac tctcaaaggg 2040
   aacaaaaact ggtaatatta ttgtcaaatc ttttcctctt tgctttaacc ttttcttggc 2100
   agatgcaact atctcaatat gcacgttgta tatatggcag gatgaaagct cgtgagggtc 2160
   tcttggagtg tgagaagctt ggcctatcac aggatgaaat gtcaaanatt cttccaatag 2220
   tngatgccac ttcttcagat tcaggtccgc agtctaatat gcagcatgaa ttccacttgg 2280
   caaatanaaa ttcatctcgg ttttgngagt tactaagtgt gaaaaaacat gtgaattcta 2340
   ggtgcatttg atggtgttct ngagctcctt atccgtggtg gaagaagcct gccagaagct 2400
   gtgatgatga tgatcccnga ggcgtggcag aatgatgtaa acatggaacc tgataagaaa 2460
   gctctgtacg agttcttgtc agcccttatg gagccttggg atggacctgc tctcatatct 2520
   tgtaaaaaac ttaaacccgt ttcttttttc tgatcatact tctaactgat tacttaagct 2580
   aacatgggta caacctgtag ttaccgatgg ccgctacctt ggagctaccc tggatcgcaa 2640
   tggccttagg cctggtcgat tttatgtaac ccacagtgga cgtgtggtca tgggtagtga 2700
   agttggtgtt gtggatattc ctgcccaaga tgtgttgaga aagggtcggc ttaaccctgg 2760
   aatgatgtta ctcgttgact tcgacgacca tactgtagta gatgatgaag cactcaaggc 2820
   acagtactct aaagctcacc cgtatggaga atggctcaag cgacaaaaga tgtacctcaa 2880
   agacattgta gaatctgtcc cagaaactga cagagttgct ccaagcattt ctggttctat 2940
   tacggtaagt acttacgtgg tccaacttcc tcattcactg ggtagtttgg tgatgaaaat 3000
   tggtaaacca taaggaacat ggtgatttta atattctttt acaatttggt gtagcaaaca 3060
   aatgagaaca aggaatgtgt aggcatcaat gcaattgtga ctccactaaa ggcgtttggg 3120
   taagcaagga ttgaacattg tcaactatca ctcatgtttc aatttctgct ggctaatgat 3180
   ccaaccgatt aactacaggt acacattgga agccctagaa atgttgctgc tgccaatggc 3240
   taaagatgga gtggaagctc ttggatcaat gggaaatgat gcacccctag cagtgatgtc 3300
   aaacagagag aagctgactt ttgagtactt caagcagatg tttgcacaag taacaaaccc 3360
   tccaatcgat ccaattaggg agaagattgt tacatctatg gaatgtatga ttgggccaga 3420
   aggagatttg ctggaaataa ccgaaaagca atgcaaccgc cttgcactta aaggtccttt 3480
   ggtgtcaatg gatgaaatgg aatctatcaa gaagatgaac taccgtggtt ggcgcagcaa 3540
   ggtgctcgac ataacttatc cgaagaattc tggaaggaag ggcttggaag aaactttgga 3600
   tagaatttgt gctgaagccc gggaagctat acgcaaggga tacaaaattt tagttctttc 3660
   agacagaggt cagtgactta ttcactttat acttgcatcc cattgtttga tggtncaaat 3720
   tactcataag ttgacaaaaa attctagcag atgggtcagg tgtgcacaat ttattatcgt 3780
   tagcttccag caaacatatc acatcaccat atantaactt aaatatgatc ataattttnt 3840
   ttgatagtca attgtttnca gtgtaatgtg ttgtatgaag gacattctta ctttgtcacc 3900
   ttggacataa ttggaatgta tattaagttt attatgcaac tcaacatctt ccaatactga 3960
   aatttgttgt attttcttga ttcaggattt tcttcagacc gtgttgctgt cagttccctc 4020
   ttagcagttg gagcagtaca tcaacacctt gttgcaaatc ttgagaggac acgcgtagga 4080
   ttattggttg agtctgctga acctcgtgaa gtgcaccatt tctgtacact cgttggattt 4140
   ggtgcagatg ctatatgccc ttatttggcc attgaagcaa tttggtgctt gcagactgat 4200
   gggaagattc cccctaccga ctcaaaggag gaacttgtcg aaaaatattt ttatgcttcc 4260
   atctatggaa tgatgaaggt tcttgcaaag atgggaatat ccacccttgc atcttacaaa 4320
   ggggcacaga tttttgaagc tcttggactt tcttctgaag tgattcacaa gtgttttgaa 4380
   ggcactccta gcagaattga gggtgcaacg ttcgaaatgc ttgcacgtga tgctctccgt 4440
   cttcacgagt tggcattccc atcaagaaca cctccgcctg gcagtgcaga tgcaaaagcc 4500
   cttcccaatc caggggatta ccactggagg aaaaatggtg aagtccatct aaatgatcct 4560
   cttgcaatgg caaaattaca agaagcagct aaagtaaaca gccgggaagc atacaaagaa 4620
   tattctaagc gaattcaaga gcttaacaag gcgtgcaatc tgcgtggtat gctgaaattt 4680
   atagatagca ctagcaagat ttctttggat gaggttgaac ctgcaagtga gatagtgaaa 4740
   cgcttttgta ctggggcaat gagttatgga tctatttcgt tggaggcaca tactgccctt 4800
   gctgtggcca tgaacaaact gggaggcaaa tctaacacag gtatttcatg catatgtgac 4860
   attttttacc ctttggctgg ttgttaccct tctgatgaaa tcatgtatag tagctttact 4920
   tgcacatcaa ctcttagagc cttgttgcat aacatatttt tcttattttt gctgcagtag 4980
   tgtagtgtct atgtttctac tgtttaaata tgccctcgaa gttggtactt tatacatgac 5040
   catgtctaga aatgcactac ttattttgat gctatcacat gaaaactata ctctcatatt 5100
   tacttgtgga catacttgta ctgcacttgt gtgtttggtt gcattctcgt ctcagcatag 5160
   ttccctcttc atgcatgctc aactcaacac ccctcttcat gcatgctttc ttcatgcatg 5220
   cttctagtgt atttgtgcta agctagtgtg gactcatgca ccctccatac actctaccaa 5280
   acacccaaaa gtgggtcgag aagggaactc ttgggctcat gcacccttca tacaccctac 5340
   caaacacacc cttaatgcgc taagcttttc atcttcctgt tgatgttgaa tgcacttctg 5400
   tgaacaaatt atcccagttc atagcaagag ttaacttcta agcttttatc tatctgttga 5460
   tgttgaatgc acttctggta gcaaattatc ctnattcnta gcaagattta acttctgctt 5520
   gcgtagaaac ccagacagct tgcacgcact attcttcttt ggactgtttg tattagatat 5580
   tttctgccca cacagttaat attatattgc caaatctttg tatttatttt cgctcctaaa 5640
   ctaaactcat tttacccatc tgctaattta ggggagggag gggagcagcc ttctcgtatg 5700
   gagcctcttc ctgatggttc gatgaatcca aaacgaagtg caatcaagca agttgccagt 5760
   ggacgatttg gagtttccag ctattatctg actaatgcag atgagctgca gataaaaatg 5820
   gctcaggtac ttgacaatgc tgtgcattgc gcaatcactg gaatgatctc agtttataga 5880
   aagcaactgt attcaacgtc ctgtggttgg catttattct ttttttgtat cgtatatgta 5940
   tagggtgcta agcctggtga aggtggtgag cttccaggtc acaaggttat cggtgacatt 6000
   gcagttacca gacattctac agctggtgtt gggcttatta gtccacctcc tcaccatgat 6060
   atatattcta tcgaggatct tgcacagctt atccatgacc ttaaggtaca ttacctgatg 6120
   ctcttctata gccaaggcat ctgcctattt atgtatggtt gttagttgct acttctagat 6180
   tatattggtt atctaaacgc tgtgatggtc taaccctcct gagaagctag aatcacttgc 6240
   taaactggta caagtgatcc tgcacatatg catgtgtctc catttggggg tttaccctcc 6300
   tttttgaaaa actcattttg caaatgttac ctgctgatgt agtgatattc atgcctgttt 6360
   tctctctgca gaattcaaat cctcaagctc gaatcagcgt gaagctagtg tctgaggctg 6420
   gtgttggagt tgtagctagc ggtgttgtaa aaggacacgc agaccatgtt cttatttctg 6480
   gccatgacgg tggcaccggt gcatcaagat ggacaggtat caagaatgct ggacttccat 6540
   ggggaactag gattggctga gacacatcaa actttagtgg caaatgggct tcgtggtcga 6600
   gctgtcctac aaacagatgg ccaattaaaa actggtagag atgttgctgt ggcgtgctta 6660
   cttggtgcag aggaatttgg tttcagcact gctccactga tcacacttgg ctgcattatg 6720
   atgcggaagt gccataccaa tacttgccct gttggtatag ccactcaaga tccagtgctc 6780
   cgagaaaaat ttgctggaga accagagcat gtcattaact ttttcttcat gcttgctgag 6840
   gagctaagag aaattatggc tcagcttggc ctccgtacaa ttaatgaaat ggttggacgc 6900
   tctgatatgc ttgaagttga tccagaagta gttaagagca atgagaaact tgaaaatatt 6960
   gatctctcat tgatcttaaa accagctgca gagattcgtc ctggggctgc tcagtactgt 7020
   gttgaaaagc aagaccatgc cttgacatgg ctttggataa caaacttata gctttatcaa 7080
   ggnctgcact tgaaaaggaa gttcgtgttt tcnttgagac tccaatcaag aacactaatc 7140
   gggcagtagg cacnacactt agccatgaag tcacaaaacg ttatcacatg aagggattag 7200
   atcctggaac cattcatgtg aaactcactg gaagtgctgg tcagagtttt ggtgcttttc 7260
   tctgtcctgg aataaccctt gagcttgaag gagacagcaa tgattacgtt ggcaaaggat 7320
   tatctggtgg aaagattgtt gtgtacccac ccaggaacag tacatttagt gcagaagaca 7380
   atattgtcat tggtaatgtg gccctgtatg gtgctaccaa gggagaagca tacttcaatg 7440
   gaatggcagc agaaaggttt tgtgttcgta attctggtgc tcgaacagtg gttgaaggaa 7500
   ttggtgatca tggatgcgag tacatgacag ggggtactgt agtcatcctt ggtaaaacag 7560
   gaagaaattt tgctgctggg atgagtggag gcatcgctta tgtttatgat gttgatggga 7620
   cattcagtgt ccgctgtaac aatgagttgg ttgatctata tcatgtggag gaagaggatg 7680
   atgtaaccac tttgaaaatg atgatagagc aacatcgact tcacacagag agtgtcctgg 7740
   ccaaagacat actctctaaa ttcgacactc ttcttccaaa atttgtaaaa gtatacccaa 7800
   gggattataa gagggttcta gaagaaatga aggcagaaaa agctgcagct aggcctacaa 7860
   aggaacctaa ggtggcaaat ggtgtttctg tgacaactaa ggtaatatat gatttaagta 7920
   ataattcttc tgcatacctt tttaatttga atttatagta gagatggtgt tctccattta 7980
   ggtttatcca tcagaaattg caatgataca cattgccact actgtgtgaa cttctatttt 8040
   tgcctaagta gattaacttt gatctacaga aaatacaaac agagaagtca tccagccgac 8100
   caacacgtgt tgccaacgcc aaaaagtacc ggggttttgt aacatatgag cgagagggtg 8160
   tttcttaccg tgatccaaat gagcgtgtta aagattggaa cgaggttgcc attgaatcag 8220
   ttccagggcc actgttaaac acacagtctg ctcgttgtat ggattgtggc actcctttct 8280
   gtcatcaggt gaagctgagt tcatcttttc tatttaacat tgatgattgt agttggattt 8340
   attgtggggt taaggattga tatgtttagt cattggctac atatgacatt gagatgctaa 8400
   gatttcatga ttatttgtac aggaaagctc aggtgctggc tgtcctcttg gaaataagat 8460
   cccagagttc aatgaattag ttcaccagaa tagatggcgt gaagcattgg atcgcctact     8520
   agagacaaac aacttccctg aatttactgg acgtgtgtgc cctgctcctt gtgaggggtc     8580
   ttgtgttctt ggcattattg agaacccagt atcaatcaaa agcatagaat gctcgattat     8640
   agacaaaggt tttgaagagg gatggatggt accacgacca ccacttcaaa gaacagggta     8700
   tgtctttttt accatctagt tcggcttatt cccttattta tttacttctg tttcattaat     8760
   aggtaacatt tgagttacac tttctaatat aaactgttcc tttcgcagaa agaaaatcgc     8820
   tatagttggc agtggtcctg ctggtttggc tgccgctgat caactaaata aaatgggcca     8880
   ttttgtaact gtatttgaac gttcggatcg tataggaggt cttatgatgt atggagtacc     8940
   aaacatgaag acagacaaga ttggagttgt tcagcgtcgt gtcaatttaa tggccgaaga     9000
   gggtntaaca tttgtggtga atgctaatnt agggagtgat cctttatact caattgaacg     9060
   tctccgttct gagaacaatg cagttatttt ggcttgtgga gctacaaaac caaggtaact     9120
   aattggaacg aggatttttt ttttctagtt tactccaaga gtagcaacaa tctcaaaaga     9180
   actgncatat atcttttgac actaactgac taacatcatg tgggatattc ttgaacaggg     9240
   acctcagtat tcctggncgt gagctagctg gagttcattt tgccatggaa tttctccacg     9300
   caaataccaa aagtttgctt gatagcaacc tggaggatgg aagatacata tctgcccagg     9360
   gtaagaaggt ggtggtcatt ggtggtggag acacaggcac agattgcatc ggtacgtctg     9420
   ttaggcatgg ttgcagcagc attgtaaatc tggagcttct caccaagcca ccaagcaaga     9480
   gagcttctga caacccctgg ccccaggtaa atgcaaaaaa aaaaaaactg gatatttccc     9540
   tgcacatgtg aatttggcca ttccatttcc agcttggaaa gttcttaagt catcattctg     9600
   tttttgcatg cagtggccta gagtcttccg agtggactat gggcaccagg aagcatctac     9660
   caagtttgga aatgatccaa gaacttacga agtcttaacc aagcgtttca ttggtgatga     9720
   agatggaaaa ttgaaggccc ttgaggtggt gcgcgtgaag tgggagaaag tagatggaaa     9780
   attccagttc aaggagattg aaggatcaca agagatcatc gaggcagacc ttgtcctcct     9840
   tgccatggga ttcctgggcc ctgaagaggt tagttatgca gactctgaac tactgtcacg     9900
   cctgagtttt atcttgatga taaatgttgg acaaacaact caccgtttta tttctctgca     9960
   gaacatcgct gacaaactgg gtttagagaa agacaaccgt tccaacttca aagctcaatt    10020
   cggacacttc gggaccagtg tggatggcgt ttttgccgct ggggattgca ggcgcgggca    10080
   atcgctggtt gtttgggcca tcaccgaagg gcgtgaagct gctgctgcag tagataagta    10140
   cttgtcaagg gatgaacaaa atgtcgcagg ccttacatag                          10180
<210> 26
   <211> 6435
   <212> DNA
   <213> Triticum aestivum
<400> 26
   atgccgacgg cgcaggggat tggtttgaag cacgcggcgc cgccgggcgg ccgcagggcc      60
   cgccgcagcc agtccgcggc cgcgccgggc cgctccgcaa ggcaggcgca cggcgccatg     120
   tccctggatg gcgggttcct cggcggcgcg cagcgcaccg aggaacgcgt cgcgccacgc     180
   ccgcctcggg ccgcggcgcg cgacgccgag tccatcaggc ccatgtctct gctacccgag     240
   agcagcattg ggctgtacga cccggcgttc gagcgtgact cgtgcggcgt tggcttcgtc     300
   gccgagctgt cgggcgttga caaccgggcg accgtcgtcg atgccattca gatgcttgaa     360
   agaatggcac accgaggtgc ctgcggctgt gagaaaaaca ctggtgatgg tgccggcatt     420
   ctcgttgctc taccacacac cttcttccga gaggtgacaa aggatgccgg tttcgagtta     480
   ccgccaccag gtgagtatgc tgttggaatg gtcttcctgc caaccgatga gaagcgccgc     540
   gagaggagca aaactgagtt tacaaaggtc gctgagtctc taggacattc gatacttggg     600
   tggcgccagg ttcccactga caattcagac ttgggccaag ctgcgctcga tactgaacca     660
   gccattgaac aggttttcct caccaagagt tcaaaatcga aggccgactt cgaacagcag     720
   ttgtttatcc tgaggaggct ctcaattgta tctatccggg ccgcgctgaa tcttcagcgt     780
   ggaggagaga gagatttcta catgtgctct ctatcttcaa ggaccattgt ttacaagggc     840
   caacttatgc catcccagct ccaagggtac tactatgcgg atataggtca tgaaaacttc     900
   tccagttata tggctctggt tcactcaagg ttctccacca acaccttccc cagctgggac     960
   cgtgcacagc caatgcgtgt cttaggccac aatggagaga tcaatactct caaagggaac    1020
   aaaaactgga tgaaagctcg tgagggtctc ttggagtgtg agaagcttgg cctatcacag    1080
   gatgaaatgt caaagattct tccaatagta gatgccactt cttcagattc aggtgcattt    1140
   gatggtgttc tcgagctcct tatccgtggt ggaagaagcc tgccagaagc tgtgatgatg    1200
   atgatccctg aggcgtggca gaatgatgta aacatggaac ctgataagaa agctctgtac    1260
   gagttcttgt cagcccttat ggagccttgg gatggacctg ctctcatatc ttttaccgat    1320
   ggccgctacc ttggagctac cctggatcgc aatggcctta ggcctggtcg attttatgta    1380
   acccacagtg gacgtgtggt catgggtagt gaagttggtg ttgtggatat tcctgcccaa    1440
   gatgtgttga gaaagggtcg gcttaaccct ggaatgatgt tactcgttga cttcgacaac    1500
   catactgtag tagatgatga agcactcaag gcacagtact ctaaagctca cccgtatgga    1560
   gaatggctca agcgacaaaa gatgtacctc aaagacattg tagaatctgt cccagaaact    1620
   gacagagttg ctccaagcat ttctggttct attacgcaaa caaatgagaa caaggaatgt    1680
   gtaggcatca atgcaattgt gactccacta aaggcgtttg ggtacacatt ggaagcccta    1740
   gaaatgttgc tgctgccaat ggctaaagat ggagtggaag ctcttggatc aatgggaaat    1800
   gatgcacccc tagcagtgat gtcaaacaga gagaagctga cttttgagta cttcaagcag    1860
   atgtttgcac aagtaacaaa ccctccaatc gatccaatta gggagaagat tgttacatct    1920
   atggaatgta tgattgggcc agaaggagat ttgctggaaa taaccgaaaa gcaatgcaac    1980
   cgccttgcac ttaaaggtcc tttggtgtca atggatgaaa tggaatctat caagaagatg 2040
   aactaccgtg gttggcgcag caaggtgctc gacataactt atccgaagaa ttctggaagg 2100
   aagggcttgg aagaaacttt ggatagaatt tgtgctgaag cccgggaagc tatacgcgag 2160
   ggatacaaaa ttttagttct ttcagacaga ggattttctt cagaccgtgt tgctgtcagt 2220
   tccctcttag cagttggagc agtacatcaa caccttgttg caaatcttga gaggacacgc 2280
   gtaggattat tggttgagtc tgctgaacct cgtgaagtgc accatttctg tacactcgtt 2340
   ggatttggtg cagatgctat atgcccttat ttggccattg aagcaatttg gtgcttgcag 2400
   actgatggga agattccccc taccgactca aaggaggaac ttgtcgaaaa atatttttat 2460
   gcttccatct atggaatgat gaaggttctt gcaaagatgg gaatatccac ccttgcatct 2520
   tacaaagggg cacagatttt tgaagctctt ggactttctt ctgaagtgat tcacaagtgt 2580
   tttgaaggca ctcctagcag aattgagggt gcaacgttcg aaatgcttgc acgtgatgct 2640
   ctccgtcttc acgagttggc attcccatca agaacacctc cgcctggcag tgcagatgca 2700
   aaagcccttc ccaatccagg ggattaccac tggaggaaaa atggtgaagt ccatctaaat 2760
   gatcctcttg caatggcaaa attacaagaa gcagctaaag taaacagccg ggaagcatac 2820
   aaagaatatt ctaagcgaat tcaagagctt aacaaggcgt gcaatctgcg tggtatgctg 2880
   aaatttatag atagcactag caagatttct ttggatgagg ttgaacctgc aagtgagata 2940
   gtgaaacgct tttgtactgg ggcaatgagt tatggatcta tttcgttgga ggcacatact 3000
   gcccttgctg tggccatgaa caaactggga ggcaaatcta acacagggga gggaggggag 3060
   cagccttctc gtatggagcc tcttcctgat ggttcgatga atccaaaacg aagtgcaatc 3120
   aagcaagttg ccagtggacg atttggagtt tccagctatt atctgactaa tgcagatggg 3180
   ctgcagataa aaatggctca gggtgctaag cctggtgaag gtggtgagct tccaggtcac 3240
   aaggttatcg gtgacattgc agttaccaga cattctacag ctggtgttgg gcttattagt 3300
   ccacctcctc accatgatat atattctatc gaggatcttg cacagcttat ccatgacctt 3360
   aagaattcaa atcctcaagc tcgaatcagc gtgaagctag tgtctgaggc tggtgttgga 3420
   gttgtagcta gcggtgttgt aaaaggacac gcagaccatg ttcttatttc tggccatgac 3480
   ggtggcaccg gtgcatcaag atggacaggt atcaagaatg ctggacttcc atgggaacta 3540
   ggattggctg agacacatca aactttagtg gcaaatgggc ttcgtggtcg agctgtccta 3600
   caaacagatg gccaattaaa aactggtaga gatgttgctg tggcgtgctt acttggtgca 3660
   gaggaatttg gtttcagcac tgctccactg atcacacttg gctgcattat gatgcggaag 3720
   tgccatacca atacttgccc tgttggtata gccactcaag atccagtgct ccgagaaaaa 3780
   tttgctggag aaccagagca tgtcattaac tttttcttca tgcttgctga ggagctaaga 3840
   gaaattatgg ctcagcttgg cctccgtaca attaatgaaa tggttggacg ctctgatatg 3900
   cttgaagttg atccagaagt agttaagagc aatgagaaac ttgaaaatat tgatctctca 3960
   ttgatcttaa aaccagctgc agagattcgt cctggggctg ctcagtactg tgttgaaaag 4020
   caagaccatg gccttgacat ggctttggat aacaaactta tagctttatc aagggctgca 4080
   cttgaaaagg aagttcgtgt tttcattgag actccaatca agaacactaa tcgggcagta 4140
   ggcaccacac ttagccatga agtcacaaaa cgttatcaca tgaagggatt agatcctgga 4200
   accattcatg tgaaactcac tggaagtgct ggtcagagtt ttggtgcttt tctctgtcct 4260
   ggaataaccc ttgagcttga aggagacagc aatgattacg ttggcaaagg attatctggt 4320
   ggaaagattg ttgtgtaccc acccaggaac agtacattta gtgcagaaga caatattgtc 4380
   attggtaatg tggccctgta tggtgctacc aagggagaag catacttcaa tggaatggca 4440
   gcagaaaggt tttgtgttcg taattctggt gctcgaacag tggttgaagg aattggtgat 4500
   catggatgcg agtacatgac agggggtact gtagtcatcc ttggtaaaac aggaagaaat 4560
   tttgctgctg ggatgagtgg aggcatcgct tatgtttatg atgttgatgg gacattcagt 4620
   gtccgctgta acaatgagtt ggttgatcta tatcatgtgg aggaagagga tgatgtaacc 4680
   actttgaaaa tgatgataga gcaacatcga cttcacacag agagtgtcct ggccaaagac 4740
   atactctcta aattcgacac tcttcttcca aaatttgtaa aagtataccc aagggattat 4800
   aagagggttc tagaagaaat gaaggcagaa aaagctgcag ctaggcctac aaaggaacct 4860
   aaggtggcaa atggtgtttc tgtgacaact aagaaaatac aaacagagaa gtcatccagc 4920
   cgaccaacac gtgttgccaa cgccaaaaag taccggggtt ttgtaacata tgagcgagag 4980
   ggtgtttctt accgtgatcc aaatgagcgt gttaaagatt ggaacgaggt tgccattgaa 5040
   tcagttccag ggccactgtt aaacacacag tctgctcgtt gtatggattg tggcactcct 5100
   ttctgtcatc aggaaagctc aggtgctggc tgtcctcttg gaaataagat cccagagttc 5160
   aatgaattag ttcaccagaa tagatggcgt gaagcattgg atcgcctact agagacaaac 5220
   aacttccctg aatttactgg acgtgtgtgc cctgctcctt gtgaggggtc ttgtgttctt 5280
   ggcattattg agaacccagt atcaatcaaa agcatagaat gctcgattat agacaaaggt 5340
   tttgaagagg gatggatggt accacgacca ccacttcaaa gaacaggaaa gaaaatcgct 5400
   atagttggca gtggtcctgc tggtttggct gccgctgatc aactaaataa aatgggccat 5460
   tttgtaactg tatttgaacg ttcggatcgt ataggaggtc ttatgatgta tggagtacca 5520
   aacatgaaga cagacaagat tggagttgtt cagcgtcgtg tcaatttaat ggccgaagag 5580
   ggtgtaacat ttgtggtgaa tgctaatgta gggagtgatc ctttatactc aattgaacgt 5640
   ctccattctg agaacaatgc agttattttg gcttgtggag ctacaaaacc aagggacctc 5700
   agtattcctg gccgtgagct agctggagtt cattttgcca tggaatttct ccacgcaaat 5760
   accaaaagtt tgcttgatag caacctggag gatggaagat acatatctgc ccagggtaag 5820
   aaggtggtgg tcattggtgg tggagacaca ggcacagatt gcatcggtac gtctgttagg 5880
   catggttgca gcagcattgt aaatctggag cttctcacca agccaccaag caagagagct 5940
   tctgacaacc cctggcccca gtggcctaga gtcttccgag tggactatgg gcaccaggaa 6000
   gcatctacca aatttggaaa tgatccaaga acttacgaag tcttaaccaa gcgtttcatt 6060
   ggtgatgaag atggaaaatt gaaggccctt gaggtggtgc gcgtgaagtg ggagaaagta 6120
   gatggaaaat tccagttcaa ggagattgaa ggatcacaag agatcatcga ggcagacctt 6180
   gtcctccttg ccatgggatt cctgggccct gaagagaaca tcgctgacaa actgggttta 6240
   gagaaagaca accgttccaa cttcaaagct caattcggac acttcgggac cagtgtggat 6300
   ggcgtttttg ccgctgggga ttgcaggcgc gggcaatcgc tggttgtttg ggccatcacc 6360
   gaagggcgtg aagctgctgc tgcagtagat aagtacttgt caagggatga acaaaatgtc 6420
   gcaggcctta catag 6435
<210> 27
   <211> 23
   <212> DNA
   <213> Artificial Sequence
<220>
   <223> Primer
<400> 27
   cgagcttgag gatttgagtt cta 23
<210> 28
   <211> 22
   <212> DNA
   <213> Artificial Sequence
<220>
   <223> Primer
<400> 28
   cacttgctaa actggtataa tg 22
<210> 29
   <211> 19
   <212> DNA
   <213> Artificial Sequence
<220>
   <223> Primer
<400> 29
   tcgctgagtc tctaggaca 19
<210> 30
   <211> 20
   <212> DNA
   <213> Artificial Sequence
<220>
   <223> Primer
<400> 30
   gttcaatggc tggttcagta 20
<210> 31
   <211> 24
   <212> DNA
   <213> Artificial Sequence
<220>
   <223> Primer
<400> 31
   ggatttgaat tctgcagaga gaaa 24
<210> 32
   <211> 22
   <212> DNA
   <213> Artificial Sequence
<220>
   <223> Primer
<400> 32
   cacttgctaa actggtacaa gt 22
<210> 33
   <211> 19
   <212> DNA
   <213> Artificial Sequence
<220>
   <223> Primer
<400> 33
   ctacagagag aagacaggc 19
<210> 34
   <211> 25
   <212> DNA
   <213> Artificial Sequence
<220>
   <223> Primer
<400> 34
   gtacaattga tcctgcacat atact 25
<210> 35
   <211> 301
   <212> DNA
   <213> Triticum aestivum
<400> 35
   agacaatatt gtcattggta atgtggccct gtatggtgct accaagggag aagcatactt        60
   caatggaatg gcagcagaaa ggttttgtgt tcgtaattct ggtgctcgra cagtggttga       120
   aggaattggt gatcatggat gcgagtacat gacagggggt actgtagtca tccttggtaa       180
   aacaggaaga aattttgctg ctgggatgag tggaggcatc gcttatgttt atgatgttga       240
   tgggacattc agtgtccgct gtaacaatga gttggttgat ctatatcatg tggaggaaga       300
   g                                                                       301
<210> 36
   <211> 196
   <212> DNA
   <213> Triticum aestivum
<220>
   <221> variation
   <222> (112)..(112)
   <223> the adenine can be deleted
<400> 36
   gtctgttagg catggttgca gcagcattgt aaatctggag cttctcacca agccaccaag        60
   caagagagct tctgacaacc cctggcccca ggtaaatgca aaaaaaaaaa aactggatat       120
   ttccctgcac atgtgaattt ggccattcca tttccagctt ggaaagttct taagtcatca       180
   ttctgttttt gcatgc                                                       196
<210> 37
   <211> 206
   <212> DNA
   <213> Triticum aestivum
<400> 37
   gggagggagg ggagcagcct tctcgtatgg agcctcttcc tgatggttcg atgaatccaa        60
   aacgaagtgc aatcaagcaa gttgccagtg gacgatttgg agtttccagc tattatctga       120
   ctaatgcaga tgrgctgcag ataaaaatgg ctcaggtact tgacaatgct gtgcattgcg       180
   caatcactgg aatgatctca gtttat                                            206
<210> 38
   <211> 179
   <212> DNA
   <213> Triticum aestivum
<400> 38
   taacatttgt ggtgaatgct aatgtaggga gtgatccttt atactcaatt gaacgtctcc        60
   rttctgagaa caatgcagtt attttggctt gtggagctac aaaaccaagg taactaattg       120
   gaacgaggat tttttttttc tagtttactc caagagtagc aacaatctca aaagaactg         179
<210> 39
   <211> 518
   <212> DNA
   <213> Triticum aestivum
<400> 39
   aggtacacat tggaagccct agaaatgttg ctgctgccaa tggctaaaga tggagtggaa        60
   gctcttggat caatgggaaa tgatgcaccc ctagcagtga tgtcaaacag agagaagctg       120
   acttttgagt acttcaagca gatgtttgca caagtaacaa accctccaat cgatccaatt       180
   agggagaaga ttgttacatc tatggaatgt atgattgggc cagaaggaga tttgctggaa       240
   ataaccgaaa agcaatgcaa ccgccttgca cttaaaggtc ctttggtgtc aatggatgaa       300
   atggaatcta tcaagaagat gaactaccgt ggttggcgca gcaaggtgct cgacataact       360
   tatccgaaga attctggaag gaagggcttg gaagaaactt tggatagaat ttgtgctgaa       420
   gcccgggaag ctatacgcra gggatacaaa attttagttc tttcagacag aggtcagtga       480
   cttattcact ttatacttgc atcccattgt ttgatggt                               518
<210> 40
   <211> 227
   <212> DNA
   <213> Triticum aestivum
<400> 40
   atcataattt tgtttgatag tcaattgttt ccagtgtaat gtgttgtatg aaggacattc        60
   ttactttgtc accttggaca taactggaat gtatattaag tttatyatgc aactcaatat       120
   cttccaatac tgaaatttgt tgtattttct tgattcagga ttttcttcag atcgtgttgc       180
   tgtcagttcc ctcttagcag ttggagcagt acatcaacac cttgttg                     227

## Description

The present invention relates to methods for controlling yield of a plant, preferably a wheat plant, through the modulation of NADH-dependent glutamate synthase (NADH-GoGAT) activity.

High grain yield with adequate protein content is an important goal in crop improvement especially in bread wheat (*Triticum aestivum L*.) and maize (*Zea mays*). Unfortunately, it has been shown in various cereals including wheat that these two traits are genetically negatively correlated either in extensive North American farming or in intensive farming in Europe (Simmonds 1995; Oury *et al*., 2003). This correlation can be broken down by adequate nitrogen (N) supply late in the plant development (Krapp *et al*., 2005; Laperche *et al*., 2006). Nitrogen fertilizers are used as an important agronomic tool to improve output quantity as well as quality in all cultivated crops. However, the current agricultural and economic environment concerns impose farmers to constantly optimize the application of nitrogen fertilizers in order to avoid pollution by nitrates and preserve their economic margin.

Therefore, the selection for cereal cultivars that absorb and metabolize nitrogen in the most efficient way for grain or silage production is becoming increasingly important. Such improved crops would make a better use of nitrogen fertilizer supplies as they would produce higher yields with better protein content. This might be achieved, at least in part, through a better understanding of nitrogen metabolism and its regulation, and by identifying target genes to monitor nitrogen uptake by either direct gene transfer or marker-assisted breeding. Either directly for the grain protein content or indirectly for the photosynthetic production in plant, nitrogen uptake is an essential element in crop improvement.

Some genetic variability in nitrogen use efficiency (NUE) and its components, namely nitrogen uptake and nitrogen utilization, has been reported in rice (Borrell *et al*., 1998) and wheat (Le Gouis *et al*., 2000). Further, various QTL (Quantitative Trait Loci) analyses for NUE have been performed during the last decades for barley (Kjaer and Jensen, 1995), maize (Agrama *et al*., 1999; Bertin and Gallais, 2001; Hirel *et al*., 2001) rice (Obara *et al*., 2001; Lian *et al*., 2005), and wheat (An *et al.* 2006; Laperche *et al*., 2007; Habash *et al*., 2007; Fontaine *et al*., 2009), and for *Arabidopsis thaliana* (Rauh *et al*., 2002; Loudet *et al*., 2003). Major enzyme coding genes have been cloned and shown to drive nitrogen economy in plants (for review Miin and Habash, 2002; Bernard and Habash, 2009).

The glutamine synthetase (GS; E.C.6.3.1.2) is the first key enzyme for nitrogen metabolism, as it catalyses the assimilation of all inorganic nitrogen incorporated into organic compounds, such as proteins and nucleic acids. This reaction is coupled to the formation of glutamate by glutamate synthase (GoGAT) as part of the GS/GoGAT cycle.

In rice, two GoGAT types have been identified: a Ferredoxin (Fd)-dependent GoGAT (E.C. 1.4.7.1) and a NADH-dependent GoGAT (E.C. 1.4.1.14). Fd-GoGAT is known to be involved in photorespiration (Ireland and Lea, 1999). NADH-GoGAT is active in developing organs, such as unexpanded non-green leaves and developing grains (Yamaya *et al*., 1992).

NADH-GoGAT catalyzes the reductive transfer of amide group of glutamine to 2-oxoglutarate to form two glutamate molecule (Krapp *et al*., 2005):

2 L-glutamate + NAD⁺ ⇄ L-glutamine + 2-oxoglutarate + NADH + H⁺.

It is hypothesized that NADH-GoGAT is probably involved in the utilization of remobilized nitrogen, since this protein is located in the specific cell types which are important for solute transport from the phloem and xylem elements (Hayakawa *et al*., 1994). Yamaya *et al.* (2002) reported that, in rice, GoGAT enhances the grain filling suggesting that it is one of the potential candidate genes for NUE determinant. However, the authors have shown that in T0 transgenic rice plants over-producing NADH-GoGAT, the rate of increase in the NADH-GoGAT protein content in unexpanded non-green leaf blades was inversely correlated with that the one spikelet weight and the panicle weight.

Further, although Ferredoxin-GoGAT plays a critical part in the re-assimilation of ammonium released by glycine decarboxylase during photorespiration, NADH-GoGAT is involved in the assimilation of ammonium from both primary and secondary sources during nitrogen remobilization (Lea and Miin, 2003).

Genes coding for these two key enzymes involved in the NH₄ assimilation (*GS* and *NADH-GoGAT*) have been cloned in monocots such as rice (Tabuchi *et al*., 2007 for both *GS* and *NADH-GoGAT*; Cai *et al.,* 2009 for *GS*), wheat (Caputo *et al*., 2009 showing a physiological role of GS in the modulation of amino acids export levels in wheat) and maize (Valadier *et al*., 2008 for both *GS* and *NADH-GoGAT*); and eudicots such as *Arabidopsis* (Ishiyama *et al*., 2004 for *GS*; Potel *et al*., 2009 for *NADH-GoGAT*), Brassicaceae (Ochs *et al*., 1999 for *GS*) and *Medicago* (Lima *et al*., 2006 for *GS*).

Bread wheat is a hexaploid species with three diploid genomes named A, B and D; each genome consisting of seven pairs of chromosomes. The interactions between these 3 genomes are still unclear. Several putative *NADH-GoGAT* expressed sequence tags (ESTs), homolog to *NADH-GoGAT* ESTs in rice, have been found in bread wheat. However, until now, the functional ortholog of rice *NADH-GoGAT* has not been cloned in bread wheat.

The Inventors have now found, in bread wheat, a *NADH-GoGAT* gene which plays a major role in driving NUE. This gene is located on chromosome 3B.

The Inventors have also found that the wheat NADH-GoGAT proteins playing a major role in driving NUE show at least 98% identity between them and that such a wheat NADH-GoGAT protein has a percent identity inferior or equal to 95% with the rice NADH-GoGAT, whose the amino acid sequence is available in GENBANK database under accession number GI: 115439209 (and herein reproduced as SEQ ID NO: 6).

This finding from the Inventors that NADH-GoGAT protein plays a major role in driving NUE in wheat can also apply to other plants such as maize.

Accordingly, the present invention provides a method for improving the grain filling of a plant, preferably a wheat plant wherein said method comprises overexpressing in said plant a NADH-dependent glutamate synthase (NADH-GoGAT) having at least 95% identity, or by order of increasing preference at least 96%, 97%, 98% or 99% identity, with the polypeptide of sequence SEQ ID NO: 1.

Unless otherwise specified, the percents of identity between two sequences which are mentioned herein are calculated from an alignment of the two sequences over their whole length.

The term "overexpressing" a NADH-dependent glutamate synthase (NADH-GoGAT) in a plant, herein refers to artificially increasing the quantity of said NADH-GoGAT produced in said plant compared to a reference (control) plant.

The term "plant" includes any monocot or dicot plant producing edible seeds. Preferably, said plant is a wheat plant.

The terms "wheat plant" and "wheat plant cell" as used herein, include any plant or plant cell of the genus *Triticum*, preferably of the species *Triticum aestivum L.* (bread wheat).

The terms "maize plant" and "maize plant cell" as used herein, include any plant or plant cell of the genus *Zea*, preferably of the species *Zea mays*, more preferably of the subspecies *Zea mays mays.*

According to a preferred embodiment of the invention the grain filling is improved by increasing the grain weight and/or the grain protein content.

Advantageously, the improvement of the grain filling involves an improvement of the grain yield, either in limited or non-limited nitrogen supply condition.

According to another preferred embodiment of the invention said NADH-GoGAT has the amino acid sequence SEQ ID NO: 22, which corresponds to the NADH-GoGAT amino acid sequence of the bread wheat cultivar *Chinese Spring.* This sequence has 99.6% identity with the sequence SEQ ID NO: 1.

According to another preferred embodiment of the invention said NADH-GoGAT is encoded by a nucleotide sequence selected from the group consisting of SEQ ID NO: 2, SEQ ID NO: 3, SEQ ID NO: 4, SEQ ID NO: 23, SEQ ID NO: 24 and SEQ ID NO: 25, more preferably by a nucleotide sequence selected from the group consisting of SEQ ID NO: 23, SEQ ID NO: 24 and SEQ ID NO: 25, which correspond respectively to the genomic DNA sequences (allele) encoding the NADH-GoGAT protein of the bread wheat cultivars *Chinese Spring, Arche* and *Récital.*

According to another preferred embodiment of the invention said NADH-GoGAT is encoded by the nucleotide sequence SEQ ID NO: 5 or SEQ ID NO: 26, preferably by the nucleotide sequence SEQ ID NO: 26, which corresponds to the coding DNA sequence (CDS) of *Chinese Spring NADH-GoGAT* gene.

A preferred method for overexpressing a NADH-GoGAT comprises introducing into the genome of said plant a DNA construct comprising a nucleotide sequence encoding said NADH-GoGAT, placed under control of a promoter.

The instant invention also provides means for carrying out said overexpression.

This includes, in particular, recombinant DNA constructs for expressing a NADH-GoGAT in a host-cell (*e.g*., plant cell), or a host organism, in particular a wheat plant cell or a wheat plant. These DNA constructs can be obtained and introduced in said host cell or organism by the well-known techniques of recombinant DNA and genetic engineering.

Recombinant DNA constructs of the invention include in particular expression cassettes, comprising a polynucleotide encoding a NADH-GoGAT as defined above, under control of a heterologous promoter functional in plant cell.

The expression cassette of the invention may comprise a polynucleotide encoding at least two identical or different NADH-GoGAT as defined above.

The heterologous promoter of the invention is any promoter functional in a plant cell, *i.e*., capable of directing transcription of a polynucleotide encoding a NADH-GoGAT as defined above, in said plant cell. The choice of the more appropriate promoter may depend in particular on the organ(s) or tissue(s) targeted for expression, and on the type of expression (*i.e.* constitutive or inducible) that one wishes to obtain.

A large choice of promoters suitable for expression of heterologous genes in plants is available in the art. They can be obtained for instance from plants, plant viruses, or bacteria such as *Agrobacterium.* They include constitutive promoters, *i.e.* promoters which are active in most tissues and cells and under most environmental conditions, tissue or cell specific promoters which are active only or mainly in certain tissues or certain cell types, and inducible promoters that are activated by physical or chemical stimuli.

Non-limitative examples of constitutive promoters that are commonly used are the cauliflower mosaic virus (CaMV) 35S promoter, the nopaline synthase (Nos) promoter, the Cassava vein Mosaic Virus (CsVMV) promoter (Verdaguer *et al*., 1996), the rice actin promoter followed by the rice actin intron (RAP-RAI) contained in the plasmid pAct1-F4 (McElroy *et al*., 1991).

Non-limitative examples of organ or tissue specific promoters that can be used in the present invention include for instance High Molecular Weight (HMW) promoter which is kernel specific (Thomas and Flavell, 1990), or the leaf specific promoters as pPEPc promoter (Jeanneau *et al*., 2002), or the Rubisco small subunit promoter (rbcS) (Katayama *et al*., 2000) which is specific of the bundle-sheath, or the root specific promoter PRO110 from rice (International Application WO 2004/070039).

Inducible promoters include for instance drought stress responsive promoters, such as the rd29A promoter which comprises a dehydration-responsive element (Kasuga *et al*., 1999; Narusaka *et al*., 2003), or the senescence specific SAG12 promoter (Noh and Amasino, 1999).

The expression cassettes generally also include a transcriptional terminator, such as the 35S transcriptional terminator or Nos terminator (Depicker *et al*., 1982). They may also include other regulatory sequences, such as transcription enhancer sequences.

Recombinant DNA constructs of the invention also include recombinant vectors containing an expression cassette comprising a polynucleotide encoding a NADH-GoGAT as defined above, under transcriptional control of a suitable promoter. Said expression cassette may be a recombinant expression cassette of the invention, or a cassette wherein the polynucleotide encoding a NADH-GoGAT is under control of its endogenous promoter.

A recombinant vector of the invention may include at least two polynucleotides encoding two identical or different NADH-GoGAT as defined above.

Recombinant vectors of the invention may also include other sequences of interest, such as, for instance, one or more marker genes, which allow for selection of transformed hosts.

Advantageously, the selectable marker gene is comprised between two Ds (*Dissociation*) elements (*i.e*., transposons) in order for its removal at a later stage by interacting with the Ac (Activator) transposase. This elimination system is known from one skilled in the art. By way of example, it has been described in Goldsbrough *et al.* (1993).

The selection of suitable vectors and the methods for inserting DNA constructs therein are well known to persons of ordinary skill in the art. The choice of the vector depends on the intended host and on the intended method of transformation of said host.

A variety of techniques for genetic transformation of plant cells (*e.g*., wheat or maize plant cells), or plants (*e.g*., wheat or maize plants) are available in the art. By way of non-limitative examples, one can mention methods of direct transfer of genes such as direct micro-injection into plant embryoids, vacuum infiltration (Bechtold *et al.* 1993) or electroporation (Chupeau *et al*., 1989), or the bombardment by gun of particules covered with the plasmidic DNA of interest (Fromm *et al*., 1990; Finer *et al*., 1992). *Agrobacterium* mediated transformation methods may also be used such as *Agrobacterium tumefaciens*, in particular according to the method described in the article by An *et al.* (1986), or *Agrobacterium rhizogenes*, in particular according to the method described in the article by Guerche *et al*., (1987). According to a particular embodiment, it is possible to use the method described by Ishida *et al.* (1996) for the transformation of maize. According to another embodiment, the wheat is transformed according to the method described in International Application WO 00/63398.

The invention also comprises host cells containing a recombinant DNA construct of the invention. These host cells can be prokaryotic cells or eukaryotic cells, in particular plant cells, and preferably wheat plant cells.

The invention also provides a method for producing a transgenic plant, preferably a transgenic wheat or maize plant, having an improved grain filling. Said method comprises transforming a plant cell by a DNA construct of the invention and regenerating from said plant cell a transgenic plant overexpressing a NADH-GoGAT as defined above.

According to a preferred embodiment of the method of the invention, it comprises transforming a plant cell by a recombinant vector of the invention comprising a polynucleotide encoding a NADH-GoGAT as defined above, and regenerating from said plant cell a transgenic plant overexpressing a NADH-GoGAT as defined above.

The invention also comprises plants, preferably wheat plants, genetically transformed by a recombinant DNA construct of the invention, and overexpressing a NADH-GoGAT as defined above. In said transgenic plants a DNA construct of the invention is comprised in a transgene stably integrated in the plant genome, so that it is passed onto successive plant generations. Thus the transgenic plants of the invention include not only the plants resulting from the initial transgenesis, but also their descendants, as far as they contain a recombinant DNA construct of the invention. The overexpression of a NADH-GoGAT as defined above in said plants provides them an improved grain filling, when compared with a plant devoid of said transgene(s).

The invention also comprises a transgenic plant, preferably a transgenic wheat plant, obtainable by a method of the invention, overexpressing a NADG-GoGAT as defined above, said plant containing a recombinant expression cassette of the invention.

The invention further comprises a transgenic plant, preferably a transgenic wheat plant, or an isolated organ or tissue thereof comprising, stably integrated in its genome, a recombinant expression cassette comprising a polynucleotide encoding a NADH-GoGAT as defined above.

Accordingly, the invention also encompasses isolated organs or tissues of said transgenic plant (such as seeds, leafs, flowers, roots, stems, ears) containing a recombinant expression cassette of the invention.

The present invention also provides an isolated wheat NADH-dependent glutamate synthase protein having at least 95% identity with the polypeptide of sequence SEQ ID NO: 1. Preferably, said NADH-dependent glutamate synthase protein has the amino acid sequence SEQ ID NO: 22.

The present invention also provides an isolated polynucleotide chosen from the group consisting of:
a) a polynucleotide encoding a wheat NADH-GoGAT involved in Nitrogen Use Efficiency, which polypeptide has at least 95%, or by order of increasing preference at least 96%, 97%, 98% or 99% identity, with the polypeptide of sequence SEQ ID NO: 1;
b) a polynucleotide complementary to the polynucleotide a);
c) a polynucleotide capable of hybridizing selectively, under stringent conditions, with the polynucleotide a) or the polynucleotide b).

According to a preferred embodiment, the polynucleotide encoding a wheat NADH-GoGAT is selected from the group consisting of sequences SEQ ID NO: 2, 3, 4, 5, 23, 24, 25 and 26, preferably selected from the group consisting of sequences SEQ ID NO: 23, 24, 25 and 26.

Stringent hybridization conditions, for a given nucleotide, can be identified by those skilled in the art according to the size and the base composition of the polynucleotide concerned, and also according to the composition of the hybridization mixture (in particular pH and ionic strength). Generally, stringent conditions, for a polynucleotide of given size and given sequence, are obtained by carrying out procedures at a temperature approximately 5°C to 10°C below the melting temperature (Tm) of the hybrid formed, in the same reaction mixture, by this polynucleotide and the polynucleotide complementary thereto.

A "polynucleotide capable of hybridizing selectively with a polynucleotide a) or b) in accordance with the invention" is here defined as any polynucleotide which, when it is hybridized under stringent conditions with a wheat nucleic acid library (in particular a genomic DNA or cDNA library), produces a detectable hybridization signal (*i.e.* at least twice as great, preferably at least five times as great, as the background noise) with said polynucleotide, but produces no detectable signal with other sequences of said library, and in particular with sequences encoding other proteins of the GoGAT family.

A subject of the present invention is also polynucleotide probes or amplification primers obtained from polynucleotides a) or b) in accordance with the invention or fragments thereof.

The present disclosure also encompasses any polynucleotide encoding a wheat NADH-GoGAT involved in Nitrogen Use Efficiency (NUE) and which can be obtained from a plant genomic DNA or cDNA library by screening said library with probes or primers in accordance with the invention. This includes in particular other alleles of the wheat *NADH-GoGAT* gene, and in particular other alleles capable of conferring an improved NUE and/or grain filling.

By way of example, one can also use at least one of the following pairs of primers:
- TTAGTGGCAAATGGGCTTCG (SEQ ID NO: 7) and CGCCACAGCAACATCTCTACC (SEQ ID NO: 8);
- CAGCTGCAGAGATTCGTCCTG (SEQ ID NO: 9) and TGTTATCCAAAGCCATGTCAAGG (SEQ ID NO: 10);
- TGGAATGGCAGCAGAAAGGT (SEQ ID NO: 11) and TCGCATCCATGATCACCAATT (SEQ ID NO: 12);
- GCACCATTTCTGTACACTCGTTG (SEQ ID NO: 13) and ATCTTCCCATCAGTCTGCAAGC (SEQ ID NO: 14);
- TTCAAGAGCTTAACAAGGCGTG (SEQ ID NO: 15) and CACTTGCAGGTTCAACCTCATC (SEQ ID NO: 16);
- TGGGAAATGATGCACCCCTA (SEQ ID NO: 17) and CTTGTGCAAACATCTGCTTGAAG (SEQ ID NO: 18);
- AATTCTGGAAGGAAGGGCTTG (SEQ ID NO: 19) and TTTGTATCCCTCGCGTATAGCTT (SEQ ID NO: 20);
- CGAGCTTGAGGATTTGAGTTCTA (SEQ ID NO: 27) and CACTTGCTAAACTGGTATAATG (SEQ ID NO: 28), useful to amplify a fragment of a wheat *NADH-GoGAT* gene on chromosome 3A;
- TCGCTGAGTCTCTAGGACA (SEQ ID NO: 29) and GTTCAATGGCTGGTTCAGTA (SEQ ID NO: 30), useful to amplify a fragment of a wheat *NADH-GoGAT* gene on chromosome 3B;
- GGATTTGAATTCTGCAGAGAGAAA (SEQ ID NO: 31) and CACTTGCTAAACTGGTACAAGT (SEQ ID NO: 32), useful to amplify a fragment of a wheat *NADH-GoGAT* gene on chromosome 3B;
- CTACAGAGAGAAGACAGGC (SEQ ID NO: 33) and GTACAATTGATCCTGCACATATACT (SEQ ID NO: 34), useful to amplify a fragment of a wheat *NADH-GoGAT* gene on chromosome 3D;
preferably at least one of the following pairs of primers selected from the group consisting of SEQ ID NO: 27 and 28, SEQ ID NO: 29 and 30, SEQ ID NO: 31 and 32, and SEQ ID NO: 33 and 34.

The disclosure also provides means for identifying and selecting wheat plants which have an improved grain filling compared to a reference wheat plant.

The disclosure thus provides a method for identifying an allele of a wheat *NADH-GoGAT* gene associated with a given phenotype of grain filling, wherein said method comprises isolating a nucleic acid fragment comprising said *NADH-GoGAT* gene or a portion thereof from at least one wheat plant expressing said phenotype, and sequencing said fragment.

The disclosure further provides a method for identifying polymorphisms associated with grain filling, in a *NADH-GoGAT* gene, wherein said method comprises identifying, as described above, at least two different alleles of said *NADH-GoGAT* gene associated with different phenotypes of grain filling, and comparing the sequences of said alleles.

Based on the *NADH-GoGAT* allele sequences characterised in wheat genotypes, the Inventors have identified 6 DNA sequence variations (5 Single Nucleotide Polymorphisms (SNPs) and 1 Insertion/Deletion (InDel)), represented by the sequences SEQ ID NO: 35, 36, 37, 38, 39 and 40, that can be used in Marker Assisted Selection (MAS) breeding programs for improving the grain filling of a wheat plant (NUE improvement for instance).

The Inventors have also identified, in *Chinese Spring, Arche* and *Récital* genotypes, 23 other DNA sequence variations (18 Single Nucleotide Polymorphisms (SNPs) and 5 Insertion/Deletion (InDels)) shown in Table 1 below, that can be used in Marker Assisted Selection (MAS) breeding programs for improving the grain filling of a wheat plant (NUE improvement for instance).

**Table 1: Detailed information regarding 18 SNP and 5 InDels identified between Chinese Spring, Récital and Arche genotypes. SNP and InDels coordinates are based on the Chinese Spring (CS) allele (SEQ ID NO: 2).**

| Base Coordinate (CS allele) | *Chinese Spring* | *Arche* | *Récital* |
|---|---|---|---|
| #2545 | A | A | G |
| #2663 | A | G | A |
| #2737 | G | G | A |
| #2871 | T | T | C |
| #2892 | G | G | T |
| #3010 | G | G | A |
| #3039 | A | A | G |
| #3512 | G | A | G |
| #4752 | G | G | C |
| #5426 | C | C | T |
| #5452 | x | x | TA |
| #5509 | G | G | A |
| #5681 | G | A | A |
| #6420 | x | G | G |
| #6916 | G | x | G |
| #8253 | A | G | G |
| #8882 | G | x | A |
| #8943 | A | G | G |
| #9404 | A | A | x |
| #9489 | T | A | A |
| #9541 | A | G | G |
| #9566 | T | G | G |
| #9592 | G | x | x |

| | | | |
|---|---|---|---|
| A, C, G and T represent the 4 nucleotide bases, repectively adenine, cytosine, guanine and thymine. "x" represents a deletion. | | | |

Once a polymorphism has been identified, reagents and kits allowing the routine detection of said polymorphism can be designed. Commonly used reagents are nucleic acid probes, or restriction enzymes, or PCR primers, or combinations thereof. The choice of a reagent or of a combination of reagents depends of the nature of the polymorphism.

Preferred kits and reagents are those comprising a set of primers allowing specific PCR amplification of a DNA segment spanning the polymorphic locus. For microsatellites and insertion/deletion polymorphisms, PCR primers may be sufficient, since the allelic forms of the polymorphism may be differentiated by the size of the amplification product. In the case of single nucleotide polymorphisms (SNP), one will generally also use a restriction enzyme, which allows the differentiation of allelic forms by the presence or size of restriction fragments.

For these purposes, it is possible to use a nucleic acid encoding a NADH-GoGAT as defined above, or a fragment thereof, as a probe or a target for amplification, for selecting wheat plants naturally overexpressing a NADH-GoGAT as defined above, and therefore exhibiting an improved grain filling. Preferably, the amplified fragment has a length of about 500 pb, more preferably, of about 500 to 1000 pb.

The disclosure also provides a method for identifying in a wheat plant (a) genetic marker(s) associated with an improved grain filling, said method comprising genotyping said wheat plant and identifying one or more of the following alleles encoding an NADH-GoGAT as defined above:
- an allele comprising the sequence SEQ ID NO: 35 wherein the nucleotide at position 109 of said sequence is guanine (corresponding to the favourable allele for improving the grain filing in cultivar *Arche*);
- an allele comprising the sequence SEQ ID NO: 36, wherein a nucleotide adenine is present at position 112 of said sequence (corresponding to the favourable allele for improving the grain filing in cultivar *Arche*);
- an allele comprising the sequence SEQ ID NO: 37 wherein the nucleotide at position 133 of said sequence is adenine (corresponding to the favourable allele for improving the grain filing in cultivar *Arche*);
- an allele comprising the sequence SEQ ID NO: 38 wherein the nucleotide at position 61 of said sequence is guanine (corresponding to the favourable allele for improving the grain filing in cultivar *Arche*);
- an allele comprising the sequence SEQ ID NO: 39 wherein the nucleotide at position 439 of said sequence is guanine (corresponding to the favourable allele for improving the grain filing in cultivar *Arche*);
- an allele comprising the sequence SEQ ID NO: 40 wherein the nucleotide at position 106 of said sequence is thymine.

Many techniques are known by the person skilled in art to identify a specific allele. By way of example, said allele can be identified by sequencing or by hybridization with a nucleotide sequence complementary to the sequences SEQ ID NO: 35-40 respectively. Said allele can be amplified using a pair of primers according to the present invention as defined above.

The disclosure further provides a method for selecting a wheat plant having an improved grain filling, wherein said method comprises identifying in wheat plants to be tested (a) genetic marker(s) associated with an improved grain filling by the method defined above, and selecting a plant containing said genetic marker(s).

The Inventors also disclose a method for inhibiting in a plant, preferably a wheat or maize plant, a NADH-dependent glutamate synthase (NADH-GoGAT) having at least 95% identity, or by order of increasing preference at least 96%, 97%, 98% or 99% identity, with the polypeptide of sequence SEQ ID NO: 1 or SEQ ID NO: 22 as defined above.

The inhibition of a NADH-GoGAT protein can be obtained either by abolishing, blocking or decreasing its function (*i.e.* catalyzing the reductive transfer of amide group of glutamine to 2-oxoglutarate to form two glutamate molecule), or advantageously, by preventing or down-regulating the expression of its gene.

By way of example, inhibition of said NADH-GoGAT protein can be obtained by mutagenesis of the corresponding gene or of its promoter, and selection of the mutants having partially or totally lost the NADH-GoGAT protein activity. For instance, a mutation within the coding sequence can induce, depending on the nature of the mutation, the expression of an inactive protein, or of a protein with impaired activity; in the same way, a mutation within the promoter sequence can induce a lack of expression of said NADH-GoGAT protein, or decrease thereof.

Mutagenesis can be performed for instance by targeted deletion of the *NADH-GoGAT* coding sequence or promoter, or of a portion thereof, or by targeted insertion of an exogenous sequence within said coding sequence or said promoter. It can also be performed by random chemical or physical mutagenesis, followed by screening of the mutants within the *NADH-GoGAT* gene. Methods for high throughput mutagenesis and screening are available in the art. By way of example, one can mention TILLING (Targeting Induced Local Lesions IN Genomes, described by McCallum *et al*., 2000).

Advantageously, the inhibition of said NADH-GoGAT protein is obtained by silencing of the corresponding gene. Methods for gene silencing in plants are known in themselves in the art. For instance, one can mention by antisense inhibition or co-suppression, as described by way of example in U.S. Patents 5,190,065 and 5,283,323. It is also possible to use ribozymes targeting the mRNA of said NADH-GoGAT protein.

Preferred methods are those wherein post transcriptional gene silencing is induced by means of RNA interference (RNAi) targeting the *NADH-GoGAT* gene to be silenced. Various methods and DNA constructs for delivery of RNAi are available in the art (for review, Watson *et al*., 2005). Typically, DNA constructs for delivering RNAi in a plant include at least a fragment of 300 bp or more (generally 300-800 bp, although shorter sequences may sometime induce efficient silencing) of the cDNA of the target gene, under transcriptional control of a promoter active in said plant. Currently, the more widely used DNA constructs are those that encode hairpin RNA (hpRNA). In these constructs, the fragment of the target gene is inversely repeated, with generally a spacer region between the repeats.

The Inventors further disclose chimeric DNA constructs for silencing a *NADH*-*GoGAT* gene.

Such a chimeric DNA construct comprises:
- a promoter functional in a plant cell;
- a DNA sequence of 200 to 1000 bp, preferably of 300 to 900 bp, consisting of a fragment of a cDNA encoding a NADH-GoGAT protein or of its complementary, or having at least 95% identity, and by order of increasing preference, at least 96%, 97%, 98% or 99 % identity with said fragment, said DNA sequence being placed under transcriptional control of said promoter.

Said chimeric DNA construct comprises:
- a first DNA sequence of 200 to 1000 bp, preferably of 300 to 900 bp, consisting of a fragment of a cDNA encoding a NADH-GoGAT protein, or having at least 95% identity, and by order of increasing preference, at least 96%, 97%, 98% or 99 % identity with said fragment;
- a second DNA sequence that is the complementary of said first DNA, said first and second sequences being in opposite orientations ;
- a spacer sequence separating said first and second sequence, such that these first and second DNA sequences are capable, when transcribed, of forming a single double-stranded RNA molecule.

The spacer can be a random fragment of DNA. However, preferably, one will use an intron which is spliceable by the target plant cell. Its size is generally 400 to 2000 nucleotides in length.

A large choice of promoters suitable for expression of heterologous genes in plants is available in the art. They can be chosen among those disclosed above.

DNA constructs for silencing a *NADH-GoGAT* gene as defined above generally also include a transcriptional terminator (for instance the 35S transcriptional terminator, or the nopaline synthase (Nos) transcriptional terminator).

These DNA constructs for silencing a *NADH-GoGAT* gene as defined above can be obtained and introduced in a host cell or organism by the well-known techniques of recombinant DNA and genetic engineering, such as those described above.

The Inventors futher disclose plant cells (preferably wheat or maize plant cells) or plants (preferably wheat or maize plants) genetically modified by a DNA construct for silencing a *NADH-GoGAT* gene as defined above. The polynucleotide may be transiently expressed; it can also be incorporated in a stable extrachromosomal replicon, or integrated in the chromosome.

In particular the Inventors disclose a transgenic plant, preferably a transgenic wheat or maize plant, containing a transgene comprising a DNA construct for silencing a *NADH-GoGAT* gene as defined above.

Foregoing and other objects and advantages of the invention will become more apparent from the following detailed description and accompanying drawing. It is to be understood however that this foregoing detailed description is exemplary only and is not restrictive of the invention.
Figure 1 represents the linear regression observed between the *GoGAT* gene expression (expressed as ΔΔCT) and the NNI status of the *Arche* (square) and *Soissons* (round) wheat genotypes for 29 leaf samples collected after flowering (respectively at Z75 and Z65).
Figure 2 shows the cloning strategy for pSC4Act-synGOGAT TaMod-SCV.
Figure 3 shows the cloning strategy for pAct-TaGOGAT-RNAi-66-SCV.
Figure 4 shows the difference in NADH-GoGAT expression between *Arche* and *Recital* wheat genotypes under different N supply levels.

### EXAMPLE 1: EXPERIMENTAL VALIDATION OF THE NADH-GoGAT GENE IN NITROGEN USE EFFICIENCY (NUE) IN WHEAT

### 1) Materials & Methods

Wheat leaf samples were collected on 2 trials (La Minière and Boigneville stations - Arvalis Institut du Vegetal; France): one in field for cultivar *Arche* and the other in green house for cultivar *Soissons.* Different nitrogen treatments were applied to lead to samples with a range of Nitrogen Nutrional Index (NNI) from 0.49 to 1.34 after flowering. During wheat culture, sampling has been done at 2 stages corresponding to the Zadoks scale: Z65 (Soissons) and Z75 (Arche).

Total RNAs were extracted from all the samples with the SV96 Total RNA Isolation System (Promega) according to the manufacturer instructions. RNA integrity was verified on the Agilent Bioanalyzer and presence of potential genomic DNA was checked by *q*PCR on RNA. In the absence of genomic DNA no amplification is expected from RNA.

For each sample 2 µg of total RNA were submitted to the reverse transcription using the High capacity reverse transcription kit (Applied Biosystems) and random primers in 100µl. RT reaction was then 1/10^{th} diluted and 2µl of cDNA used for the amplification. Each RNA sample was submitted to 2 independent RT reactions for technical reproducibility evaluation.

Quantitative PCR was performed on an ABI7900 machine (Applied Biosystems), using Applied Biosystems reagents. The PCR reactions consisted of a hot-start Taq Polymerase activation step of 95°C for 5 minutes, followed by 2 steps amplification cycles (denaturation 95°C, 30sec, annealing/elongation 60°C, 1min). Expression levels of mRNA for *NADH-GoGAT* gene were calculated using the Ct estimated by the SDS software (Applied Biosystems) and normalized across samples using 4 control genes. Normalized and Relative expression was then considered as the ΔC and ΔΔCT respectively, between *NADH-GoGAT* gene and the average of controls.

### 2) Results

In order to validate the role of the *NADH-GoGAT* gene in NUE, an experiment on two bread wheat genotypes, *i.e. Arche* and *Soissons,* was conducted. Twenty nine leaf samples for *Arche* and nine for *Soissons* were collected after flowering (respectively at Z75 and Z65). The N nutrition index (NNI) value was calculated (ranking from 0.49 to 1.34) for each sample. Moreover, for the same samples, RNA was extracted and the expression pattern of *GoGAT* was analysed through *q*PCR (ranking from 0 to 14 ΔΔCT) using sequence primers based on the 3B contig sequence (forward: AATTCTGGAAGGAAGGGCTTG; SEQ ID NO: 19; reverse: TTTGTATCCCTCGCGTATAGCTT; SEQ ID NO: 20). The results are shown in Table 2 here-after.

**Table 2: GoGAT gene expression analysed through qPCR (expressed as ΔCT and ΔΔCT) and Nitrogen Nutrition Index (NNI value) for 29 leaf samples on Arche and Soissons genotypes. The ΔΔCT value of the Z75N1F2 and Z65_F1_T1 samples was set to 1.**

| ***Arche*** | | | |
|---|---|---|---|
| Sample name | NNI value | ΔCT value | ΔΔCT value |
| Z75N1F1 | 0.49 | 8.99 | 1.30 |
| Z75N1F2 | 0.49 | 9.37 | 1.00 |
| Z75N2F1 | 0.66 | 7.91 | 2.75 |
| Z75N2F2 | 0.66 | 8.32 | 2.07 |
| Z75N2F3 | 0.66 | 7.52 | 3.60 |
| Z75N3F1 | 0.67 | 7.82 | 2.94 |
| Z75N3F2 | 0.67 | 8.14 | 2.35 |
| Z75N4F1 | 0.83 | 7.93 | 2.71 |
| Z75N4F2 | 0.83 | 7.51 | 3.63 |
| Z75N4F3 | 0.83 | 6.92 | 5.46 |
| Z75N5F1 | 0.74 | 7.62 | 3.36 |
| Z75N5F2 | 0.74 | 6.90 | 5.56 |
| Z75N5F3 | 0.74 | 7.10 | 4.82 |
| Z75N6F1 | 0.96 | 7.11 | 4.81 |
| Z75N6F2 | 0.96 | 6.71 | 6.31 |
| Z75N6F3 | 0.96 | 7.80 | 2.96 |
| Z75N7F1 | 1.12 | 6.73 | 6.26 |
| Z75N7F2 | 1.12 | 6,11 | 9.55 |
| Z75N8F1 | 1.25 | 6.99 | 5.23 |
| Z75N8F2 | 1.25 | 5.68 | 12.93 |

| ***Soissons*** | | | |
|---|---|---|---|
| Sample name | NNI score | ΔCT value | ΔΔCT value |
| Z65_F1_T1 | 0.62 | 8.1 | 1.00 |
| Z65_F1_T2 | 0.99 | 7.5 | 1.54 |
| Z65_F1_T3 | 1.34 | 6.7 | 2.63 |
| Z65_F2_T1 | 0.62 | 6.7 | 2.59 |
| Z65 F2 T2 | 0.99 | 6.6 | 2.80 |
| Z65_F2_T3 | 1.34 | 6.4 | 3.34 |
| Z65_F3_T1 | 0.62 | 7.6 | 1.38 |
| Z65_F3_T2 | 0.99 | 6.7 | 2.68 |
| Z65_F3_T3 | 1.34 | 6.9 | 2.25 |

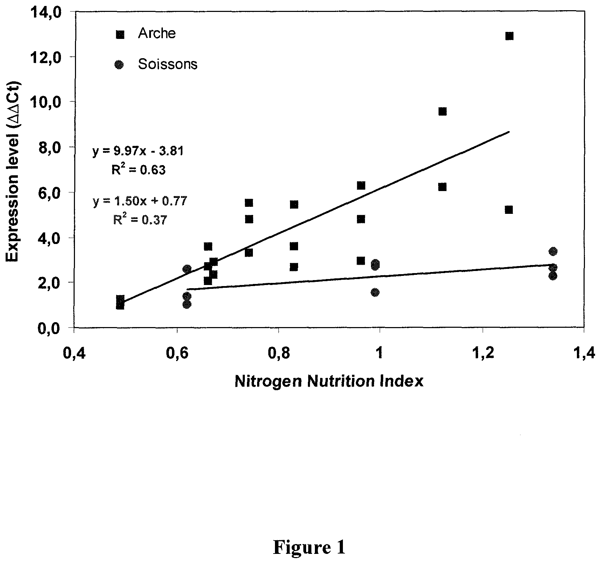

A significant correlation of R²=63% and 37% was found between the expression (ΔΔCT values) of the *NADH-GoGAT* gene and the NNI score of the 29 leaves samples for both the *Arche* and *Soissons* genotypes, respectively. These results confirm that the *NADH-GoGAT* gene is the major candidate gene driving NUE on chromosome 3B (Figure 1).

### EXAMPLE 2: CONSTRUCTION OF TRANSGENIC WHEAT PLANTS OVEREXPRESSING A WHEAT NADH-GoGAT

### 1) Wheat transformation constructs for NADH-GoGAT over-expression

The *Nco*I-*Xba*I synthetic fragment of the wheat *NADH-GOGAT* is cloned in the pUC57 vector (GenBank accession number: Y14837 (GI:2440162)), leading to the pUC57_synGOGAT TaMod vector. The *Nco*I*-Xba*I GOGAT fragment from pUC57_synGOGAT TaMod is then introduced in the pENTR4 vector (Invitrogen) linearised with *Nco*I-*Xba*I, to create the pENTR4_synGOGAT TaMod.

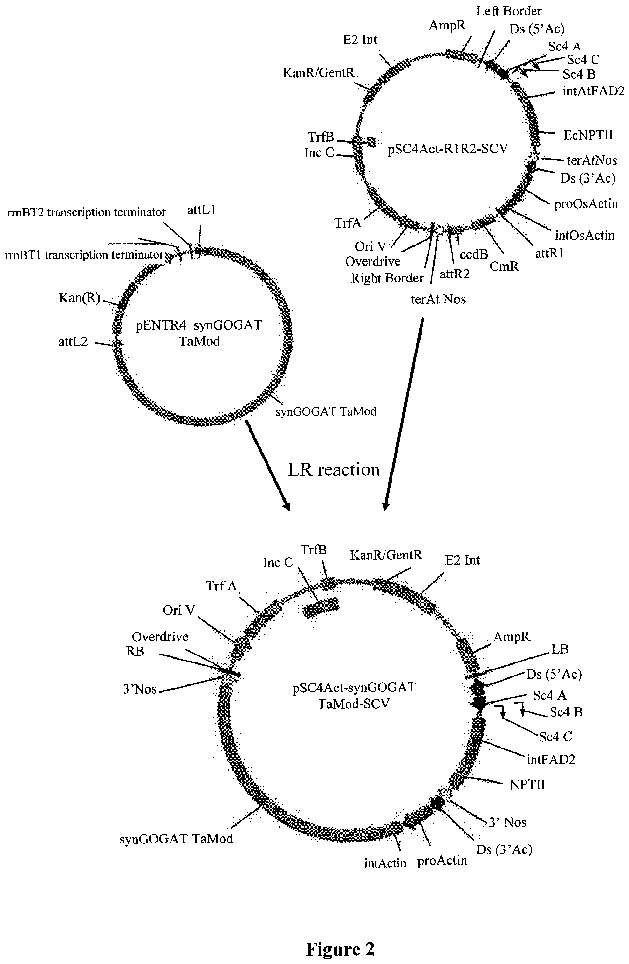

An LR clonase reaction between the pENTR4_synGOGAT TaMod and the pSC4Act-R1R2-SCV, allows the creation of pSC4Act-synGOGAT TaMod-SCV (Figure 2). pSC4Act-R1R2-SCV is a vector using the Gateway approach to introduce genes to be expressed under the control of the rice *Actin* gene promoter (McElroy *et al*., 1990). pSC4Act-R1R2-SCV is obtained after introduction of the proActin-R1R2-terNOS cassette into the binary vector pSCV1 (Firek *et al*., 1993). The binary plasmid pSC4Act-synGOGAT TaMod-SCV, is then introduced in the *A. tumefaciens* hypervirulent strain EHA105, and used for transformation experiments.

### 2) Wheat transformation protocol

The method is essentially similar to the one described in International Application WO 00/63398. Wheat tillers, approximately 14 days post-anthesis (embryos approximately 1mm in length), are harvested from glasshouse grown plants to include 50cm tiller stem (22/15°C day/night temperature, with supplemented light to give a 16 hour day). All leaves are then removed except the flag leaf and the flag leaf is cleaned to remove contaminating fungal spores. The glumes of each spikelet and the lemma from the first two florets are then carefully removed to expose the immature seeds. Only these two seeds in each spikelet are generally uncovered. This procedure is carried out along the entire length of the inflorescence. The ears are then sprayed with 70% IMS as a brief surface sterilization.

*Agrobacterium tumefaciens* strains containing the vector for transformation are grown on solidified YEP media with 20mg/l kanamycin sulphate at 27°C for 2 days. Bacteria are then collected and re-suspended in TSIM1 (MS media with 100mg/l myoinositol, 10g/l glucose, 50mg/l MES buffer pH5.5) containing 400µM acetosyringone to an optical density of 2.4 at 650nm.

Agrobacterium suspension (1µl) is inoculated into the immature seed approximately at the position of the scutellum: endosperm interface, using a 10µl Hamilton, so that all exposed seed are inoculated. Tillers are then placed in water, covered with a translucent plastic bag to prevent seed dehydration, and placed in a lit incubator for 3 days at 23°C, 16hr day, 45µEm-2s-1 PAR.

After 3 days of co-cultivation, inoculated immature seeds are removed and surface sterilized (30 seconds in 70% ethanol, then 20 minutes in 20% Domestos, followed by thorough washing in sterile distilled water). Immature embryos are aseptically isolated and placed on W4 medium (MS with 20g/l sucrose, 2mg/l 2,4-D, 500mg/l Glutamine, 100mg/l Casein hydrolysate, 150mg/l Timentin, pH5.8, solidified with 6g/l agarose) and with the scutellum uppermost. Cultures are placed at 25°C in the light (16 hour day). After 12 days cultivation on W4, embryogenic calli are transferred to W425G media (W4 with 25mg/l Geneticin (G418)). Calli are maintained on this media for 2 weeks and then each callus is divided into 2mm pieces and re-plated onto W425G.

After a further 2 week culture, all tissues are assessed for development of embryogenic callus: any callus showing signs of continued development after 4 weeks on selection is transferred to regeneration media MRM 2K 25G (MS with 20g/l sucrose, 2mg/l Kinetin, 25mg/l Geneticin (G418), pH5.8, solidified with 6g/l agarose). Shoots are regenerated within 4 weeks on this media and then transferred to MS20 (MS with 20g/l sucrose, pH5.8, solidified with 7g/l agar) for shoot elongation and rooting.

The presence of the T-DNA, and the number of copies are quantified by quantitative PCR (*q*PCR).

### EXAMPLE 3: ASSOCIATION STUDIES

The aim of association studies is to identify loci contributing to quantitative traits, based on statistical association between genotypes and phenotypes using a large germplasm collection (panel) without knowledge on pedigree. At the opposite of linkage mapping, association studies can be performed using a selection of cultivars without the need for crossing and screening offspring. In this way, it can be looked at a maximum of genotypic variability (depending on panel selection) in a single study. Thus, using this technique, it is possible to identify favorable alleles of the *NADH-GoGAT* gene linked to phenotypic data, with a high resolution.

After identification of QTL's *NADH-GoGAT* gene, a SNP discovery has been carried out by sequencing this gene in several genotypes. Several SNPs have been identified and have been genotyped in a panel of 200 varieties using the SNP / InDel Genotyping Service of KBioscience (Kaspar Technology; http://www.kbioscience.co.uk). Genotyping data have been used for association studies using both General Linear Model (GLM) and Mixed linear model (MLM), and also using structure and Kinship matrix information.

One SNP (namely SNP_3927; shown in SEQ ID NO: 40) located at position 3927 in the coding sequence (intron 12) of *NADH-GoGat* gene on chromosome 3A (homeologous to *NADH-GoGat* gene on chromosome 3B) has been found statiscaly associated with yield, nitrogen uptake efficiency, grain weight and grain protein content in several field trials (2 years, 2 differents locations under several nitrogen conditions (optimal and sub-optimal).

The result of the allelic effect obtained by MLM statistical analysis on associated traits has shown that the allele comprising the sequence SEQ ID NO: 40 wherein the nucleotide at position 106 of said sequence is thymine, is the favourable allele for the yield and grain weight.

Accordingly, this association study in wheat shows the involvement of the *NADH-GoGAT* gene in NUE, yield and grain protein content in several nitrogen conditions (optimal and sub-optimal).

### EXAMPLE 4: WHEAT RNAi TRANSFORMATION CONSTRUCTS FOR NADH-GoGAT REPRESSION

A 500 bp *Xba*I-*Xmn*I synthetic fragment (represented as SEQ ID NO: 21) of wheat *NADH-GoGAT* is cloned in the pUC57 vector, leading to the pUC57_TaGOGAT vector. The *Xba*I-*Xmn*I GOGAT RNAi fragment from pUC57-TaGOGAT is then introduced in the pENTR1A vector (Invitrogen) linearised with *Xba*I-*Xmn*I, to create the pENTR1A_TaGOGAT.

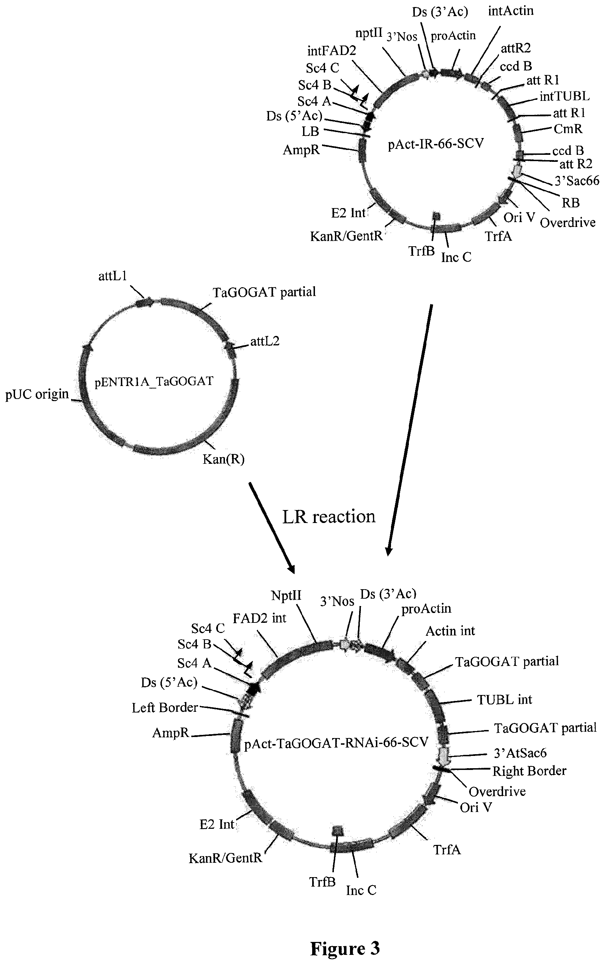

An LR clonase reaction between the pENTR1A_TaGOGAT and the pAct-IR-66-SCV, allows the creation of pAct-TaGOGAT-RNAi-66-SCV (Figure 3). pAct-IR-66-SCV is a vector used to create RNAi vectors under the control of the rice *Actin* gene promoter (McElroy *et al*., 1990). pAct-IR-66-SCV is obtained after introduction of the proActin-RNAi-terSac66 cassette from pBIOS890 into the binary vector pSCV1 (Firek *et al*., 1993). The binary plasmid pAct-TaGOGAT-RNAi-66-SCV is then introduced in the *A. tumefaciens* hypervirulent strain EHA105, and used for transformation experiments.

### EXAMPLE 5: EXPERIMENTAL VALIDATION OF THE NADH-GoGAT GENE EXPRESSION BETWEEN ARCHE AND RECITAL

### 1) Materials & Methods

*NADH-GOGAT* gene expression has been analyzed for two bread wheat lines, *i.e*., *Arche* and *Récital*, using RT-PCR analysis with the following pair of primers: forward, AATTCTGGAAGGAAGGGCTTG; SEQ ID NO: 19; reverse: TTTGTATCCCTCGCGTATAGCTT; SEQ ID NO: 20. Samples (glumes, leave blades) were collected in Clermont-Ferrand (France) in 2008 under high N supply (240 kg N ha⁻¹ in four applications) and low N supply (40 kg N ha⁻¹ in one application). The experimental design was a split-plot with N treatment as the main plot and three replicates. Biological repetitions have been polled and RNA extracted.

### 2) Results

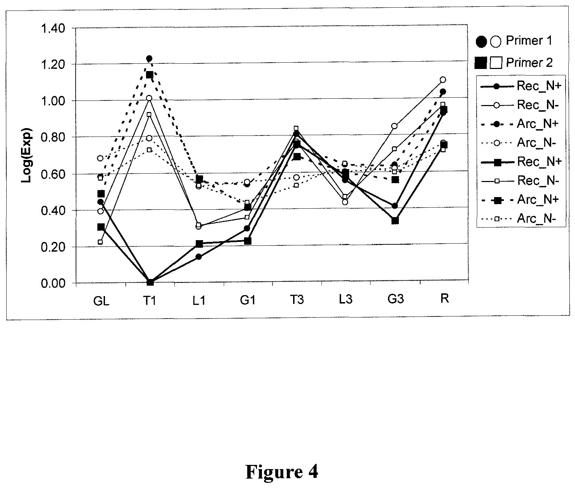

The results (see Figure 4) show a significant difference in *NADH-GoGAT* expression between the two varieties under high nitrogen levels in the stems and leaves of the stage closest to the ear. In the rest of the plant and under low nitrogen levels, the level of *NADH-GoGAT* expression is very similar. These results support the hypothesis that *NADH-GoGAT* is a gene candidate for improving the grain filling of a wheat plant, beauce (1) there is a difference in *GoGAT* expression between the two varieties, (2) said difference appears under high level of nitrogen as appears the major QTL detected in the same genomic region, (3) the nitrogen assimilation mainly occurs in the upper leaves and stem segments.

### EXAMPLE 6: CONSTRUCTION OF A TRANSGENIC MAIZE PLANT OVEREXPRESSING A WHEAT NADH-GoGAT

The maize cultivar A188 is transformed by the strain of *Agrobacterium* containing the vector-pSC4Act SynGOGAT TaMod-SCV described in Example 2 above, using the method described by Ishida *et al*., 1996 (cited above).

The genetically modified plant material (transformants) is selected as follows: the presence of the T-DNA and the number of copies of the transgene are determined by quantitative PCR (*q*PCR). In addition, the presence of the GFP reporter gene in both vectors used to obtain the transgenic plants allows sorting the transgenic seeds from the non-transgenic wild-type segregants.

The selected transformants is then regenerated into plants.

The transgenic plants are analyzed using routine methods: the number of copies of the integrated transgene and the integrity of the T-DNA. The full expression of the mRNA and the level of expression of the gene of interest are determined by quantitative PCR.

### REFERENCES

- Agrama et al., (1999) Molecular Breeding 5:187-195.
- An et al., (1986) Plant Physiology 81:86-91.
- An et al., (2006) Plant and Soil 284:73-84.
- Bechtold et al., (1993) C R Acad Sci III 316:1194-1199.
- Bernard & Habash, (2009) New Phytologist 182:608-620.
- Bertin & Gallais, (2001) Maydica 46:53-68.
- Borrell et al., (1998) Australian Journal of Agricultural Research 49:829-843.
- Cai et al., (2009) Plant Cell 28:527-537.
- Caputo et al., (2009) Plant Physiol. Biochem. 47:335-342.
- Chupeau et al., (1989) Biotechnology 7:503-508.
- Depicker et al., (1982J) Mol. Appl. Genet. 1:561-73.
- Finer et al., (1992) Plant Cell Report 11:323-328.
- Firek et al., (1993) Plant Mol Biol. 22:129-142.
- Fontaine et al., (2009) Theoretical and Applied Genetics 119:645-662.
- Fromm et al., (1990) Nature 319:791-793.
- Guerche et al., (1987) Biochimie 69:621-628.
- Goldsbrough et al., (1993) Biotechnology 11:1286-1292.
- Habash et al., (2007) Theoretical and Applied Genetics 114:403-419.
- Hayakawa et al., (1994) Planta 193:455-460.
- Hirel et al., (2001) Plant Physiology 125:1258-1270.
- Ireland & Lea eds, (1999) The enzymes of glutamine, glutamate, asparagine and aspartate metabolism (Marcel Dekker, New York), pp 49-109.
- Ishida et al., (1996) Nature Biotechnology 14:745-750.
- Ishiyama et al., (2004) Journal of Biological Chemistry 279:16598-16605.
- Jeanneau et al., (2002) Biochimie 84:1127-1135.
- Kasuga et al., (1999) Nature Biotech. 17:287-291.
- Katayama et al., (2000) Plant Mol. Biol. 44:99-106.
- Kjaer et al., (1995) Genome 38:1098-1104.
- Krapp et al., (2005) Photosynthesis Research 83:251-263.
- Laperche et al., (2006) Theoretical and Applied Genetics 112:797-807.
- Laperche et al., (2007) Theoretical and Applied Genetics 115:399-415.
- Le Gouis et al., (2000) European Journal of Agronomy 12:163-173.
- Lea & Miin, (2003) Plant Physiology and Biochemistry 41:555-564.
- Lian et al., (2005) Theoretical and Applied Genetics 112:85-96.
- Lima et al., (2006) Journal of Experimental Botany 57:2751-2761.
- Loudet et al., (2003) Plant Physiology 131:1508-1508.
- McCallum et al., (2000) Plant Physiology 123:439-442.
- McElroy et al., (1990) Plant Cell 2:163-171.
- McElroy et al., (1991) Mol. Gen. Genet. 231:150-160.
- Miin & Habash, (2002) Journal of experimental botany 53:979-987.
- Narusaka et al., (2003) Plant J. 34:137-148.
- Noh and Amasino, (1999) Plant Mol. Biol. 41:181-194.
- Obara, et al., (2001) Journal of Experimental Botany 52:1209-1217.
- Ochs et al., (1999) Plant Molecular Biology 39:395-405.
- Oury et al., (2003) Journal of Genetics & Breeding 57:59-68.
- Potel et al., (2009) Febs Journal 276:4061-4076.
- Rauh et al., (2002) Theoretical and Applied Genetics 104:743-750.
- Simmonds, (1995) Journal of the Science of Food and Agriculture 67:309-315.
- Tabuchi et al., (2007) Journal of Experimental Botany 58:2319-2327.
- Thomas & Flavell, (1990) Plant Cell 2:1171-80.
- Valadier et al., (2008) Febs Journal 275:3193-3206.
- Verdaguer et al., (1996) Plant Mol. Biol. 6:1129-39.
- Watson et al., (2005) FEBS Letters 579: 5982-5987.
- Yamaya et al., (1992) Plant Physiology 100:1427-1432.
- Yamaya et al., (2002) Journal of Experimental Botany 53:917-925.

## Claims

1. A method for improving the grain filling of a wheat plant, wherein said method comprises overexpressing in said wheat plant a NADH-dependent glutamate synthase (NADH-GoGAT) having at least 95% identity with the polypeptide of sequence SEQ ID NO: 1, wherein the percent of identity between two sequences is calculated from an alignment of the two sequences over their whole length.

2. The method of claim 1, wherein the grain filling is improved by increasing the grain weight and/or the grain protein content.

3. The method of claim 1 or claim 2, wherein said NADH-GoGAT has the amino acid sequence SEQ ID NO: 22.

4. The method of anyone of claims 1 to 3, wherein said NADH-GoGAT is encoded by a nucleotide sequence selected from the group consisting of SEQ ID NO: 2, SEQ ID NO: 3, SEQ ID NO: 4, SEQ ID NO: 5, SEQ ID NO: 23, SEQ ID NO: 24, SEQ ID NO: 25 and SEQ ID NO: 26, preferably SEQ ID NO: 23, SEQ ID NO: 24, SEQ ID NO: 25 and SEQ ID NO: 26.

5. A recombinant expression cassette, wherein it comprises a polynucleotide encoding a NADH-GoGAT as defined in any of claims 1, 3 or 4, under control of a heterologous promoter functional in a plant cell.

6. A recombinant vector, wherein it contains an expression cassette comprising a polynucleotide encoding a NADH-GoGAT as defined in any of claims 1, 3 or 4, under control of a promoter.

7. A host cell, wherein it contains a recombinant expression cassette of claim 5 or a recombinant vector of claim 6.

8. A host cell of claim 7 which is a wheat plant cell.

9. A method for producing a transgenic wheat plant, having an improved grain filling, wherein said method comprises:
- providing a wheat plant cell of claim 8;
- regenerating from said wheat plant cell a transgenic wheat plant overexpressing a NADH-GoGAT as defined in any of claims 1 or 3.

10. A transgenic wheat plant obtainable by the method of claim 9, said transgenic wheat plant containing a recombinant expression cassette as defined in claim 5.

11. A transgenic wheat plant or an isolated organ or tissue thereof, wherein it comprises, stably integrated in its genome, a recombinant expression cassette comprising a polynucleotide encoding a NADH-GoGAT as defined in any of claims 1, 3 or 4.

12. Wheat seeds, wherein it contains a recombinant expression cassette of claim 5 obtained from a transgenic wheat plant of any of claims 10 or 11.

13. An isolated wheat NADH-dependent glutamate synthase protein having at least 95% identity with the polypeptide of sequence SEQ ID NO: 1, wherein the percent of identity between two sequences is calculated from an alignment of the two sequences over their whole length.

14. An isolated wheat NADH-dependent glutamate synthase protein of claim 13, wherein it has the amino acid sequence SEQ ID NO: 22.

15. An isolated polynucleotide chosen from the group consisting of:
a) a polynucleotide encoding a wheat NADH-GoGAT, which polypeptide has at least 95% identity with the polypeptide of sequence SEQ ID NO: 1, wherein the percent of identity between two sequences is calculated from an alignment of the two sequences over their whole length;
b) a polynucleotide complementary to the polynucleotide a).

16. An isolated polynucleotide according to claim 15, wherein it is selected from the group consisting of SEQ ID NO: 2, SEQ ID NO: 3, SEQ ID NO: 4, SEQ ID NO: 5, SEQ ID NO: 23, SEQ ID NO: 24, SEQ ID NO: 25 and SEQ ID NO: 26, preferably SEQ ID NO: 23, SEQ ID NO: 24, SEQ ID NO: 25 and SEQ ID NO: 26.

17. A pair of primers, wherein it is selected from the group consisting of the sequences SEQ ID NO: 7 and SEQ ID NO: 8, SEQ ID NO: 9 and SEQ ID NO: 10, SEQ ID NO: 11 and SEQ ID NO: 12, SEQ ID NO: 13 and SEQ ID NO: 14, SEQ ID NO: 15 and SEQ ID NO: 16, SEQ ID NO: 17 and SEQ ID NO: 18, and SEQ ID NO: 19 and SEQ ID NO: 20, SEQ ID NO: 27 and SEQ ID NO: 28, SEQ ID NO: 29 and SEQ ID NO: 30, SEQ ID NO: 31 and SEQ ID NO: 32, SEQ ID NO: 33 and SEQ ID NO: 34, preferably SEQ ID NO: 27 and SEQ ID NO: 28, SEQ ID NO: 29 and SEQ ID NO: 30, SEQ ID NO: 31 and SEQ ID NO: 32, SEQ ID NO: 33 and SEQ ID NO: 34.

## Patentansprüche

1. Verfahren zum Verbessern der Kornfüllung einer Weizenpflanze, wobei das Verfahren in der Weizenpflanze das Überexprimieren einer NADH-abhängigen Glutamatsynthase (NADH-GoGAT) umfasst, die mindestens zu 95 % identisch mit dem Polypeptid von SEQ ID NO: 1 ist, wobei der Prozentsatz der Identität zwischen zwei Sequenzen aus einer Ausrichtung der zwei Sequenzen über die gesamte Länge berechnet wird.

2. Verfahren nach Anspruch 1, wobei die Kornfüllung durch Erhöhen des Korngewichts und/oder des Kornproteingehalts verbessert wird.

3. Verfahren nach Anspruch 1 oder Anspruch 2, wobei die NADH-GoGAT die Aminosäuresequenz SEQ ID NO: 22 hat.

4. Verfahren nach einem der Ansprüche 1 bis 3, wobei die NADH-GoGAT durch eine Nucleotidsequenz codiert wird, die aus der Gruppe ausgewählt wird, die aus SEQ ID NO: 2, SEQ ID NO: 3, SEQ ID NO: 4, SEQ ID NO: 5, SEQ ID NO: 23, SEQ ID NO: 24, SEQ ID NO: 25 und SEQ ID NO: 26, vorzugsweise SEQ ID NO: 23, SEQ ID NO: 24, SEQ ID NO: 25 und SEQ ID NO: 26 besteht.

5. Rekombinante Expressionskassette, wobei sie ein Polynucleotid umfasst, das eine NADH-GoGAT nach einem der Ansprüche 1, 3 oder 4 codiert, unter der Kontrolle eines heterologen Promoters, der in einer Pflanzenzelle funktionsfähig ist.

6. Rekombinanter Vektor, wobei er eine Expressionskassette enthält, die ein Polynucleotid umfasst, das eine NADH-GoGAT codiert, nach einem der Ansprüche 1, 3 oder 4, unter Kontrolle eines Promoters.

7. Wirtszelle, wobei sie eine rekombinante Expressionskassette nach Anspruch 5 oder einen rekombinanten Vektor nach Anspruch 6 enthält.

8. Wirtszelle nach Anspruch 7, die eine Weizenpflanzenzelle ist.

9. Verfahren zum Herstellen einer transgenen Weizenpflanze, die eine verbesserte Kornfüllung hat, wobei das Verfahren umfasst:
- Bereitstellen einer Weizenpflanzenzelle nach Anspruch 8;
- aus der Weizenpflanzenzelle, neu Generieren einer transgenen Weizenpflanze, die eine NADH-GoGAT nach einem der Ansprüche 1 oder 3 überexprimiert.

10. Transgene Weizenpflanze, die durch das Verfahren nach Anspruch 9 erzielbar ist, wobei die transgene Weizenpflanze eine rekombinante Expressionskassette nach Anspruch 5 enthält.

11. Transgene Weizenpflanze oder ein isoliertes Organ oder Gewebe derselben, wobei sie, stabil in ihr Genom integriert, eine rekombinante Expressionskassette umfasst, die ein Polynucleotid enthält, das eine NADH-GoGAT codiert, wie in einem der Ansprüche 1, 3 oder 4 definiert.

12. Weizensamen, wobei sie eine rekombinante Expressionskassette nach Anspruch 5 enthalten, die aus einer transgenen Weizenpflanze nach einem der Ansprüche 10 oder 11 erhalten wurde.

13. Isoliertes Weizen-NADH-abhängiges Glutamatsynthaseprotein, das mindestens 95 % Identität mit dem Polypeptid von SEQ ID NO: 1 hat, wobei der Prozentsatz der Identität zwischen zwei Sequenzen aus einer Ausrichtung der zwei Sequenzen über die gesamte Länge berechnet wird.

14. Isoliertes Weizen-NADH-abhängiges Glutamatsynthaseprotein nach Anspruch 13, wobei es die Aminosäuresequenz SEQ ID NO: 22 hat.

15. Isoliertes Polynucleotid, das aus der Gruppe gewählt wird, die besteht aus:
a) einem Polynucleotid, das eine Weizen-NADH-GoGAT codiert, wobei dieses Polypeptid mindestens 95 % Identität mit dem Polypeptid von SEQ ID NO: 1 hat, wobei der Prozentsatz der Identität zwischen zwei Sequenzen aus einer Ausrichtung der zwei Sequenzen über die gesamte Länge berechnet wird;
b) einem Polynucleotid, das komplementär zum Polynucleotid a) ist.

16. Isoliertes Polynucleotid nach Anspruch 15, wobei es aus der Gruppe ausgewählt wird, die aus SEQ ID NO: 2, SEQ ID NO: 3, SEQ ID NO: 4, SEQ ID NO: 5, SEQ ID NO: 23 SEQ ID NO: 24, SEQ ID NO: 25 und SEQ ID NO: 26, vorzugsweise SEQ ID NO: 23, SEQ ID NO: 24, SEQ ID NO: 25 und SEQ ID NO: 26 besteht.

17. Paar von Primern, wobei es aus der Gruppe ausgewählt wird, die aus den Sequenzen SEQ ID NO: 7 und SEQ ID NO: 8, SEQ ID NO: 9 und SEQ ID NO: 10, SEQ ID NO: 11 und SEQ ID NO: 12, SEQ ID NO: 13 und SEQ ID NO: 14, SEQ ID NO: 15 und SEQ ID NO: 16, SEQ ID NO: 17 und SEQ ID NO: 18, und SEQ ID NO: 19 und SEQ ID NO: 20, SEQ ID NO: 27 und SEQ ID NO: 28, SEQ ID NO: 29 und SEQ ID NO: 30, SEQ ID NO: 31 und SEQ ID NO: 32, SEQ ID NO: 33 und SEQ ID NO: 34, vorzugsweise SEQ ID NO: 27 und SEQ ID NO: 28, SEQ ID NO: 29 und SEQ ID NO: 30, SEQ ID NO: 31 und SEQ ID NO: 32, SEQ ID NO: 33 und SEQ ID NO: 34 besteht.

## Revendications

1. Procédé pour améliorer le remplissage des grains d'une plante de blé, ledit procédé comprenant la surexpression dans ladite plante de blé d'un glutamate synthase NADH-dépendante (NADH-GoGAT) présentant au moins 95 % d'identité avec le polypeptide de séquence SEQ ID NO : 1, le pourcentage d'identité entre deux séquences étant calculé à partir d'un alignement des deux séquences sur leur longueur entière.

2. Procédé selon la revendication 1, dans lequel le remplissage des grains est amélioré par l'augmentation du poids des grains et/ou de la teneur en protéines des grains.

3. Procédé selon la revendication 1 ou la revendication 2, dans lequel ladite NADH-GoGAT a la séquence d'acides aminés SEQ ID NO : 22.

4. Procédé selon l'une quelconque des revendications 1 à 3, dans lequel ladite NADH-GoGAT est codée par une séquence nucléotidique choisie dans le groupe constitué de SEQ ID NO : 2, SEQ ID NO : 3, SEQ ID NO : 4, SEQ ID NO : 5, SEQ ID NO : 23, SEQ ID NO : 24, SEQ ID NO : 25 et SEQ ID NO : 26, de préférence SEQ ID NO : 23, SEQ ID NO : 24, SEQ ID NO : 25 et SEQ ID NO : 26.

5. Cassette d'expression recombinante, qui comprend un polynucléotide codant pour une NADH-GoGAT telle que définie dans l'une quelconque des revendications 1, 3 ou 4, sous le contrôle d'un promoteur hétérologue fonctionnel dans une cellule végétale.

6. Vecteur recombinant, qui contient une cassette d'expression comprenant un polynucléotide codant pour une NADH-GoGAT telle que définie dans l'une quelconque des revendications 1, 3 ou 4, sous le contrôle d'un promoteur.

7. Cellule hôte, qui contient une cassette d'expression recombinante selon la revendication 5 ou un vecteur recombinant selon la revendication 6.

8. Cellule hôte selon la revendication 7 qui est une cellule de plante de blé.

9. Procédé de production d'une plante de blé transgénique, présentant un remplissage des grains amélioré, ledit procédé comprenant :
- la fourniture d'une cellule de plante de blé selon la revendication 8 ;
- la régénération à partir de ladite cellule de plante de blé d'une plante de blé transgénique surexprimant une NADH-GoGAT telle que définie dans l'une quelconque des revendications 1 ou 3.

10. Plante de blé transgénique pouvant être obtenue par le procédé selon la revendication 9, ladite plante de blé transgénique contenant une cassette d'expression recombinante telle que définie dans la revendication 5.

11. Plante de blé transgénique ou l'un de ses organes ou tissus isolé, qui comprend, intégrée de façon stable dans son génome, une cassette d'expression recombinante comprenant un polynucléotide codant pour une NADH-GoGAT telle que définie dans l'une quelconque des revendications 1, 3 ou 4.

12. Graines de blé, qui contiennent une cassette d'expression recombinante selon la revendication 5 obtenues à partir d'une plante de blé transgénique selon l'une quelconque des revendications 10 ou 11.

13. Protéine glutamate synthase NADH-dépendante de blé isolée présentant au moins 95 % d'identité avec le polypeptide de séquence SEQ ID NO : 1, le pourcentage d'identité entre deux séquences étant calculé à partir d'un alignement des deux séquences sur leur longueur entière.

14. Protéine glutamate synthase NADH-dépendante de blé isolée selon la revendication 13, qui a la séquence d'acides aminés SEQ ID NO : 22.

15. Polynucléotide isolé choisi dans le groupe constitué de :
a) un polynucléotide codant pour une NADH-GoGAT de blé, lequel polypeptide présentant au moins 95 % d'identité avec le polypeptide de séquence SEQ ID NO : 1, le pourcentage d'identité entre deux séquences étant calculé à partir d'un alignement des deux séquences sur leur longueur entière ;
b) un polynucléotide complémentaire du polynucléotide a).

16. Polynucléotide isolé selon la revendication 15, qui est choisi dans le groupe constitué de : SEQ ID NO : 2, SEQ ID NO : 3, SEQ ID NO : 4, SEQ ID NO : 5, SEQ ID NO : 23, SEQ ID NO : 24, SEQ ID NO : 25 et SEQ ID NO : 26, de préférence SEQ ID NO : 23, SEQ ID NO : 24, SEQ ID NO : 25 et SEQ ID NO : 26.

17. Paire d'amorces, qui est choisie dans le groupe constitué des séquences SEQ ID NO : 7 et SEQ ID NO : 8, SEQ ID NO : 9 et SEQ ID NO : 10, SEQ ID NO : 11 et SEQ ID NO : 12, SEQ ID NO : 13 et SEQ ID NO : 14, SEQ ID NO : 15 et SEQ ID NO : 16, SEQ ID NO : 17 et SEQ ID NO : 18, et SEQ ID NO : 19 et SEQ ID NO : 20, SEQ ID NO : 27 et SEQ ID NO : 28, SEQ ID NO : 29 et SEQ ID NO : 30, SEQ ID NO : 31 et SEQ ID NO : 32, SEQ ID NO : 33 et SEQ ID NO : 34, de préférence SEQ ID NO : 27 et SEQ ID NO : 28, SEQ ID NO : 29 et SEQ ID NO : 30, SEQ ID NO : 31 et SEQ ID NO : 32, SEQ ID NO : 33 et SEQ ID NO : 34.
